# EUROPEAN PATENT SPECIFICATION

(11) **EP 2 836 229 B1**
(45) Date of publication and mention of the grant of the patent: **05.12.2018**
(21) Application number: 13715237.7
(22) Date of filing: 11.04.2013
(51) Int. Cl.: A61K 39/00, A61K 9/127, C07K 14/47

(54) **LIPOSOMES CONTAINING OLIGOPEPTIDE FRAGMENTS OF MYELIN BASIC PROTEIN, A PHARMACEUTICAL COMPOSITION AND A METHOD FOR TREATMENT OF MULTIPLE SCLEROSIS**
LIPOSOME MIT OLIGOPEPTIDFRAGMENTEN EINES BASISCHEN MYELINPROTEINS, PHARMAZEUTISCHE ZUSAMMENSETZUNG UND VERFAHREN ZUR BEHANDLUNG VON MULTIPLER SKLEROSE
LIPOSOMES CONTENANT DES FRAGMENTS OLIGOPEPTIDIQUES DE LA PROTÉINE BASIQUE DE LA MYÉLINE, COMPOSITION PHARMACEUTIQUE ET PROCÉDÉ DE TRAITEMENT DE LA SCLÉROSE EN PLAQUES

(30) Priority: 11.04.2012 US 201213444788
(43) Date of publication of application: 18.02.2015
(73) Proprietor: Lipoxen Technologies Limited, London SW1 2DD (GB)
(72) Inventor: GABIVOV, Alexander, Moscow 8, 10902 (RU); BELOGUROV, Alexey, Moscow 9, 12160 (RU); PONOMARENKO, Natalia, Moscow 3, 11933 (RU); SMIRNOV, Ivan, St. Petersburg 6, 19720 (RU); BACON, Andrew, Lexington, MA 02421 (US); GREGORIADIS, Gregory, Lexington, MA 02421 (US)
(74) Representative: Icosa
(86) International application number: PCT/EP2013/057629
(87) International publication number: WO 2013/153179

(56) References cited:
- WO-A2-2010/024927
- WO-A2-2011/065867
- STEIN C S ET AL: "Myelin-Liposome Protection against Experimental Autoimmune Encephalomyelitis Is Associated with Reduced Neuroantigen-Specific T-Cell-Mediated Responses", CELLULAR IMMUNOLOGY, ACADEMIC PRESS, SAN DIEGO, CA, US, vol. 146, no. 1, 1 January 1993 (1993-01-01), pages 80-95, XP024000523, ISSN: 0008-8749, DOI: 10.1006/CIMM.1993.1008 [retrieved on 1993-01-01]
- A. A. BELOGUROV ET AL: "Liposome-encapsulated peptides protect against experimental allergic encephalitis", THE FASEB JOURNAL, vol. 27, no. 1, 1 January 2013 (2013-01-01), pages 222-231, XP055068040, ISSN: 0892-6638, DOI: 10.1096/fj.12-213975

### SEQUENCE LISTING

<110> LIPOXEN TECHNOLOGIES LIMITED
   GABIVOV, Alexander
   BELOGUROV, Alexey
   PONOMARENKO, Natalia
   SMIRNOV, Ivan
   BACON, Andrew
   GREGORIADIS, Gregory
<120> LIPOSOMES CONTAINING OLIGOPEPTIDE FRAGMENTS OF MYELIN BASIC PROTEIN, A PHARMACEUTICAL COMPOSITION AND A METHOD FOR TREATMENT OF MULTIPLE SCLEROSIS
<130> P39946WO
<140> Filed herewith
   <141> 2013-04-11
<150> US 13/444,788
   <151> 2012-04-11
<160> 20
<170> PatentIn version 3.5
<210> 1
   <211> 17
   <212> PRT
   <213> Artificial Sequence
<220>
   <223> MBP peptide
<400> 1
   Gly Gly Asp Arg Gly Ala Pro Lys Arg Gly Ser Gly Lys Asp Ser His
   1               5                   10                  15
   His
<210> 2
   <211> 16
   <212> PRT
   <213> Artificial Sequence
<220>
   <223> MBP peptide
<400> 2
   Gly Phe Gly Tyr Gly Gly Arg Ala Ser Asp Tyr Lys Ser Ala His Lys
   1               5                   10                  15
<210> 3
   <211> 24
   <212> PRT
   <213> Artificial Sequence
<220>
   <223> MBP peptide
<400> 3
   Gln Gly Thr Leu Ser Lys Ile Phe Lys Leu Gly Gly Arg Asp Ser Arg
   1               5                   10                  15
   Ser Gly Ser Pro Met Ala Arg Arg
               20
<210> 4
   <211> 17
   <212> PRT
   <213> Artificial Sequence
<220>
   <223> MBP encephalogenic peptide fragment
<400> 4
   Ala Arg Thr Thr His Tyr Gly Ser Leu Pro Gln Lys Ser Gln Arg Ser
   1               5                   10                  15
   Gln
<210> 5
   <211> 18
   <212> PRT
   <213> Artificial Sequence
<220>
   <223> a prototype peptide
<400> 5
   Asp Glu Asn Pro Val Val Ile Ile Phe Phe Lys Asn Ile Val Thr Pro
   1               5                   10                  15
   Arg Thr
<210> 6
   <211> 12
   <212> PRT
   <213> Artificial Sequence
<220>
   <223> MBP fragments
<400> 6
   Met Asp His Ala Arg His Gly Phe Leu Pro Arg His
   1               5                   10
<210> 7
   <211> 14
   <212> PRT
   <213> Artificial Sequence
<220>
   <223> MBP fragments
<400> 7
   Gln Asp Glu Asn Pro Val Val His Phe Phe Lys Asn Ile Val
   1               5                   10
<210> 8
   <211> 18
   <212> PRT
   <213> Artificial Sequence
<220>
   <223> MBP fragments
<400> 8
   Ile Phe Lys Leu Gly Gly Arg Asp Ser Arg Ser Gly Ser Pro Met Ala
   1               5                   10                  15
   Arg Arg
<210> 9
   <211> 21
   <212> PRT
   <213> Artificial Sequence
<220>
   <223> MBP1FL
<400> 9
   Arg Thr Gly Gly Ile Leu Asp Ser Ile Gly Arg Phe Phe Gly Gly Asp
   1               5                   10                  15
   Arg Gly Ala Pro Lys
               20
<210> 10
   <211> 19
   <212> PRT
   <213> Artificial Sequence
<220>
   <223> MBP1FR
<400> 10
   Lys Arg Gly Ser Gly Lys Asp Ser His His Pro Ala Arg Thr Ala His
   1               5                   10                  15
   Tyr Gly Ser
<210> 11
   <211> 22
   <212> PRT
   <213> Artificial Sequence
<220>
   <223> MBP peptide (43-64)
<400> 11
   Arg Phe Phe Gly Gly Asp Arg Gly Ala Pro Lys Arg Gly Ser Gly Lys
   1 5 10 15
   Asp Ser His His Pro Ala
   20
<210> 12
   <211> 57
   <212> PRT
   <213> Artificial Sequence
<220>
   <223> MBP peptide (115-170)
<400> 12
   Phe Ser Trp Gly Ala Glu Gly Gln Arg Pro Gly Phe Gly Tyr Gly Gly
   1 5 10 15
   Arg Ala Ser Asp Tyr Lys Ser Ala His Lys Gly Phe Lys Gly Val Asp
   20 25 30
   Ala Gln Gly Thr Leu Ser Lys Ile Phe Lys Leu Gly Gly Arg Asp Ser
   35 40 45
   Arg Ser Gly Ser Pro Met Ala Arg Arg
   50 55
<210> 13
   <211> 304
   <212> PRT
   <213> Artificial Sequence
<220>
   <223> MBP splice isoforms
<400> 13
   Met Gly Asn His Ala Gly Lys Arg Glu Leu Asn Ala Glu Lys Ala Ser
   1 5 10 15
   Thr Asn Ser Glu Thr Asn Arg Gly Glu Ser Glu Lys Lys Arg Asn Leu
               20                  25                  30
   Gly Glu Leu Ser Arg Thr Thr Ser Glu Asp Asn Glu Val Phe Gly Glu
           35                  40                  45
   Ala Asp Ala Asn Gln Asn Asn Gly Thr Ser Ser Gln Asp Thr Ala Val
       50                  55                  60
   Thr Asp Ser Lys Arg Thr Ala Asp Pro Lys Asn Ala Trp Gln Asp Ala
   65                  70                  75                  80
   His Pro Ala Asp Pro Gly Ser Arg Pro His Leu Ile Arg Leu Phe Ser
                   85                  90                  95
   Arg Asp Ala Pro Gly Arg Glu Asp Asn Thr Phe Lys Asp Arg Pro Ser
               100                 105                 110
   Glu Ser Asp Glu Leu Gln Thr Ile Gln Glu Asp Ser Ala Ala Thr Ser
           115                 120                 125
   Glu Ser Leu Asp Val Met Ala Ser Gln Lys Arg Pro Ser Gln Arg His
       130                 135                 140
   Gly Ser Lys Tyr Leu Ala Thr Ala Ser Thr Met Asp His Ala Arg His
   145                 150                 155                 160
   Gly Phe Leu Pro Arg His Arg Asp Thr Gly Ile Leu Asp Ser Ile Gly
                   165                 170                 175
   Arg Phe Phe Gly Gly Asp Arg Gly Ala Pro Lys Arg Gly Ser Gly Lys
               180                 185                 190
   Asp Ser His His Pro Ala Arg Thr Ala His Tyr Gly Ser Leu Pro Gln
           195                 200                 205
   Lys Ser His Gly Arg Thr Gln Asp Glu Asn Pro Val Val His Phe Phe
       210                 215                 220
   Lys Asn Ile Val Thr Pro Arg Thr Pro Pro Pro Ser Gln Gly Lys Gly
   225                 230                 235                 240
   Arg Gly Leu Ser Leu Ser Arg Phe Ser Trp Gly Ala Glu Gly Gln Arg
                   245                 250                 255
   Pro Gly Phe Gly Tyr Gly Gly Arg Ala Ser Asp Tyr Lys Ser Ala His
               260                 265                 270
   Lys Gly Phe Lys Gly Val Asp Ala Gln Gly Thr Leu Ser Lys Ile Phe
   275 280 285
   Lys Leu Gly Gly Arg Asp Ser Arg Ser Gly Ser Pro Met Ala Arg Arg
   290 295 300
<210> 14
   <211> 197
   <212> PRT
   <213> Artificial Sequence
<220>
   <223> MBP splice isoforms
<400> 14
   Met Gly Asn His Ala Gly Lys Arg Glu Leu Asn Ala Glu Lys Ala Ser
   1 5 10 15
   Thr Asn Ser Glu Thr Asn Arg Gly Glu Ser Glu Lys Lys Arg Asn Leu
   20 25 30
   Gly Glu Leu Ser Arg Thr Thr Ser Glu Asp Asn Glu Val Phe Gly Glu
   35 40 45
   Ala Asp Ala Asn Gln Asn Asn Gly Thr Ser Ser Gln Asp Thr Ala Val
   50 55 60
   Thr Asp Ser Lys Arg Thr Ala Asp Pro Lys Asn Ala Trp Gln Asp Ala
   65 70 75 80
   His Pro Ala Asp Pro Gly Ser Arg Pro His Leu Ile Arg Leu Phe Ser
   85 90 95
   Arg Asp Ala Pro Gly Arg Glu Asp Asn Thr Phe Lys Asp Arg Pro Ser
   100 105 110
   Glu Ser Asp Glu Leu Gln Thr Ile Gln Glu Asp Ser Ala Ala Thr Ser
   115 120 125
   Glu Ser Leu Asp Val Met Ala Ser Gln Lys Arg Pro Ser Gln Arg His
   130 135 140
   Gly Ser Lys Tyr Leu Ala Thr Ala Ser Thr Met Asp His Ala Arg His
   145 150 155 160
   Gly Phe Leu Pro Arg His Arg Asp Thr Gly Ile Leu Asp Ser Ile Gly
   165 170 175
   Arg Phe Phe Gly Gly Asp Arg Gly Ala Pro Lys Arg Gly Ser Gly Lys
               180                 185                 190
   Val Ser Ser Glu Glu
           195
<210> 15
   <211> 197
   <212> PRT
   <213> Artificial Sequence
<220>
   <223> MBP splice isoforms
<400> 15
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Val Pro Trp Leu Lys
       50                  55                  60
   Pro Gly Arg Ser Pro Leu Pro Ser His Ala Arg Ser Gln Pro Gly Leu
   65                  70                  75                  80
   Cys Asn Met Tyr Lys Asp Ser His His Pro Ala Arg Thr Ala His Tyr
                   85                  90                  95
   Gly Ser Leu Pro Gln Lys Ser His Gly Arg Thr Gln Asp Glu Asn Pro
               100                 105                 110
   Val Val His Phe Phe Lys Asn Ile Val Thr Pro Arg Thr Pro Pro Pro
           115                 120                 125
   Ser Gln Gly Lys Gly Arg Gly Leu Ser Leu Ser Arg Phe Ser Trp Gly
       130                 135                 140
   Ala Glu Gly Gln Arg Pro Gly Phe Gly Tyr Gly Gly Arg Ala Ser Asp
   145                 150                 155                 160
   Tyr Lys Ser Ala His Lys Gly Phe Lys Gly Val Asp Ala Gln Gly Thr
                   165                 170                 175
   Leu Ser Lys Ile Phe Lys Leu Gly Gly Arg Asp Ser Arg Ser Gly Ser
                   180                 185                 190
   Pro Met Ala Arg Arg
           195
<210> 16
   <211> 186
   <212> PRT
   <213> Artificial Sequence
<220>
   <223> MBP splice isoforms
<400> 16
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Val Pro Trp Leu Lys
       50                  55                  60
   Pro Gly Arg Ser Pro Leu Pro Ser His Ala Arg Ser Gln Pro Gly Leu
   65                  70                  75                  80
   Cys Asn Met Tyr Lys Asp Ser His His Pro Ala Arg Thr Ala His Tyr
                   85                  90                  95
   Gly Ser Leu Pro Gln Lys Ser His Gly Arg Thr Gln Asp Glu Asn Pro
               100                 105                 110
   Val Val His Phe Phe Lys Asn Ile Val Thr Pro Arg Thr Pro Pro Pro
           115                 120                 125
   Ser Gln Gly Lys Gly Ala Glu Gly Gln Arg Pro Gly Phe Gly Tyr Gly
       130                 135                 140
   Gly Arg Ala Ser Asp Tyr Lys Ser Ala His Lys Gly Phe Lys Gly Val
   145                 150                 155                 160
   Asp Ala Gln Gly Thr Leu Ser Lys Ile Phe Lys Leu Gly Gly Arg Asp
                   165                 170                 175
   Ser Arg Ser Gly Ser Pro Met Ala Arg Arg
               180                 185
<210> 17
   <211> 171
   <212> PRT
   <213> Artificial Sequence
<220>
   <223> MBP splice isoforms
<400> 17
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Asp Ser His His Pro
       50                  55                  60
   Ala Arg Thr Ala His Tyr Gly Ser Leu Pro Gln Lys Ser His Gly Arg
   65                  70                  75                  80
   Thr Gln Asp Glu Asn Pro Val Val His Phe Phe Lys Asn Ile Val Thr
                   85                  90                  95
   Pro Arg Thr Pro Pro Pro Ser Gln Gly Lys Gly Arg Gly Leu Ser Leu
               100                 105                 110
   Ser Arg Phe Ser Trp Gly Ala Glu Gly Gln Arg Pro Gly Phe Gly Tyr
           115                 120                 125
   Gly Gly Arg Ala Ser Asp Tyr Lys Ser Ala His Lys Gly Phe Lys Gly
       130                 135                 140
   Val Asp Ala Gln Gly Thr Leu Ser Lys Ile Phe Lys Leu Gly Gly Arg
   145                 150                 155                 160
   Asp Ser Arg Ser Gly Ser Pro Met Ala Arg Arg
                   165                 170
<210> 18
   <211> 160
   <212> PRT
   <213> Artificial Sequence
<220>
   <223> MBP splice isoforms
<400> 18
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Asp Ser His His Pro
       50                  55                  60
   Ala Arg Thr Ala His Tyr Gly Ser Leu Pro Gln Lys Ser His Gly Arg
   65                  70                  75                  80
   Thr Gln Asp Glu Asn Pro Val Val His Phe Phe Lys Asn Ile Val Thr
                   85                  90                  95
   Pro Arg Thr Pro Pro Pro Ser Gln Gly Lys Gly Ala Glu Gly Gln Arg
               100                 105                 110
   Pro Gly Phe Gly Tyr Gly Gly Arg Ala Ser Asp Tyr Lys Ser Ala His
           115                 120                 125
   Lys Gly Phe Lys Gly Val Asp Ala Gln Gly Thr Leu Ser Lys Ile Phe
       130                 135                 140
   Lys Leu Gly Gly Arg Asp Ser Arg Ser Gly Ser Pro Met Ala Arg Arg
   145                 150                 155                 160
<210> 19
   <211> 74
   <212> PRT
   <213> Artificial Sequence
<220>
   <223> MBP splice isoforms
<400> 19
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Ser Leu Pro Gln
               20                  25                  30
   Lys Ser His Gly Arg Thr Gln Asp Glu Asn Pro Val Val His Phe Phe
           35                  40                  45
   Lys Asn Ile Val Thr Pro Arg Thr Pro Pro Pro Ser Gln Gly Lys Gly
       50                  55                  60
   Arg Gly Leu Ser Leu Ser Arg Phe Ser Trp
   65                  70
<210> 20
   <211> 9
   <212> PRT
   <213> Artificial Sequence
<220>
   <223> MBP epitope
<400> 20
   Arg His Gly Phe Leu Pro Arg His Arg
   1               5

## Description

### BACKGROUND OF THE INVENTION

Multiple sclerosis (MS) is neurodegenerative disease in which the fatty myelin sheaths around the axons of the brain and spinal cord are damaged, leading to demyelination and scarring. The damage caused to the central nerve system (CNS) results in a wide spectrum of neurological symptoms. Approximately one million people worldwide suffer from this autoimmune disease, which has an enigmatic etiology and poorly understood pathogenesis. B- and T-cells reactive against components of the myelin membrane mediate the demyelination of the brain and spinal cord and appear to be responsible for a large portion of disease progression.

The list of potential autoantigens to which B- and T-cells are reactive against in MS patients is progressively growing and includes several oligodendrocyte-associated proteins, most-notably myelin basic protein (MBP) and myelin oligodendrocyte glycoprotein (MOG). Infiltration of the central nervous system by these macrophages and lymphocytes, through the blood brain barrier (BBB), results in the formation of inflammatory demyelinating lesions in the brain and spinal cord.

While T cells are responsible for a large portion of the demyelinating effect, B cells play a substantial role as well. This is because B cells function as antigen presenting cells and cytokine producing cells, in addition to their well-recognized role in antibody production (Hikada and Zouali, Nat Immunol 2010;11:1065-8). Additional evidence of the involvement of B cells in demyelination is the detection of catalytic antibodies to MBP in multiple sclerosis patients. These catalytic antibodies are able to not only bind their antigen, but to cleave it as well (Ponomarenko NA et al., Proc Natl Acad Sci U S A 2006;103:281-6). Evidence suggests that there is a strong environmental component to the progression of MS, in which autoantibodies cross-reactive to neuronal and viral antigens contribute to the etiology and pathogenesis of MS (Gabibov AG et al., FASEB J 2011;25:4211-21).

Many MS therapies have been proposed, including: (i) administration of glatiramer acetate (GA); (ii) administration of "altered peptide ligands" (APLs) that interact with T cell receptors (TCR); (iii) IFNβ administration; (iv) administration of anti-CD20 anti-CD25, and anti-CD52 monoclonal antibodies; (v) various oral therapies; (vi) vaccination with inactivated T-cells or TCR hypervariable regions; (vii) tolerization of the immune system by administration of autoantigens, or DNA-vaccination; and (viii) B cell-targeted depletion therapy.

Nevertheless, despite promising clinical, immunological, and biochemical data, none of the existing therapies are capable of curing or preventing MS progression. Thus, there is a great need in the art for efficacious MS therapeutic approaches.

### BRIEF SUMMARY OF INVENTION

In one aspect, the present invention satisfies a need in the field of medicine for efficacious compositions and methods of treating multiple sclerosis (MS), by providing a therapeutic composition of immunodominant MBP peptides linked to a vector for administration to a subject in need thereof. In a specific embodiment, the composition comprises immunodominant MBP peptides encapsulated in a mannosylated liposome. As shown herein, administration of these compositions ameliorates ongoing experimental autoimmune encephalomyelitis in an EAE-induced rat model of MS.

The present invention is based, in part, on the discovery that certain MBP peptides are major B cell epitopes in patients suffering from multiple sclerosis. It was found that administration of liposomal formulations of these peptides, but not the free peptides, to rodent models of MS resulted in a statistically significant reduction in paralysis. Without being bound by theory, it is possible that liposomal formulation of these peptides results improved delivery of these peptides to immune cells (for example, B cells and/or antigen presenting cells) and/or improves intake of these peptides into immune cells (for example, B cells and/or antigen presenting cells).

Accordingly, the present invention provides, among other aspects, compositions and methods for treating multiple sclerosis. The compositions comprise one or more of the identified MBP peptides linked to a vector (e.g., a mannosylated liposome).

In one aspect, the present invention provides a composition for use in medicine, the composition consisting of two myelin basic protein (MBP) peptides linked to a vector, the first MBP peptide consisting of the amino acid sequence of SEQ ID NO:11 and the second MBP peptide consisting of the amino acid sequence of SEQ ID NO:12, and the vector comprising a liposome having a surface exposed targeting moiety comprising a manDOG mannose residue.

In one embodiment of the compositions provided above, the MBP peptide is covalently linked to the vector. In another embodiment of the compositions provided above, the MBP peptide is non-covalently linked to the vector.

In one embodiment of the compositions provided above, the vector comprises a targeting moiety. In a specific embodiment of the compositions provided above, the vector is a targeting moiety.

In one embodiment of the compositions provided above, the targeting moiety increases: (a) delivery of the MBP peptide to an immune cell; or (b) intake of the MBP peptide into an immune cell, as compared to an MBP peptide linked to a vector in the absence of a targeting moiety.

In one embodiment of the compositions provided above, the targeting moiety comprises a mannose residue. In one embodiment of the compositions provided above, the immune cell is a B cell. In another embodiment of the compositions provided above, the immune cell is an antigen presenting cell (APC).

In one embodiment of the compositions provided above, the MBP peptide(s) are non-covalently linked to the liposome. In another embodiment of the compositions provided above, the MBP peptide(s) are encapsulated by the liposome.

In one embodiment of the compositions provided above, the liposome has an average diameter of from 100 nm to 200 nm.

In one embodiment of the compositions provided above, the mannose residue is manDOG.

In one embodiment of the methods provided above, the composition is administered to the patient at least once a week. In another embodiment of the methods provided above, the composition is administered to the patient at least twice a week. In another embodiment of the methods provided above, the composition is administered to the patient daily.

In one embodiment of the methods provided above, the composition is administered by topical administration, enteric administration, or parenteral administration.

### BRIEF DESCRIPTION OF DRAWINGS

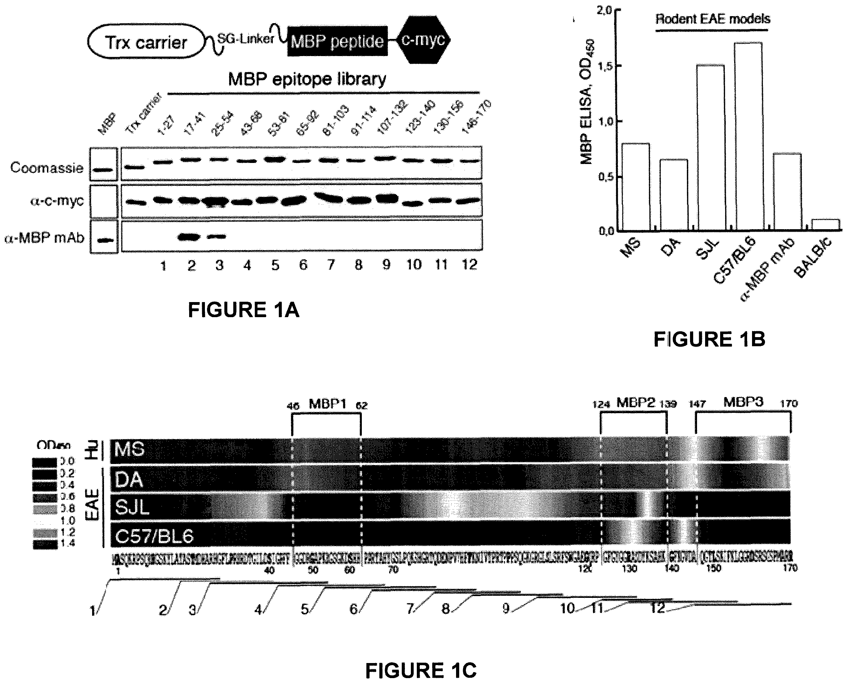

**Figure 1****.** *DA rats with induced EAE are the most relevant rodent models of MS in terms of anti-MBP autoantibodies binding pattern.* (**A**) Serum autoantibodies from MS patients and rodent models developing experimental autoimmune encephalomyelitis (DA rats, SJL and C57BL/6 mice) reproducibly bind MBP in ELISA. Serum of BALB/c mice was used as a negative control. **(B)** Design of MBP epitope library. Representative Coomassie staining and western-blotting hybridization of anti-c-myc and anti-MBP mAbs with MBP epitope library. Anti-c-myc Ab binds all members of MBP epitope library due to the presence of targeted epitope in all fusion proteins (scheme on top), thus, suggesting exposure and accessibility of all MBP peptides, located directly upstream to the c-myc epitope. Monoclonal anti-MBP Ab (clone F4A3, MBP epitope RHGFLPRHR (SEQ ID NO:20)) reacts with whole MBP and its peptides MBP2 and MBP3 as predicted. **(C)** Serum autoantibodies binding pattern to MBP epitope library according to ELISA. According to our data DA rats developing EAE are the most relevant to MS rodent model. MBP sequence with peptides presented in its epitope library is shown on bottom (SEQ ID NO: 17). Each tenth amino acid residue is marked by bold. Brackets represent immunodominant peptides MBP-1/-2/-3, selected for treatment efficiency screening.

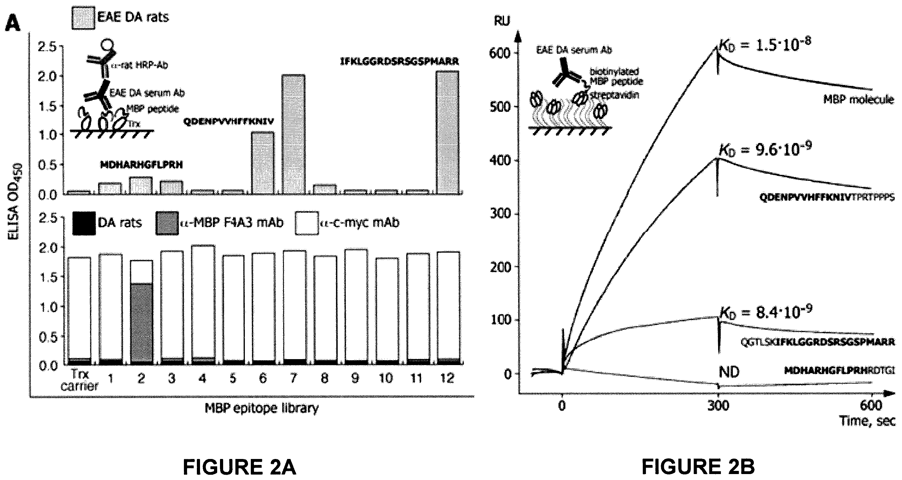

**Figure 2****.** *Characterization of specificity and affinity of polyclonal antibodies from DA rats, immunized with MBP(63-81).* **(A)** Upper panel shows that three MBP fragments are recognized by serum autoAb from DA rats with induced EAE. Immunodominant peptides were determined according to the ELISA of autoAb binding with epitope library and further theoretical calculation based on the assumption of their overlapping sequences. Additionally, the MBP epitope library was hybridized with anti-c-myc and anti-MBP F4A3 mAb to verify the binding assay (bottom panel). **(B)** Quantitative characteristics of the determined epitopes recognition by autoAb, measured by SPR technique. Respective peptides and effective dissociation constants are shown. Exact epitopes are shown in bold, ND = not determined.

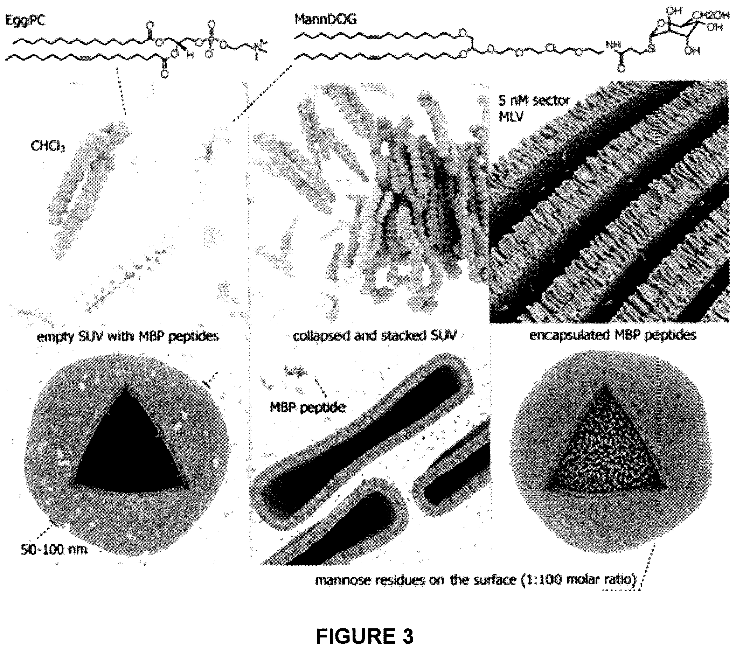

**Figure 3**. *Schematic representation of the liposomation technique used to encapsulate MBP immunodominant peptides into mannosylated SUV liposomes.* (Top Left) Mixture of lipids (Egg PC with 1% of Mannosylated DOG) in chloroform. (Top Middle) Formation of irregular lipid layers during evaporation of organic solvent. (Top Right) First rehydration leading to the multi-layer MLV liposomes formation. Average diameter of particles is between 1-5 µm. (Bottom Left) Freeze drying of SUV liposomes, obtained from MLV liposomes by high-pressure homogenization, and peptide mixture with excess sugar. (Bottom Center) Peptides are located between collapsed SUV liposomes. (Bottom Right) Encapsulation of peptides during second rehydration into the SUV liposomes with size approximately 60-80 nm and 1.0 % of mannose residues on the surface. Renderings were executed by Visual Science Company.

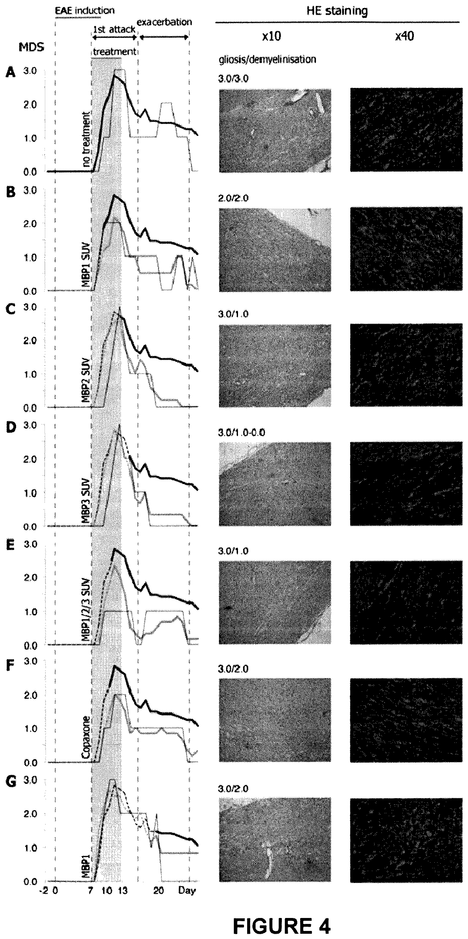

**Figure 4****.** *Liposome-entrapped immunodominant MBP peptides ameliorate experimental autoimmune encephalomyelitis in DA rats.* All groups under consideration (A-G) were examined in terms of mean disease score, gliosis/demyelination rate. Untreated control (vehicle) group is shown by bold dark line **(A)** and repeated in each plot for comparison. Three immunodominant fragments of MBP encapsulated in mannosylated SUV liposomes were used for EAE treatment in DA rats: MBP(46-62) was the most effective in decreasing of maximal disease score during first attack **(B)**, MBP(124-139) **(C)**, and MBP(147-170) **(D)** prevented development of remission stage. Administration of a mixture of MBP(46-62), MBP(124-139), and MBP(147-170) immunodominant MBP peptides entrapped in liposomes significantly ameliorated the protracted EAE **(E)**, copaxone **(F),** and free peptide MBP(46-62) **(G)** were used as positive and negative controls, respectively. Mean disease score is shown for each group. Statistically significant difference is shown by bold light line. Representative profile of selected individual rat is shown by thin line. Representative hematoxylin and eosin staining is shown on right panel.

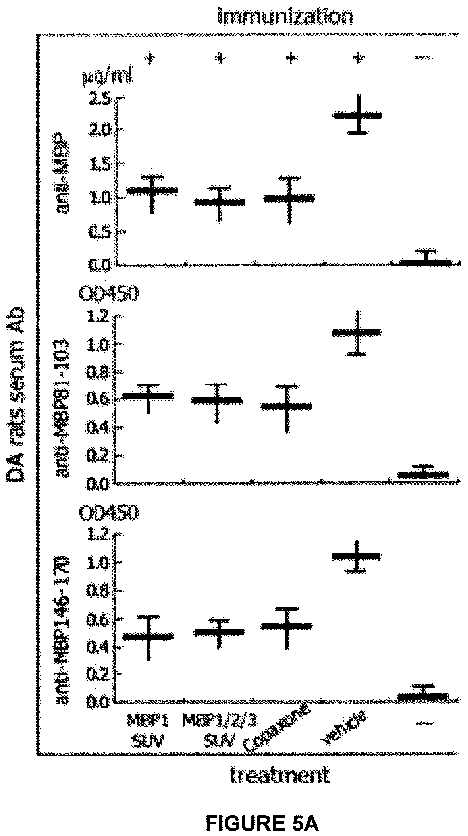

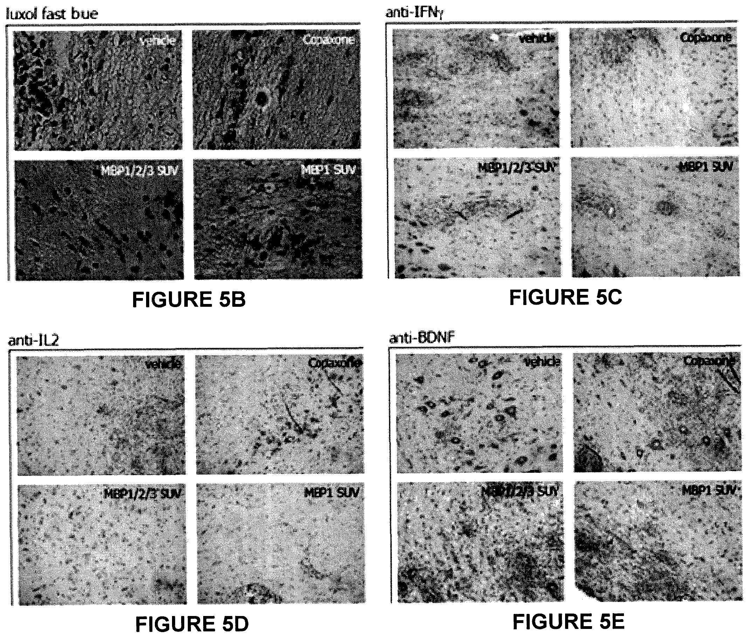

**Figure 5****.** *Liposome-entrapped immunodominant MBP peptides decrease serum anti-MBP autoantibody titer and down regulate Th1 CNS cytokine profile.* **(A)** Serum anti-MBP autoAb concentration in EAE DA rats treated with MBP1 SUV, MBP1/2/3 SUV, and copaxone compared to untreated and non-immunized rats. Representative luxol fast blue staining **(B)**, immunostaining for Th1 cytokines IFNγ (**B**) and IL2 (**C**), and immunostaining of BDNF **(D)** in brain sections of DA rats treated with MBP1 SUV (Bottom Right), MBP1/2/3 SUV (Bottom Left), and copaxone (Top Right), contrasted to untreated rats (Top Left).

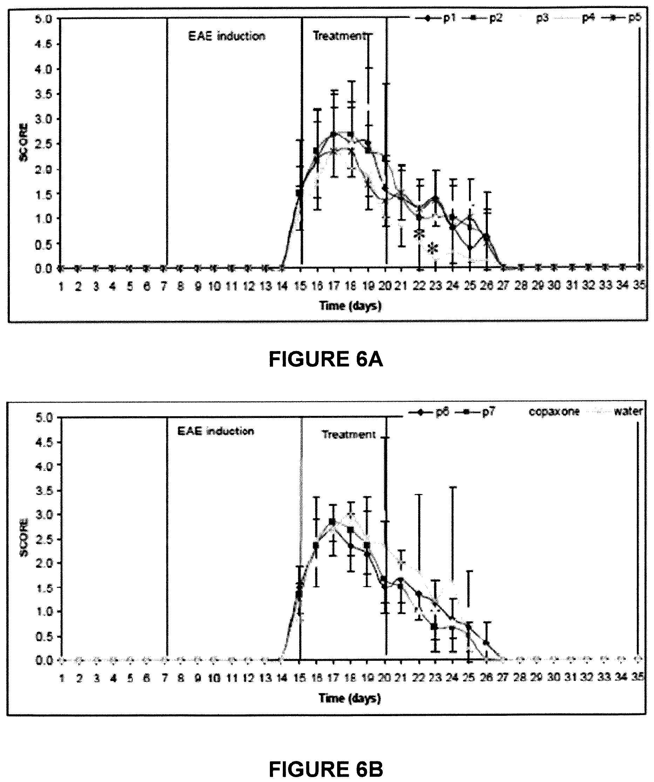

**Figure 6****.** *Mean paralysis score for EAE-induced rat models of MS.* A paralysis score was assigned daily to each rat for the duration of the study periods: acclimatization, EAE induction, treatment and post treatment (total 35 days). 54 EAE-induced DA rats were separated equally to 9 groups. Groups I-VII were treated with formulations 1-7, group VIII was treated with copaxone (positive control group), group IX was administrated a water injection (negative control group). Paralysis scores were recorded daily and are displayed as mean values for groups I-V **(A)** and groups VI-IX (B). A statistically significant reduction in paralysis score, as compared to the controls, was observed for group IV (treated with formulation 4) on days 3 and 4 after the treatment (n=6, *p<0.05). Standard deviation is indicated with error bars.

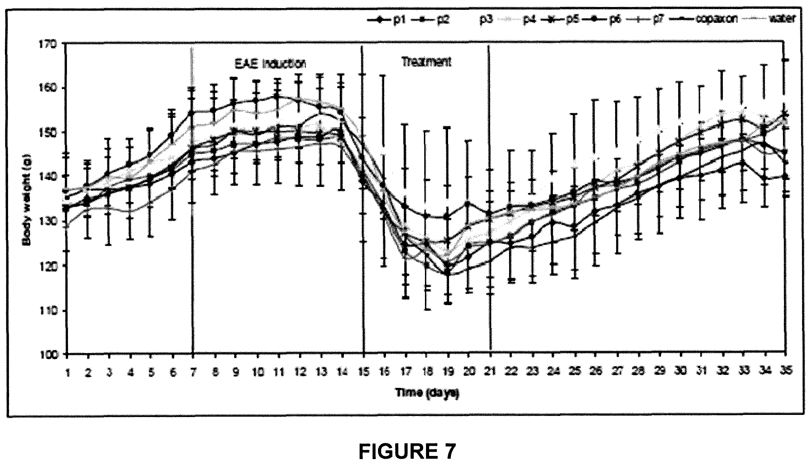

**Figure 7****.** *Mean body weights (g) of EAE-induced rat models of MS.* Body weight of all animals was recorded during all study periods: acclimatization, EAE induction, treatment, and post treatment (total 35 days). 54 EAE induced DA rats were separated equally into 9 groups. No statistically significant differences were found between the body weights of the rats treated with MBP peptide formulations and the control groups. Standard deviation is indicated with error bars.

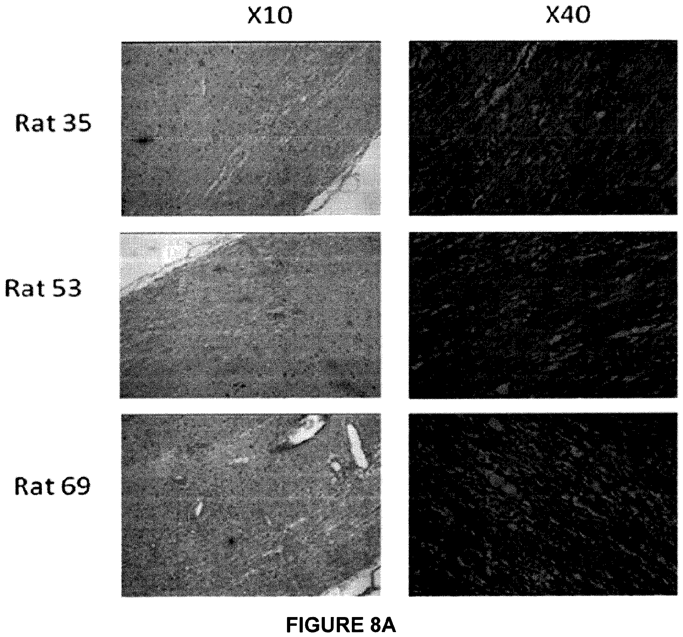

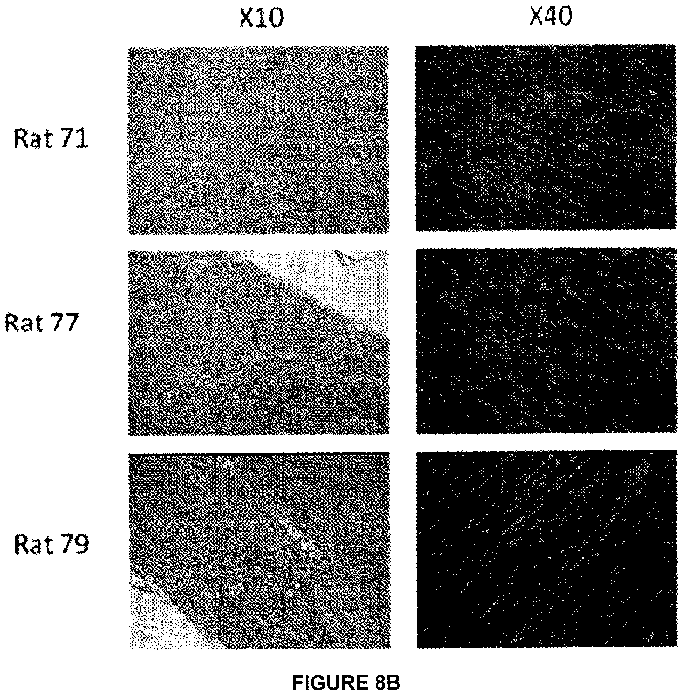

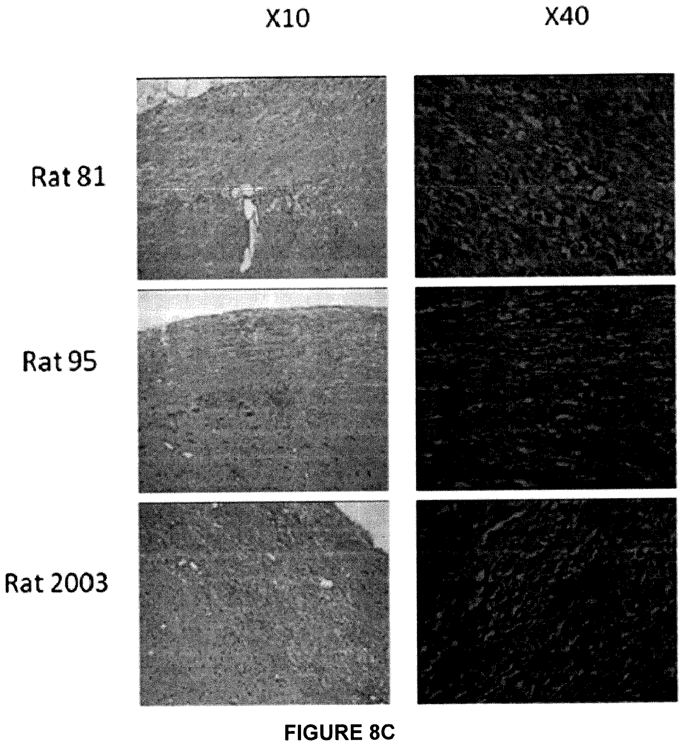

**Figure 8****.** *Hematoxylin and eosin (H&E) staining of spinal cord from EAE-induced rat models of MS*. Images at 10x and 40x magnification of spinal cord isolated from EAE-induced rats treated with: **(A)** (rat 35; group II), (rat 53; group IV), (rat 69; group IX); **(B)** (rat 71; group VIII), (rat 77; group III), (rat 79; group V); and **(C)** (rat 81; group I), (rat 95; group VII), (rat 2003; group VI).

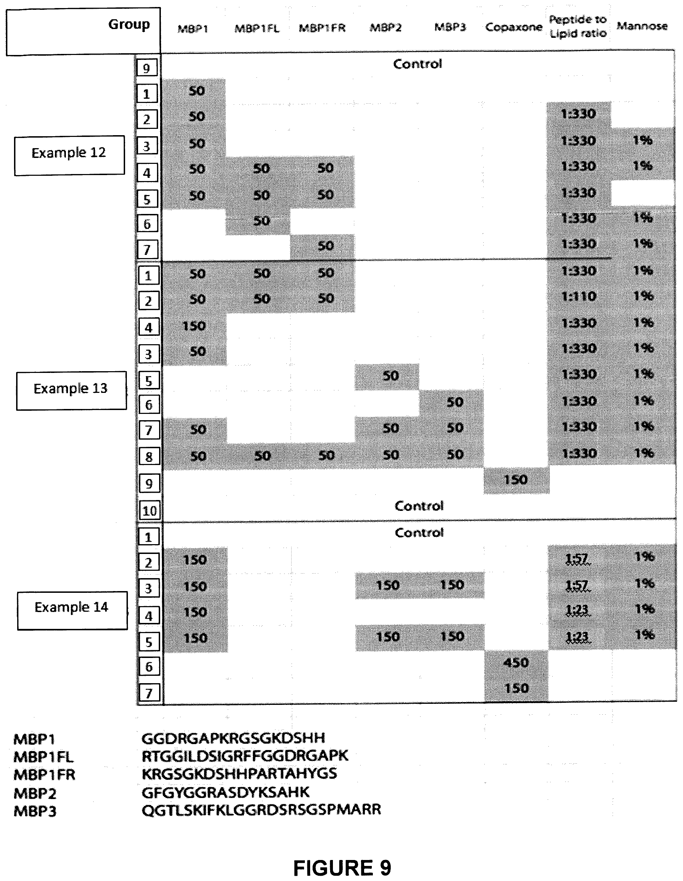

**Figure 9****.** *Experimental design.* Experimental set-up, including peptide identities, liposomal content, and dosage for all experimental formulations tested in Examples 12, 13, and 14. MBP1 (SEQ ID NO:1); MBP1FL (SEQ ID NO:9); MBP1FR (SEQ ID NO:10); MBP2 (SEQ ID NO:2); MBP3 (SEQ ID NO:3).

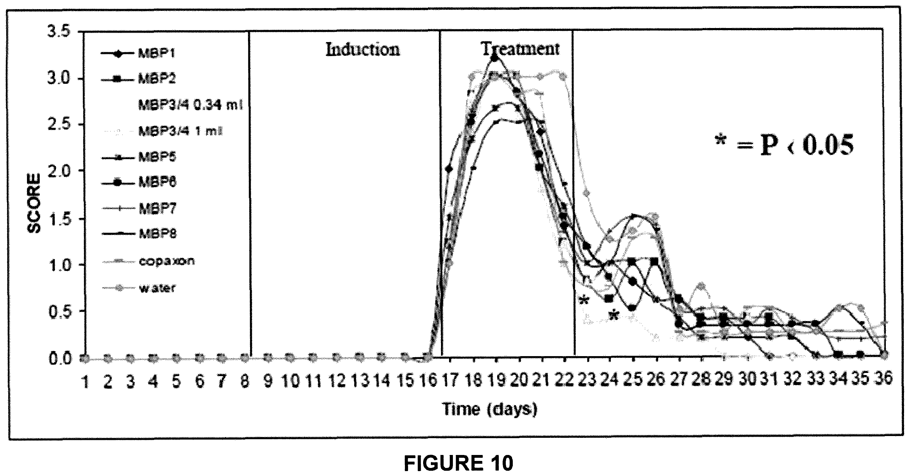

**Figure 10****.** *Mean paralysis score for EAE-induced rat models of MS.* A paralysis score was assigned daily to each rat for the duration of the study periods: acclimatization, EAE induction, treatment and post treatment (total 36 days). 54 EAE induced DA rats were separated to 10 groups. Groups I-VIII were treated with formulations MBP F 1-8, group IX was treated with Copaxone (positive control group), group X administrated water injection (negative control group). A statistically significant reduction in paralysis score, as compared to the controls, was observed for groups III and IV (treated with a 200 mg dose) on days 2 and 3 post treatment (n=5, *p<0.05). Standard deviation is indicated with error bars.

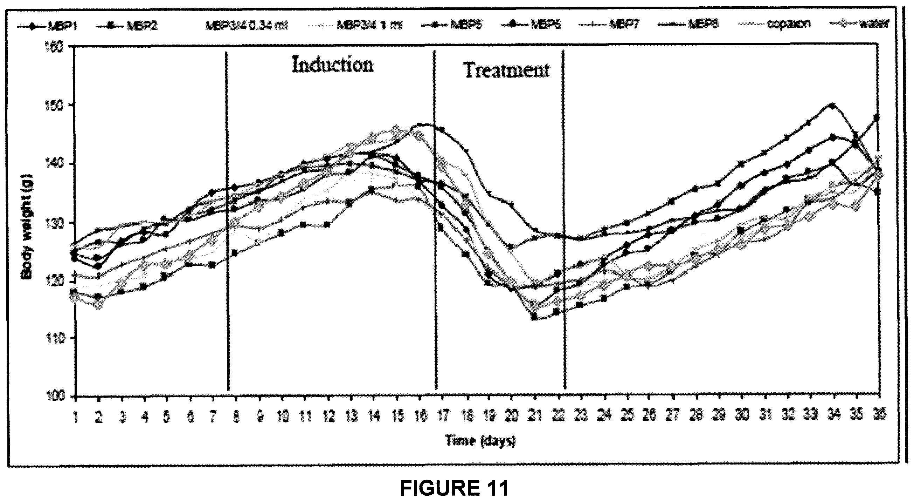

**Figure 11****.** *Mean body weights (g) of EAE-induced rat models of MS.* Body weight of all animals was recorded during all study periods: acclimatization, EAE induction, treatment, and post treatment (total 36 days). 54 EAE induced DA rats were separated equally into 10 groups. No statistically significant differences were found between the body weights of the rats treated with MBP peptide formulations and the control groups. Standard deviation is indicated with error bars.

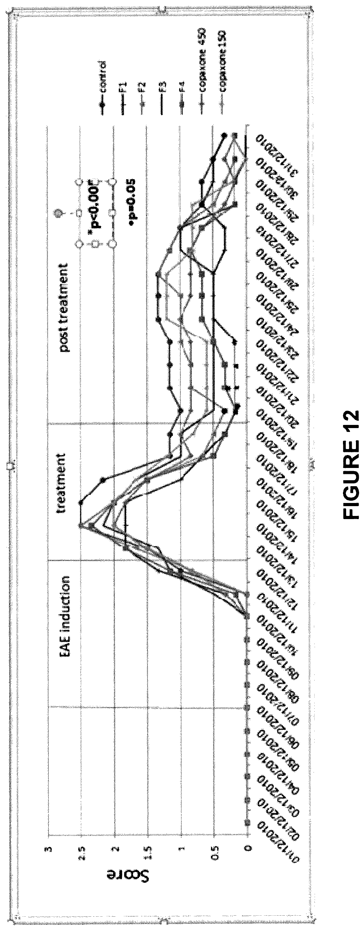

**Figure 12****.** *Mean paralysis score for EAE-induced rat models of MS.* A paralysis score was assigned daily to each rat for the duration of the study periods: acclimatization, EAE induction, treatment and post treatment. 42 EAE induced DA rats were separated to 7 groups. Groups II-V were treated with formulations MBP F I-IV, groups VI and VII were treated with copaxone (150 µg and 450 µg, respectively; positive control groups), and group I was administrated water injection (negative control group). A statistically significant reduction in paralysis score, as compared to the negative control, was observed for: group II (treated with liposomal formulation of MBP1; 1:330 peptide to lipid ratio) on days 1-4 post treatment (n=6, *p<0.005); group III (treated with liposomal formulation of MBP1/2/3; 1:330 peptide to lipid ratio) at day 1 post-treatment (n=6, *p<0.05); and group V (treated with liposomal formulation of MBP1/2/3; 1:110 peptide to lipid ratio) on days 1-3 post-treatment (n=6, *p<0.05). Standard deviation is indicated with error bars.

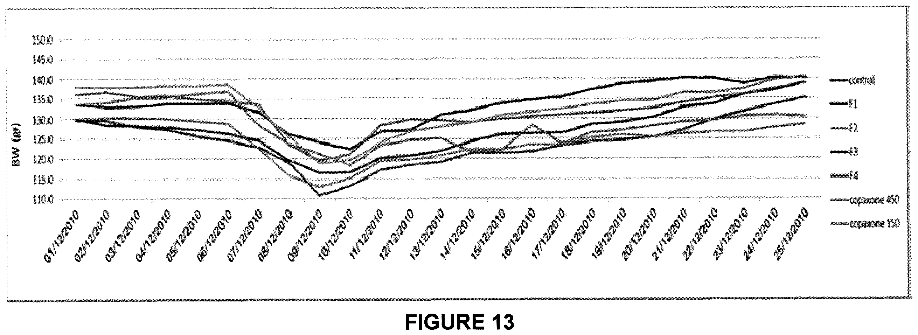

**Figure 13****.** *Mean body weights (g) of EAE-induced rat models of MS.* Body weight of all animals was recorded during all study periods: acclimatization, EAE induction, treatment, and post treatment. 42 EAE induced DA rats were separated equally into 7 groups. No statistically significant differences were found between the body weights of the rats treated with MBP peptide formulations and the control groups. Standard deviation is indicated with error bars.

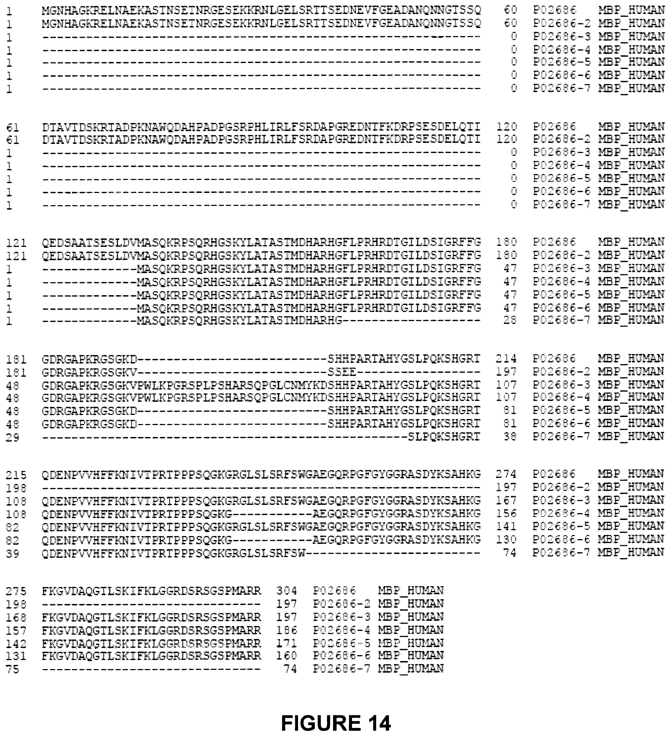

**Figure 14****.** *Sequence alignment of 7 MBP splice isoforms.* UniProt ID Nos.: P02686 (SEQ ID NO:13); P02686-2 (SEQ ID NO:14); P02686-3 (SEQ ID NO:15); P02686-4 (SEQ ID NO:16); P02686-5 (SEQ ID NO:17); P02686-6 (SEQ ID NO:18); and P02686-7 (SEQ ID NO:19).

### DETAILED DESCRIPTION OF INVENTION

### I. Introduction

Multiple sclerosis (MS) is a severe neurodegenerative disease having an autoimmune background. Although several treatments for managing multiple sclerosis are known, a cure for the disease does not exist. Furthermore, current therapies have limited efficacy and may result in unwanted side-effects. Accordingly, the development of novel approaches for MS treatment is of great importance. We report here compositions and use of B cell epitopes of myelin basic protein (MBP) encapsulated in small unilamellar (SUV) mannosylated liposomes as an effective drug for experimental autoimmune encephalomyelitis (EAE) treatment in DA rats, an art accepted model for human MS.

In one aspect, the present invention provides therapeutic compositions of antigenic MBP peptides linked to a vector, which are useful for the treatment of multiple sclerosis. In a specific embodiment, the therapeutic compositions comprise one, two, or three antigenic MBP peptides linked to a vector (*e.g*., a liposome) optionally comprising a targeting moiety (*e.g*., a mannosylated lipid). When administered to a patient with multiple sclerosis, the therapeutic compositions improve cognitive abilities and relieve symptoms of paralysis. Accordingly, the present invention also provides methods for the treatment, management, and prophylaxis of multiple sclerosis in a subject in need thereof.

Using a myelin basic protein epitope library, the binding pattern of serum autoantibodies (autoAb) of relapsing-remitting MS patients was analyzed and compared to anti-MBP autoAb from Swiss James Lambert (SJL) mice, C57 black 6 (C57BL/6) mice, and Dark Agouti (DA) rats with EAE. It was found that DA rats with EAE are the most relevant rodent models of MS based on the spectra of autoAb to MBP fragments. Three immunodominant fragments of MBP encapsulated in mannosylated SUV liposomes were used for EAE treatment in DA rats. MBP(46-62) was the most effective in decreasing the maximal disease score during first attack, whereas MBP(124-139) and MBP(147-170) prevented the development of an exacerbation stage. Administration of a mixture of immunodominant MBP peptides entrapped into liposomes significantly ameliorates protracted EAE by down-regulation of Th1 cytokines and induction of BDNF production in CNS. Synergistic effects of MBP peptides decrease overall disease course with moderate first attack and fast outcome from exacerbation, suggesting a novel therapeutic modality for MS treatment.

### II. Definitions

As used herein, the term "vector" refers to a molecular structure capable of associating with a cargo (e.g., therapeutic or diagnostic small molecules, peptides, nucleic acids, and protein biologics). In one embodiment, a vector is a molecular structure that harbors or shepherds a therapeutic cargo (*e.g*., an MBP peptide) administered to a subject in need thereof. A vector can, but does not necessarily: improve a therapeutic effect imparted by the cargo; improve or target delivery of the cargo to an *in vivo* location or cell type; improve the uptake of the cargo into cells or particular cells *in vitro* or *in vivo*; increase the *in vivo* half-life of the cargo; shield the cargo from unwanted *in vivo* interactions; or reduce the clearance rate of the cargo from the blood-stream and/or body of a subject. In one embodiment, the vector comprises a nanoparticle, as defined below, capable of encapsulating, embedding, tethering the cargo. Optionally, the vector, *e.g*., the nanoparticle vehicle, may further comprise a targeting moiety. In another embodiment, the vector is a targeting moiety that is directly linked (covalently or non-covalently) to the cargo. Non-limiting examples of vectors include: nanoparticles, such as liposomes, micelles, block copolymer micelles, polymersomes, niosomes, lipid-coated nanobubbles, and dendrimers; solid carriers, such as metallic particles and silica particles; sugar moieties, such as mannose, a mannose derivative, a mannose analog, or a carbohydrate containing one or more mannose residues, mannose derivatives, or mannose analog; peptides, such as a cell receptor ligand; polypeptides, such as an antibody or functional fragment thereof; and nucleic acids, such as an aptamer or Spiegelmer®.

As used herein, the term "targeting moiety" refers to an agent that improves the efficacy of a therapeutic or diagnostic cargo when associated with the cargo, as compared to the efficacy of the cargo alone. In one embodiment, a targeting moiety improves the delivery of the associated cargo to an *in vivo* location or cell type; and/or improves the uptake of the cargo into a cell or location *in vivo.* The targeting moiety can be covalently or non-covalently linked to the cargo (*e.g*., an MBP peptide), including through a covalent bond, ionic bond, electrostatic interaction, hydrophobic interaction, or physical entrapment. In certain embodiments, the linkage can be mediated through a linker or other vector structure. Examples of targeting moieties include a sugar moiety (*e.g*., mannose or a carbohydrate containing one or more mannose residues), a peptide *(e.g.,* a cell receptor ligand), a polypeptide (*e.g*., an antibody or functional fragment thereof), and a nucleic acid (*e.g*., an aptamer or Spiegelmer®).

As used herein, the term "vector comprising a targeting moiety" refers to a molecular structure that improves the delivery of a cargo to a cell and/or improves intake of the cargo into the cell. In one embodiment, the vector comprises a targeting moiety that is covalently or non-covalently linked to a nanoparticle vehicle capable of carrying a cargo (e.g., an MBP peptide or other therapeutic agent). Optionally, the vector comprising a targeting moiety includes a nanoparticle vehicle capable of harboring the cargo. In another embodiment, a vector comprising a targeting moiety consists of a targeting moiety that is directly linked, covalently or non-covalently, to a cargo (*e.g*., an MBP peptide or other therapeutic agent). In a specific embodiment, a vector comprising a targeting moiety is the targeting moiety.

As used herein, the term "nanoparticle" refers to a vector with an average diameter of from about 1 nm to about 1000 nm, which is linked to cargo, for example a peptide (*e.g*., an MBP peptide), nucleic acid, therapeutic moiety, or diagnostic moiety. Nanoparticles can be hollow (*e*.*g*., having an outer shell and a hallow core), solid, or multi-layered. The cargo (*e.g*., an MBP peptide) may be tethered to, embedded in, or encapsulated by the nanoparticle. Many nanoparticles are known in the art (see, for example, Elizondo et al., Prog Mol Biol Transl Sci. 2011;104:1-52) and include a liposome, a micelle, a block copolymer micelle (reviewed in Kataoka et al., Adv Drug Deliv Rev. 2001 Mar 23;47(1):113-31), a polymersome (reviewed in Christian et al., Eur J Pharm Biopharm. 2009 Mar; 71(3):463-74), a niosome (reviewed in Kazi et al., J Adv Pharm Technol Res. 2010 Oct; 1(4):374-80), a lipid-coated nanobubble (Unger et al., Adv Drug Deliv Rev. 2004 May 7;56(9):1291-314), a dendrimer, a metallic particle (for example, an iron oxide particle or gold particle), and a silica particle.

In one embodiment, a nanoparticle has an average diameter of from about 1 to about 1000 nm. In another embodiment, a nanoparticle has an average diameter of from about 20 to about 500 nm. In another embodiment, a nanoparticle has an average diameter of from about 50 to about 400 nm. In another embodiment, a nanoparticle has an average diameter of from about 75 nm to about 300 nm. In yet other embodiment, a nanoparticle has an average diameter of from about 100 nm to about 200 nm. In certain embodiments, liposomes may include cationic lipids, anionic lipids, zwitterionic lipids, neutral lipids, or combinations thereof.

As used herein, the term "liposome" refers to any structure enclosed by a lipid bilayer (i.e., lamella). The term liposome encompasses multilamellar vesicle (MLV) liposomes ranging in size from about 0.1 µm to about 5 µm, small unilamellar vesicle (SUV) liposomes ranging in size from about 0.02 µm to about 0.05 µm, and large unilamellar vesicle liposomes ranging in size from about 0.06 µm and up. As used herein, the term "multilamellar" refers to a lipid structure containing more than two lipid layers. Accordingly, the term "unilamellar" refers to a lipid structure containing two lipid layers, *i.e*., a single lipid bilayer. Generally, when present in an aqueous environment, the hydrophilic portion (*e.g*., lipid polar headgroups) of the majority of lipids comprising a lipid bilayer will be located on the surface of the structure (*i.e*., the outer or inner face of the bilayer and the hydrophobic portions (*e*.*g*., saturated or unsaturated hydrocarbon groups) of the majority of lipids comprising a lipid bilayer will be located in the interior of the bilayer.

As used herein, the term "micelle" refers to any structure enclosed by a lipid monolayer. Generally, when present in an aqueous environment, the hydrophilic portion (*e.g*., lipid polar headgroups) of the majority of lipids comprising a micelle will be located on the surface of the structure and the hydrophobic portions (*e*.*g*., saturated or unsaturated hydrocarbon groups) of the majority of lipids comprising a micelle will be located in the interior of the structure. In certain embodiments, a liposome may be encapsulated within a larger micelle. Likewise, in certain embodiments, a micelle may be encapsulated within a larger liposome.

As used herein, the term "mannosylated liposome" refers to a liposome comprising one or more mannose residues, mannose derivative, or mannose analog, associated with the exterior of the lipid bilayer. In one embodiment, a mannosylated liposome comprises a lipid conjugated to one or more mannose residues, derivatives, or analogs. In a specific embodiment, the mannose residue, derivative, or analog will be conjugated to a polar head group or other lipid structure generally located on the external side of a lipid bilayer present in an aqueous environment (*e.g*., the external and/or internal surface of a liposome). Preferably, at least a percentage of the mannose residues, derivatives, or analogs conjugated to a mannosylated liposome will be exposed to the external environment of the liposome and thus be accessible to interact, for example, with immune cells. In one embodiment, a mannosylated liposome comprises a mono-mannosylated lipid. In a specific embodiment, the mono-mannosylated lipid is ManDOG lipid (*see*, Ponpipom, M. M. et al., J. Med. Chem. 1981, 24, 1388; and Espuelas et al., Bioorg Med Chem Lett. 2003 Aug 4;13(15):2557-60). The structure of a ManDOG lipid is provided in Figure 3. In another embodiment, a mannosylated liposome comprises a tetramannosyl-3-L-lysine-dioleoyl glycerol lipid (Espuelas *et al*., *supra*). Non-limiting examples of mannose derivatives and analogs include 1-deoxymannojirimycin, methyl-a-D-mannopyranoside, 2-deoxy-D-glucose (2-DG), 2-deoxy-2-fluoro-mannose (2-FM), and 2-deoxy-2-chloro-mannose (2-CM), any of which may be conjugated to a lipid.

In certain embodiments, at least 0.01% of the lipids comprising a mannosylated liposome will be conjugated to at least one mannose residue. In another embodiment, at least 0.1% of the lipids comprising a mannosylated liposome will be conjugated to at least one mannose residue. In another embodiment, at least 1% of the lipids comprising a mannosylated liposome will be conjugated to at least one mannose residue. In yet other embodiments, at least 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% of the lipids comprising a mannosylated liposome will be conjugated to at least one mannose residue.

As used herein, the term "lipid" refers to hydrophobic or amphiphilic molecules capable of forming monolayer or bilayer structures in an aqueous environment, *e.g*., a micelle or liposome. Lipids include fats, waxes, sterols, fat-soluble vitamins, monoglycerides, diglycerides, triglycerides, and phospholipids. Lipids used to form nanoparticles, such as liposomes and micelles, may be further modified or conjugated to a targeting moiety. Non-limiting examples of targeting moieties that may be conjugated to lipids include: sugar moieties (*e.g*., mannose or a carbohydrate containing one or more mannose residues), peptides (*e.g*., a cell receptor ligand), polypeptides *(e.g.,* an antibody or functional fragment thereof), and nucleic acids (*e.g*., an aptamer or Spiegelmer®).

As used herein, the term "cholesterol" refers to a naturally-occurring steroid alcohol (sterol) having four fused rings, as well as its esters with long chain fatty acids, and analogues thereof that retain the ability to modulate membrane fluidity. Cholesterol and cholesterol esters are components of plasma lipoproteins and the outer cell membrane of animal cells, and have the ability to modulate membrane fluidity. Cholesterol analogues that retain the ability to modulate membrane fluidity are known in the art (see, *e.g*., Gimpl, G., et al. (1997) Biochemistry 36:10959-10974) and include, for example, 5-cholestene, 5-pregnen-3β-ol-20-one, 4-cholesten-3-one and 5-cholesten-3-one. Cholesterol and cholesterol analogues are common liposomal components that can impart additional fluidity into a lipid monolayer or bilayer forming a micelle or liposome.

As used herein, the terms "linked" and "conjugated" are used interchangeably and refer to a covalent or non-covalent association between two moieties, for example, between a therapeutic agent and a vector or targeting moiety. Linkages formed between the two moieties, although not necessarily covalent in nature, help to maintain the association between the moieties. Non-limited examples of linkages that may be used to associate two moieties, for example an MBP peptide and a targeting moiety, include: covalent interactions (*i.e*., a covalent chemical bond formed either directly between the first moiety and the second moiety or through a linker molecule); ionic interactions (*e.g*., an ionic bond formed either directly between the first moiety and the second moiety or through a linker molecule); electrostatic interactions (*e.g*., attraction of two opposite charges); hydrophobic interactions; interactions held together via Van der Waals forces; and interactions held together through physical entrapment (*e.g*., encapsulation or embedding of a cargo molecule within a nanoparticle). In one embodiment, a cargo molecule (*e.g*., an MBP peptide) encapsulated within a vector (*e.g*., a liposome) is linked to a targeting moiety (*e.g*., a mannose residue) that is tethered to the exterior of the vector.

As used herein, the term "embedded within" refers to the positioning of a cargo molecule with respect to a vector, in which the cargo molecule is located within the matrix of the vector structure. For example, a peptide cargo is said to be embedded within a liposomal or micelle vector when the peptide, or a portion thereof, is located within a lipid bilayer (liposome) or monolayer (micelle). Cargo molecules embedded within a vector can be covalently or non-covalently associated with the vector matrix (*e.g*., a polymeric shell) or a sub-component of the vector matrix (*e.g.,* a lipid present in a lipid bilayer of a liposome), for example, through a covalent bond, ionic bond, electrostatic interaction, hydrophobic interaction, or physical entrapment.

As used herein, the term "encapsulated in" refers to the positioning of a cargo molecule with respect to a vector, in which the cargo molecule is enclosed or contained within the inside of a vector structure. For example, a peptide cargo is said to be encapsulated in a liposomal vector when the peptide is located internal to a lipid bilayer of the liposome, thereby shielded from the environment external to the liposome. Cargo molecules encapsulated in a vector can be covalently or non-covalently associated with the vector (*e.g*., a polymeric shell) or a sub-component of the vector matrix (*e.g*., a lipid present in a lipid bilayer of a liposome), for example, through a covalent bond, ionic bond, electrostatic interaction, hydrophobic interaction, or physical entrapment.

In certain embodiments, the interior of a liposomal or micelle vector will comprise an aqueous environment. Accordingly, a hydrophilic cargo, such as a peptide or nucleic acid, may be partially or completely solvated within the interior of the vector. In other embodiments, the interior of a liposomal or micelle vector may comprise a non-aqueous environment, for example it may consist of a polar solvent. Accordingly, a hydrophobic cargo, such as a non-polar small molecule, may be partially or completely solvated within the interior of the vector.

As used herein, the term "tethered to" refers to the positioning of a cargo molecule with respect to a vector, in which the cargo molecule is linked to the vector structure at one or more points. Tethering of a cargo molecule can be done covalently (*e.g*., through a chemical bond) or non-covalently (*e.g*., through nucleic acid hybridization). Cargo molecules can be tethered to the exterior or interior of a vector (*e.g*., a polymeric shell) or a sub-component of the vector matrix (*e.g*., a lipid present in a lipid bilayer of a liposome). A cargo molecule tethered to a vector structure at a point of attachment may otherwise be free to move about space (*e.g*., otherwise solvated by the environment external or internal to the vector). Cargo molecules tethered to a vector can be covalently or non-covalently associated with the vector (*e.g*., a polymeric shell) or a sub-component of the vector matrix (*e.g*., a lipid present in a lipid bilayer of a liposome), for example, through a covalent bond, ionic bond, electrostatic interaction, or hydrophobic interaction.

As used herein, the terms "aptamer," "SPIEGELMER®," and "nucleic acid ligand" are used interchangeably and refer to a non-naturally occurring oligonucleotide (typically 15 to 250 nucleotides long) that specifically binds to a particular target. Aptamers are nucleic acids comprising a specific secondary structure that imparts specificity for a target molecule (*e.g*., a cell surface marker or receptor). Aptamers may further comprise a specific ternary, and possibly quaternary, structure that further contributes to the affinity between the nucleic acid and target molecule. When present in a proper three-dimensional structure, an aptamer specifically binds to a particular target. Aptamers encompass sequences of natural nucleic acids (*e.g*., dA, dT, dC, dG, rA, rU, rC, and rG), as well as non-natural nucleic acids (*e.g*., dU, dI, rT, rI) and modified nucleic acids. SPIEGELMERs® are aptamers formed with L-nucleic acids, rather than the naturally occurring D-nucleic acids. Aptamers and SPIEGELMERs® may include a mixture of L- and D-nucleic acids.

As used herein, the term "antibody" refers to a polypeptide that is immunologically reactive with a particular antigen. The term "immunoglobulin," as used herein, encompasses intact molecules of various isotypes as well as fragments with antigen-binding capability, *e.g*., Fab', F(ab')2, Fab, Fv and rlgG. *See, e.g.,* Pierce Catalog and Handbook, 1994-1995 (Pierce Chemical Co., Rockford, III.); Kuby, J., Immunology, 3rd Ed., W.H. Freeman & Co., New York (1998). The term also encompasses recombinant single chain Fv fragments (scFv). The term further encompasses bivalent or bispecific molecules, diabodies, triabodies, and tetrabodies. Bivalent and bispecific molecules are described in, *e.g*., Kostelny et al. (1992) J. Immunol. 148:1547; Pack and Pluckthun (1992) Biochemistry 31:1579; Hollinger et al., 1993, Proc Natl Acad Sci USA. 1993 Jul 15;90(14):6444-8; Gruber et al., (1994) J. Immunol. 5368; Zhu et al., (1997) Protein Sci 6:781; Hu et al., (1996) Cancer Res. 56:3055. The term antibody also encompasses, for example, human antibodies, humanized antibodies, and chimeric antibodies.

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g*., an enzyme, toxin, hormone, growth factor, drug, and the like; or (b) the variable region, or a portion thereof, is altered, replaced or exchanged with a variable region having a different or altered antigen specificity.

A "humanized antibody" is an immunoglobulin molecule that contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al., Nature 321:522-525 (1986); Riechmann et al., Nature 332:323-329 (1988); and Presta, Curr. Op. Struct. Biol. 2:593-596 (1992)). Humanization can be essentially performed following the method of Winter and co-workers (Jones et al., Nature 321:522-525 (1986); Riechmann et al., Nature 332:323-327 (1988); and Verhoeyen et al., Science 239:1534-1536 (1988)), by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species.

As used herein, the term "specifically binds" refer to a molecule (*e.g*., a targeting moiety) that binds to a particular target (*e.g*., a cell-surface marker or receptor) with at least 2-fold greater affinity, as compared to a non-targeted molecule. In certain embodiments, a molecule specifically binds with at least 3-fold, 4-fold, 5-fold, 6-fold, 7-fold, 8-fold, 9-fold, 10-fold, 25-fold, 50-fold, 100-fold, 500-fold, 1000-fold, 5000-fold, 10000-fold, or greater affinity, as compared to a non-targeted molecule.

As used herein, the term "immune cells" refers to cells that are of hematopoietic origin and that play a role in the immune response. Immune cells include lymphocytes, such as B cells and T cells; natural killer cells; myeloid cells, such as monocytes, macrophages, eosinophils, mast cells, basophils, and granulocytes.

As used herein, the term "B cell" refers to a lymphocyte produced in the bone marrow of most mammals, which function in the humoral immune system. During the various stages of development, B cells are referred to as: progenitor (or pre-pro) B cells; early pro (or pre-pre)-B cells; late pro (or pre-pre)-B cells; large pre-B cells; small pre-B cells; immature B cells; and mature B cells, each of which are encompassed by the term B cell. Phenotypic cell surface markers that can be used to differentiate B cells from other lymphocytes (*e.g*., T cells) include: MHC class II molecules, CD19, and CD21. The term "B cell" encompasses plasma B cells, memory B cells, B-1 cells, B-2 cells, marginal-zone B cells, and follicular B cells.

As used herein, the terms "antigen presenting cell" and "APC" are used interchangeably and refer to dedicated antigen presenting cells (*e.g*., B lymphocytes, monocytes, dendritic cells, Langerhans cells), as well as other antigen presenting cells (*e.g*., keratinocytes, endothelial cells, astrocytes, fibroblasts, and oligodendrocytes).

As used herein, the terms "cell-surface marker," "cell-surface receptor," and "cell surface molecules" refer to an antigenic structure present on the surface of a cell. The cell surface antigen may be a cell-specific antigen, an immune cell-specific antigen, a B cell-specific antigen, an antigen presenting cell-specific antigen, a lymphocyte-specific antigen, an antigen associated with multiple sclerosis, a receptor (*e.g*., a growth factor receptor), a surface epitope, an antigen which is recognized by a specific immunological effector cell such as a T-cell, and an antigen that is recognized by a non-specific immunological effector cell such as a macrophage cell or a natural killer cell. Examples of "cell surface antigens" include phenotypic markers of: NK cells (*e.g*., CD16 and CD56); helper T cells (*e.g*., TCRαβ, CD3, and CD4); cytotoxic T cells (*e.g*., TCRαβ, CD3, and CD8); γδ T cells (*e.g*., TCRγδ and CD3); and B cells (MHC class II, CD 19, and CD21). Cell surface molecules may also include carbohydrates, proteins, lipoproteins, glycoproteins, or any other molecules present on the surface of a cell of interest.

As used herein, the terms "myelin basic protein" and "MBP" are interchangeably used and refer to a protein encoded by the human myelin basic protein gene (MBP; NCBI Gene ID: 4155). *In vivo,* multiple isoforms of MBP protein arise from alternative splicing (for review, *see*, Harauz et al., Biochemistry, (2009) Sep 1;48(34):8094-104), each of which is encompassed by the term "myelin basic protein." An alignment of seven representative MBP sequences is provided in Figure 14. As used herein, the amino acid numbering of MBP peptides refers to the amino acid sequence of the predominant isoform of MBP found in mature myelin (splice isoform 5; UniProt ID No.: P02686-5), a 18.5 kDa protein consisting of 171 amino acids (SEQ ID NO:17).

As used herein, the terms "multiple sclerosis," and "MS" are interchangeably used and refers to an inflammatory disease in which the fatty myelin sheaths around the axons of the brain and spinal cord are damaged and/or depleted, leading to demyelination and scarring as well as a broad spectrum of signs and symptoms (for review, *see*, Compston and Coles, Lancet. 2008 Oct 25;372(9648): 1502-17). Several subtypes of MS have been classified, including relapsing remitting (RRMS), secondary progressive (SPMS), primary progressive, (PPMS), and progressive relapsing (PRMS), each of which are encompassed by the term multiple sclerosis.

As used herein, the term "patient" or "subject" are used interchangeably and refer to an individual in need of or seeking treatment. For example, a subject may have been diagnosed with or at risk for developing multiple sclerosis (MS) or a subtype thereof. A multiple sclerosis patient can refer to an individual that has been diagnosed with MS and is receiving treatment for MS, has previously received treatment for MS, or has never received treatment for MS, an individual that is at risk of relapse of MS, or an individual at risk for MS (*e.g*., an individual that is genetically predisposed to MS).

As used herein, the terms "therapy," "treatment" of multiple sclerosis are interchangeably used and refer to any palliation or amelioration of an undesirable physiological or psychological condition resulting from MS. For example, reduction in the severity or frequency of: hypoesthesia; paresthesia; muscle weakness; clonus; muscle spasms; paralysis; ataxia; dysarthria; dysphagia; nystagmus; optic neuritis (e.g., phosphenes or diplopia); fatigue; acute or chronic pain; bladder and bowel difficulties; cognitive impairment; depression; Uhthoff's phenomenon; and Lhermitte's sign. In one embodiment, the Expanded Disability Status Scale (EDSS) can be used as a measure of disease progression and severity in patients with MS (see, Kurtzke JF, Neurology 1983;33:1444-52). Accordingly, in one embodiment, therapy refers to an act of improving the EDSS of a patient.

As used herein, the terms "prevention," and "prophylactic treatment" of multiple sclerosis are interchangeably used and refer to therapeutic treatments that reduce the risk, severity, or onset of clinical symptoms of MS. The prophylaxis may be partial or complete. Partial prophylaxis may result in the delayed onset or delayed progression of a disease state or symptom in a patient at risk for developing MS or incurring relapse of MS. Although no strict causative genetic component of MS has been identified, several genetic factors correlated with an increased risk of developing multiple sclerosis have been identified, including SNPs identified in Hafler DA et al. (N Engl J Med. 2007 Aug 30;357(9):851-62) such as rs3135388 (A allele; HLA-DRA gene), rs12722489 (C allele; IL2RA gene), rs2104286 (T allele; IL2RA gene), rs6897932 (C allele; IL7R gene), rs6498169 (G allele; KIAA0350), rs6604026 (C allele; RPL5), rs10984447 (A allele; DBC1 gene), rs12044852 (C allele; CD58 gene), rs7577363 (A allele; ALK gene), rs7536563 (A allele; FAM69A gene), rs11164838 (C allele; FAM69A gene), rs10975200 (G allele; ANKRD15 gene), rs10735781 (G allele; EVI5 gene), rs6680578 (T allele; EVI5 gene), rs4763655 (A allele; KLRB1 gene), rs12487066 (T allele; CBLB gene), and rs1321172 (C allele; PDE4B gene).

As used herein, the terms "dose," and "dosage" are interchangeably used and refer to the amount of an active ingredient administered at a single time point. In the context of the present invention, a dose can refer to the amount of an MBP peptide administered to a subject. A dosage can also refer to the amount of a vector preparation of an MBP peptide administered to a subject, for example, a liposomal preparation of one or a combination of MBP peptides. The dosage administered to a patient will vary dependent on a number of factors, including: frequency of administration; severity of the condition (*e.g*., multiple sclerosis); subtype of the condition (*e.g*., relapsing remitting MS, secondary progressive MS, primary progressive MS, and progressive relapsing MS); stage of the condition (*e.g*., initial attack, relapse, and remission); size and tolerance of the subject; route of administration employed; risk of side effects; risk of adverse drug interactions; and response to prior treatments, each of which can be readily determined by a skilled physician. The term "dosage form" refers to the particular format of the pharmaceutical agent, *e.g*., a liquid formulation for subcutaneous administration or gel formulation for controlled release via a depot.

As used herein, the term "therapeutically effective dose," and "therapeutically effective amount" are interchangeably used and refer to a dose that produces effects for which it is administered. The exact dose will depend on the purpose of the treatment, and will be ascertainable by one skilled in the art using known techniques (see, *e.g*., Augsburger & Hoag, Pharmaceutical Dosage Forms (vols. 1-3, 3rd Ed. 2008); Lloyd, The Art, Science and Technology of Pharmaceutical Compounding (3rd Ed., 2008); Pickar, Dosage Calculations (8th Ed., 2007); and Remington: The Science and Practice of Pharmacy, 21st Ed., 2005, Gennaro, Ed., Lippincott, Williams & Wilkins).

As used herein, the term "pharmaceutical composition" refers to a formulation suitable for administration to a subject containing a therapeutic agent and optionally one or more of the following: a vector; a targeting moiety; a buffering agent; a salt; a preservation agent (*e.g*., an antioxidant or anti-microbial agent); an osmotic agent; a bulking agent; and any other excipient or carrier suited for delivery of the therapeutic agent via a particular route of administration.

As used herein, the term "control" refers to a sample, level, or phenotypic result that serves as a reference for comparison to a test result, for example, a therapeutic benefit achieved by a particular treatment. The term control encompasses both positive controls (*e.g*., a value or results that is expected for a given therapy) and negative controls (*e.g*., a value or result that would be expected in the absence of treatment).

A positive control can refer to a result achieved by the administration of a therapeutic agent known to provide a beneficial effect for a disease state or symptom. For example, the result achieved by the administration of copaxone to a subject diagnosed with MS, or an animal model of MS, can be used as a positive control for an experimental MS therapy. In this sense, an experimental therapy that results in a similar or better outcome, as compared to the result achieved with the administration of copaxone, would be considered a good candidate for the treatment of MS.

A negative control can refer to a result achieved in the absence of treatment for a disease state or symptom. For example, the administration of water or empty vector to a subject diagnosed with MS, or an animal model of MS, can be used as a negative control for an experimental MS therapy. In this sense, an experimental therapy that results in a similar result as achieved with the negative control would not be considered a good candidate for the treatment of MS. Whereas, an experimental therapy that results in a better outcome, as compared to the result achieved with the negative control, would be considered a good candidate for the treatment of MS.

As used herein, the terms "nucleic acid molecule, "oligonucleotide," and "polynucleotide" are interchangeably used and refer to a deoxyribonucleotide or ribonucleotide polymer in either single-stranded or double-stranded form, and, unless specifically indicated otherwise, encompasses polynucleotides containing known analogs of naturally occurring nucleotides that can function in a similar manner as naturally occurring nucleotides. It will be understood that when a nucleic acid molecule is represented by a DNA sequence, this also includes RNA molecules having the corresponding RNA sequence in which "U" (uridine) replaces "T" (thymidine).

As used herein, the terms "protein," "peptide," and "polypeptide" are used interchangeably and refer to a polymer of four or more amino acid residues. The terms apply to amino acid polymers in which one or more amino acid residue is an artificial chemical analogue of a corresponding naturally occurring amino acid, as well as to naturally occurring amino acid polymers. The term "recombinant peptide" refers to a peptide that is produced by expression of a nucleotide sequence encoding the amino acid sequence of the peptide from a recombinant DNA molecule.

As used herein, the term "synthetic peptide" refers to a peptide that is produced by chemical means, *e.g*., by liquid-phase or solid-phase peptide synthesis. Synthetic peptides include amino acid polymers in which one or more amino acid residue is an artificial chemical analogue of a corresponding naturally occurring amino acid, as well as to naturally occurring amino acid polymers.

As used herein, the term "amino acid" refers to naturally occurring and non-natural amino acids, including amino acid analogs and amino acid mimetics that function in a manner similar to the naturally occurring amino acids. Naturally occurring amino acids include those encoded by the genetic code, as well as those amino acids that are later modified, *e.g*., hydroxyproline, γ-carboxyglutamate, O-phosphoserine, 5-hydroxytryptophan, lanthionine. Naturally occurring amino acids can include, *e.g*., D- and L-amino acids. The amino acids used herein can also include non-natural amino acids. Amino acid analogs refer to compounds that have the same basic chemical structure as a naturally occurring amino acid, *i.e*., any carbon that is bound to a hydrogen, a carboxyl group, an amino group, and an R group, *e.g*., homoserine, norleucine, methionine sulfoxide, or methionine methyl sulfonium. Such analogs have modified R groups (*e.g*., norleucine) or modified peptide backbones, but retain the same basic chemical structure as a naturally occurring amino acid. Amino acid mimetics refer to chemical compounds that have a structure that is different from the general chemical structure of an amino acid, but that function in a manner similar to a naturally occurring amino acid. Amino acids may be referred to herein by either their commonly known three letter symbols or by the one-letter symbols recommended by the IUPAC-IUB Biochemical Nomenclature Commission.

As to amino acid sequences, one of ordinary skill in the art will recognize that individual substitutions, deletions or additions to a nucleic acid or peptide sequence that alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M). See, e.g., Creighton, Proteins (1984).

As used herein, the terms "identical" and "identity" when used in reference to two or more polynucleotide sequences or two or more polypeptide sequences, refers to the residues in the sequences that are the same when aligned for maximum correspondence. When percentage of sequence identity is used-in reference to a peptide, it is recognized that one or more residue positions that are not otherwise identical can differ by a conservative amino acid substitution, in which a first amino acid residue is substituted for another amino acid residue having similar chemical properties such as a similar charge, or hydrophobic or hydrophilic character and, therefore, does not substantially change the functional properties of the peptide. Where peptide sequences differ in conservative substitutions, the percent sequence identity can be adjusted upwards to correct for the conservative nature of the substitution. Such an adjustment can be made using well known methods, for example, scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions can be calculated using any-well known algorithm (see, for example, Meyers and Miller, Comp. Appl. Biol. Sci. 4:11-17, 1988; Smith and Waterman, Adv. Appl. Math. 2:482, 1981; Needleman and Wunsch, J. Mol. Biol. 48:443, 1970; Pearson and Lipman, Proc. Natl. Acad. Sci., USA 85:2444 (1988); Higgins and Sharp, Gene 73:237-244, 1988; Higgins and Sharp, CABIOS 5:151-153; 1989; Corpet et al., Nucl. Acids Res. 16:10881-10890, 1988; Huang, et al., Comp. Appl. Biol. Sci. 8:155-165, 1992; Pearson et al., Meth. Mol. Biol., 24:307-331, 1994). Alignment also can be performed by simple visual inspection and manual alignment of sequences.

It will be recognized that individual substitutions, deletions or additions that alter, add or remove a single amino acid or a small percentage of amino acids (*e.g*., less than 15%, less than 10%, or less than 5%) in a peptide sequence can be considered conservatively modified variations, provided alteration results in the substitution of an amino acid with a chemically similar amino acid.

Conservative amino acid substitutions providing functionally similar amino acids are well known in the art. Dependent on the functionality of the particular amino acid, *i.e*., catalytically important, structurally important, sterically important, different groupings of amino acid may be considered conservative substitutions for each other. Table 1 provides groupings of amino acids that are considered conservative substitutions based on the charge and polarity of the amino acid, the hydrophobicity of the amino acid, the surface exposure/structural nature of the amino acid, and the secondary structure propensity of the amino acid.

**Table 1. Groupings of conservative amino acid substitutions based on the functionality of the residue in the protein.**

| Important Feature | Conservative Groupings |
|---|---|
| Charge/Polarity | 1. H, R, and K |
| | 2. D and E |
| | 3. C, T, S, G, N, Q, and Y |
| | 4. A, P, M, L, I, V, F, and W |
| Hydrophobicity | 1. D, E, N, Q, R, and K |
| | 2. C, S, T, P, G, H, and Y |
| | 3. A, M, I, L, V, F, and W |
| Structural/Surface Exposure | 1. D, E, N, Q, H, R, and K |
| | 2. C, S, T, P, A, G, W, and Y |
| | 3. M, I, L, V, and F |
| Secondary Structure Propensity | 1. A, E, Q, H, K, M, L, and R |
| | 2. C, T, I, V, F, Y, and W |
| | 3. S, G, P, D, and N |
| Evolutionary Conservation | 1 . D and E |
| | 2. H, K, and R |
| | 3. N and Q |
| | 4. Sand T |
| | 5. L, I, and V |
| | 6. F, Y, and W |
| | 7. A and G |
| | 8. M and C |

Two or more amino acid sequences or two or more nucleotide sequences are considered to be "substantially identical" if the amino acid sequences or the nucleotide sequences share at least 60% sequence identity with each other, or with a reference sequence over a given comparison window. Thus, substantially identical sequences include those having, for example, at least 60% sequence identity, at least 65% sequence identity, at least 70% sequence identity, at least 75% sequence homology, at least 80% sequence homology, at least 85% sequence identity, at least 90% sequence identity, at least 95% sequence identity, or at least 99% sequence identity. In certain embodiments, substantially identical sequences will have at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity. In one embodiment, the sequences share from 60% to 95% sequence identity. In another embodiment, the sequences share from 65% to 90% sequence identity. In another embodiment, the sequences share from 70% to 85% sequence identity. In yet other embodiments, the sequences share from 65% to 95% sequence identity, from 70% to 95% sequence identity, from 75% to 95% sequence identity, from 80% to 95% sequence identity, from 85% to 95% sequence identity, or from 90% to 95% sequence identity.

In certain embodiments, two polypeptides will be considered substantially identical if they share identical or nearly identical core peptide sequences that are effective to provide therapeutic benefit. For example, where a first core peptide sequence provides therapeutic benefit regardless of presence of additional amino acids located upstream (*i.e*., N-terminal to the core sequence) or downstream (*i.e*., C-terminal to the core sequence), a polypeptide comprising a second core peptide sequence sharing at least 80% identity with the first core peptide sequence may be considered substantially identical. This is true even when the entire polypeptide sequence does not share at least 80% identity with the reference sequence. For example, two therapeutic polypeptides having the amino acid sequences: (R¹)ₐ-P₁-(R²)_{b} and (R³)_{c}-P₂-(R⁴)_{d}, may be considered substantially identical if (i) P₁ and P₂ are at least 80% identical, and (ii) P₁ and P₂ are sufficient to provide a therapeutic benefit to a subject in need thereof, regardless of the amino acid sequences of R¹, R², R³, and R⁴.

In certain embodiments, substantially identical core peptide sequences will share at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity. In a specific embodiment, the core peptide sequences will share at least 85% sequence identity (i.e., from 85% to 100% identity). In another specific embodiment, the core peptide sequences will share at least 90% sequence identity (*i.e*., from 90% to 100% identity). In another specific embodiment, the core peptide sequences will share at least 95% sequence identity (*i.e*., from 95% to 100% identity). In yet another specific embodiment, the core peptide sequences will share 100% sequence identity, regardless of the overall sequence identity of two polypeptides being compared. In one embodiment, the core peptide sequences share from 60% to 95% sequence identity. In another embodiment, the core peptide sequences share from 65% to 90% sequence identity. In another embodiment, the core peptide sequences share from 70% to 85% sequence identity. In yet other embodiments, the core peptide sequences share from 65% to 95% sequence identity, from 70% to 95% sequence identity, from 75% to 95% sequence identity, from 80% to 95% sequence identity, from 85% to 95% sequence identity, or from 90% to 95% sequence identity.

As used herein, the term "comparison window" refers to a contiguous stretch of amino acids or nucleotides over which the sequence of two polypeptides or polynucleotides are compared for sequence identity or similarity. With respect to therapeutic peptides, a comparison window may be from about 5 amino acids to about 50 amino acids long. In one embodiment, a comparison window may be from about 5 amino acids to about 25 amino acids long. In yet another embodiment, a comparison window may be from about 10 to about 20 amino acids long. Depending on factors, such as the length of the polypeptides being compared, the comparison window may be, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more amino acids long. In a particular embodiment, the comparison window is the whole length of a reference amino acid sequence.

Alignment of sequences may be conducted, for example, by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (i.e., resulting in the highest percentage of homology over the comparison window) generated by the various methods selected. The BLAST algorithm is well suited for determining percent sequence identity and sequence similarity (Altschul et al., J Mol. 215:403-410, (1990)). Several software programs incorporating the BLAST algorithm are publicly available through the National Center for Biotechnology Information (NCBI) website. These programs include the blastp, blastn, blastx, tblastn, tblastx, and PSI-blast software programs.

### III. MBP Peptides

### A. Introduction

In one aspect, the present invention provides peptides of myelin basic protein (MBP) useful for treating or preventing relapse of multiple sclerosis (MS). As shown in Figure 1C, two immunodominant regions of MBP were identified in an EAE-induced DA rat model of MS, which correlate with the immunological response to MBP in human patients diagnosed with relapsing-remitting multiple sclerosis (RRMS): MBP(43-64) and MBP(115-170). It was found that polyclonal IgG autoantibodies to these regions were generated in the EAE-induced DA rat model (Figure 2), suggesting that these regions contain B cell epitopes important to the pathology of MS. Accordingly, peptides comprising part or all of these regions may be useful for the treatment or prevention of MS. In a specific embodiment, the MBP peptide comprises a B-cell epitope.

In one aspect of the invention, peptides comprising amino acid sequences identical or substantially identical to the identified immunodominant regions of MBP are provided for the treatment of MS. In a specific embodiment, MBP peptides comprising at least 6 consecutive amino acids of MBP(43-64) (SEQ ID NO:11) or MBP(115-170) (SEQ ID NO:12) are provided for the treatment of MS. Generally, a MBP peptide will consist of from 6 to 100 amino acids in length. In one embodiment, the MBP peptide will consist of from 6 to 50 amino acids in length. In another embodiment, the MBP peptide will consist of from 6 to 25 amino acids in length. In yet other embodiments, the MBP peptide will consist of about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more amino acids. In a specific embodiment, an MBP peptide consists of from 6 to 40 amino acids in length.

In one embodiment, an MBP peptide comprises at least 10 consecutive amino acids of MBP(43-64) (SEQ ID NO:11) or MBP(115-170) (SEQ ID NO:12). In another embodiment, an MBP peptide comprises at least 15 consecutive amino acids of MBP(43-64) (SEQ ID NO:11) or MBP(115-170) (SEQ ID NO:12). In other embodiments, an MBP peptide comprises at least 7, 8, 9, 10, 11, 12, 13, 14, 15, 16, 17, 18, 19, 20, 21, or 22 consecutive amino acids of MBP(43-64) (SEQ ID NO:11) or MBP(115-170) (SEQ ID NO:12). In yet other embodiments, an MBP peptide comprises at least 23, 24, 25, 26, 27, 28, 29, 30, 31, 32, 33, 34, 35, 36, 37, 38, 39, 40, or more consecutive amino acids of MBP(115-170) (SEQ ID NO:12). In one embodiment, the MBP peptide is linked to a vector, which includes a targeting moiety. In a specific embodiment, the vector is a liposome. In a more specific embodiment, the liposome is a mannosylated liposome.

In another aspect, an MBP peptide comprises from 6 to 25 consecutive amino acids of MBP(43-64) (SEQ ID NO:11) or MBP(115-170) (SEQ ID NO:12). In another embodiment, an MBP peptide comprises from 10 to 20 consecutive amino acids of MBP(43-64) (SEQ ID NO:11) or MBP(115-170) (SEQ ID NO:12). In another embodiment, an MBP peptide comprises from 6 to 40, or from 6 to 35, or from 6 to 30, or from 6 to 20 consecutive amino acids of MBP(115-170) (SEQ ID NO:12). In yet other embodiments, an MBP peptide comprises from 6 to 20, or from 6 to 18, or from 6 to 16, or from 6 to 14, or from 6 to 12, or from 6 to 10, or from 6 to 8 consecutive amino acids of MBP(43-64) (SEQ ID NO:11) or MBP(115-170) (SEQ ID NO:12). In one embodiment, the MBP peptide is linked to a vector. In a specific embodiment, the vector is a liposome. In a more specific embodiment, the liposome is a mannosylated liposome.

In a specific embodiment, the MBP peptide comprises the sequence: GGDRGAPKRGSGKDSHH (MBP(46-62); SEQ ID NO:1). In one embodiment, MBP(46-62) is linked to a vector. In a specific embodiment, the vector is a liposome. In a more specific embodiment, the liposome is a mannosylated liposome.

In another specific embodiment, the MBP peptide comprises the sequence: GFGYGGRASDYKSAHK (MBP(124-139); SEQ ID NO:2). In one embodiment, MBP(124-139) is linked to a vector. In a specific embodiment, the vector is a liposome. In a more specific embodiment, the liposome is a mannosylated liposome.

In another specific embodiment, the MBP peptide comprises the sequence: QGTLSKIFKLGGRDSRSGSPMARR (MBP(147-170); SEQ ID NO:3). In one embodiment, MBP(147-170) is linked to a vector. In a specific embodiment, the vector is a liposome. In a more specific embodiment, the liposome is a mannosylated liposome.

### B. Amino Acid Substitutions

The MBP peptides provided herein may further comprise one or more amino acid substitutions relative to the wild type MBP sequence (SEQ ID NO:17). In one embodiment, the amino acid substitution is a conservative amino acid substitution. For example, amino acids having similar hydrophobicity (*e.g*., Leu and IIe) may be readily substituted for one another. Table 1 provides groupings of amino acids that are considered conservative substitutions based on the charge and polarity of the amino acid, the hydrophobicity of the amino acid, the surface exposure/structural nature of the amino acid, and the secondary structure propensity of the amino acid. In another embodiment, the amino acid substitution is not a conservative amino acid substitution.

In one embodiment, the MBP peptide comprises an amino acid sequence having at least 80% sequence identity to a peptide sequence of at least 6 consecutive amino acids of MBP(43-64) (SEQ ID NO:11) or MBP(115-170) (SEQ ID NO:12). In another embodiment, the MBP peptide comprises an amino acid sequence having at least 85% sequence identity to a peptide sequence of at least 6 consecutive amino acids of MBP(43-64) (SEQ ID NO:11) or MBP(115-170) (SEQ ID NO:12). In another embodiment, the MBP peptide comprises an amino acid sequence having at least 90% sequence identity to a peptide sequence of at least 6 consecutive amino acids of MBP(43-64) (SEQ ID NO:11) or MBP(115-170) (SEQ ID NO:12). In another embodiment, the MBP peptide comprises an amino acid sequence having at least 95% sequence identity to a peptide sequence of at least 6 consecutive amino acids of MBP(43-64) (SEQ ID NO:11) or MBP(115-170) (SEQ ID NO:12). In yet other embodiments, the MBP peptide comprises an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to a peptide sequence of at least 6 consecutive amino acids of MBP(43-64) (SEQ ID NO:11) or MBP(115-170) (SEQ ID NO:12).

In a specific embodiment, the MBP peptide comprises an amino acid sequence that has at least 80% sequence identity to SEQ ID NO:1. In another embodiment, the MBP peptide comprises an amino acid sequence that has at least 85% sequence identity to SEQ ID NO:1. In another embodiment, the MBP peptide comprises an amino acid sequence that has at least 90% sequence identity to SEQ ID NO:1. In another embodiment, the MBP peptide comprises an amino acid sequence that has at least 95% sequence identity to SEQ ID NO:1. In yet other embodiments, the MBP peptide comprises an amino acid sequence that has at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:1.

In a specific embodiment, the MBP peptide comprises an amino acid sequence that has at least 80% sequence identity to SEQ ID NO:2. In another embodiment, the MBP peptide comprises an amino acid sequence that has at least 85% sequence identity to SEQ ID NO:2. In another embodiment, the MBP peptide comprises an amino acid sequence that has at least 90% sequence identity to SEQ ID NO:2. In another embodiment, the MBP peptide comprises an amino acid sequence that has at least 95% sequence identity to SEQ ID NO:2. In yet other embodiments, the MBP peptide comprises an amino acid sequence that has at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:2.

In a specific embodiment, the MBP peptide comprises an amino acid sequence that has at least 80% sequence identity to SEQ ID NO:3. In another embodiment, the MBP peptide comprises an amino acid sequence that has at least 85% sequence identity to SEQ ID NO:3. In another embodiment, the MBP peptide comprises an amino acid sequence that has at least 90% sequence identity to SEQ ID NO:3. In another embodiment, the MBP peptide comprises an amino acid sequence that has at least 95% sequence identity to SEQ ID NO:3. In yet other embodiments, the MBP peptide comprises an amino acid sequence that has at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:3.

### C. Peptide Flanking Regions

Antigen presenting cells (APCs) such as β-cell lymphocytes, dendritic cells, and macrophages stimulate an immune response by internalizing, processing, and presenting processed foreign (*e.g*., in response to a pathogenic infection) or self (*e.g*., in an autoimmune disease) antigens in order to stimulate various types of T-cells. Without being bound by theory, because APCs are capable of internalizing and processing large antigens into smaller antigenic peptides recognized by the T-cell machinery, an MBP peptide, described herein as containing at least 6 consecutive amino acids of MBP(43-64) or MBP(115-170), may have additional amino acids at the N- and or C-terminus, *i.e*., flanking residues.

The MBP flanking residues may include natural flanking regions present in the wild type MBP protein (e.g., amino acids N-terminal to MBP residues 43 and 115 and/or amino acids C-terminal to MBP residues 64 and 170), or alternatively may comprise an exogenous or random sequence. In one embodiment, the N- and/or C-terminal flanking residues may impart a beneficial property to the MBP peptide. For example, flanking amino acid residues may: stabilize the peptide; target the peptide to a specific intracellular or extracellular location; improve the vector loading properties of the peptide; or improve or direct antigen processing or presentation in an immune cell (*e.g.,* a B cell or APC).

In one embodiment, an MBP peptide further comprises from 1 to 50 additional amino acids on the N- and/or C-terminus of the peptide. In another embodiment, the MBP peptide further comprises from 1 to 25 additional amino acids on the N- and/or C-terminus of the peptide. In another embodiment, the MBP peptide further comprises from 1 to 10 additional amino acids on the N- and/or C-terminus of the peptide. In yet other embodiments, the MBP peptide further comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more additional amino acids on the N- and/or C-terminus of the peptide.

In a specific embodiment, an MBP peptide containing the sequence: GGDRGAPKRGSGKDSHH (MBP(46-62); SEQ ID NO:1) further comprises from 1 to 50 additional amino acids on the N- and/or C-terminus of the peptide. In another embodiment, an MBP(46-62) peptide further comprises from 1 to 25 additional amino acids on the N- and/or C-terminus of the peptide. In another embodiment, an MBP(46-62) peptide further comprises from 1 to 10 additional amino acids on the N- and/or C-terminus of the peptide. In yet other embodiments, an MBP(46-62) peptide further comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more additional amino acids on the N- and/or C-terminus of the peptide.

In a specific embodiment, an MBP peptide containing the sequence: GFGYGGRASDYKSAHK (MBP(124-139); SEQ ID NO:2) further comprises from 1 to 50 additional amino acids on the N- and/or C-terminus of the peptide. In another embodiment, an MBP(124-139) peptide further comprises from 1 to 25 additional amino acids on the N- and/or C-terminus of the peptide. In another embodiment, an MBP(124-139) peptide further comprises from 1 to 10 additional amino acids on the N- and/or C-terminus of the peptide. In yet other embodiments, an MBP(124-139) peptide further comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more additional amino acids on the N- and/or C-terminus of the peptide.

In a specific embodiment, an MBP peptide containing the sequence: QGTLSKIFKLGGRDSRSGSPMARR (MBP(147-170); SEQ ID NO:3) further comprises from 1 to 50 additional amino acids on the N- and/or C-terminus of the peptide. In another embodiment, an MBP(147-170) peptide further comprises from 1 to 25 additional amino acids on the N- and/or C-terminus of the peptide. In another embodiment, an MBP(147-170) peptide further comprises from 1 to 10 additional amino acids on the N- and/or C-terminus of the peptide. In yet other embodiments, an MBP(147-170) peptide further comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more additional amino acids on the N- and/or C-terminus of the peptide.

An MBP peptide as provided herein may be described in terms of a core peptide sequence (Pₓ), an optional N-terminal flanking sequence (Rⁿ), and an optional C-terminal flanking sequence (R^{c}). In certain embodiments, the total number of amino acids in the Pₓ, Rⁿ, and R^{c} portions of an MBP peptide is less than 250. In another embodiment, the total number of amino acids is less than 100. In another embodiment, the total number of amino acids is less than 50. In yet other embodiments, the total number of amino acids is less than 250, 240, 230, 220, 210, 200, 190, 180, 170, 160, 150, 145, 140, 135, 130, 125, 120, 115, 110, 105, 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, or 7. In one embodiment, the total number of amino acids is from 6 to 250. In another embodiment, the total number of amino acids is from 6 to 100. In another embodiment, the total number of amino acids is from 6 to 50. In yet other embodiments, the total number of amino acids is from 10 to 250, from 10 to 100, from 10 to 50, from 15 to 250, from 15 to 200, from 15 to 175, from 15 to 150, from 15 to 125, from 15 to 100, from 15 to 90, from 15 to 80, from 15 to 75, from 15 to 70, from 15 to 65, from 15 to 60, from 15 to 55, from 15 to 50, from 15 to 45, from 15 to 40, from 15 to 35, from 15 to 30, from 15 to 25, or from 15 to 20.

In one embodiment, the core peptide sequence (Pₓ) has an amino acid sequence that is identical or substantially identical to at least 6 consecutive amino acids of MBP(43-64) or MBP(115-170), preferably at least 6 consecutive amino acids of MBP(46-62), MBP(124-139), or MBP(147-170). In specific embodiments, Pₓ has an amino acid sequence that is at least 60%, 65, 70%, 75%, 80%, 85%, 90%, 95%, or 100% identical to one of SEQ ID NO:1-3.

In one embodiment, Rⁿ and R^{c} are individually between 1 and 250 amino acids. In a particular embodiment, the combination of Rⁿ and R^{c} is between 1 and 250 amino acids. In one embodiment, an MBP peptide has both an N-terminal flanking region (Rⁿ) and a C-terminal flanking region (R^{c}). In another embodiment, the MBP peptide has an N-terminal flanking region (Rⁿ) but not a C-terminal flanking region (R^{c}). In another embodiment, the MBP peptide has a C-terminal flanking region (R^{c}) but not an N-terminal flanking region (Rⁿ).

### D. Peptide Synthesis

An MBP peptide disclosed herein may synthesized by any suitable method, for example, by solid phase synthesis including solid phase peptide synthesis. Conventional solid phase methods for synthesizing peptides may start with N-alpha-protected amino acid anhydrides that are prepared in crystallized form or prepared freshly in solution, and are used for successive amino acid addition at the N-terminus. At each residue addition, the growing peptide (on a solid support) is acid treated to remove the N-alpha-protective group, washed several times to remove residual acid and to promote accessibility of the peptide terminus to the reaction medium. The peptide is then reacted with an activated N-protected amino acid symmetrical anhydride, and the solid support is washed. At each residue-addition step, the amino acid addition reaction may be repeated for a total of two or three separate addition reactions, to increase the percent of growing peptide molecules which are reacted. Typically, 1 to 2 reaction cycles are used for the first twelve residue additions, and 2 to 3 reaction cycles for the remaining residues.

After completing the growing peptide chains, the protected peptide resin is treated with a strong acid such as liquid hydrofluoric acid or trifluoroacetic acid to deblock and release the peptides from the support. For preparing an amidated peptide, the resin support used in the synthesis is selected to supply a C-terminal amide, after peptide cleavage from the resin. After removal of the strong acid, the peptide may be extracted into 1 M acetic acid solution and lyophilized. The peptide may be isolated by an initial separation by gel filtration, to remove peptide dimers and higher molecular weight polymers, and also to remove undesired salts The partially purified peptide may be further purified by preparative HPLC chromatography, and the purity and identity of the peptide confirmed by amino acid composition analysis, mass spectrometry and by analytical HPLC (*e.g*., in two different solvent systems).

Likewise, the MBP peptides disclosed herein may be prepared by expression in a suitable host cell, followed by purification from the cell culture. Many systems are known in the art for peptide expression. Examples of suitable host strains include: fungal or yeast species such as *Aspergillus, Trichoderma, Saccharomyces, Pichia, Candida* and *Hansenula;* bacterial species such as *Salmonella, Bacillus, Acinetobacter, Rhodococcus, Streptomyces, Escherichia, Pseudomonas, Methylomonas, Methylobacter, Alcaligenes, Synechocystis, Anabaena, Thiobacillus, Methanobacterium, Klebsiella, Burkholderia, Sphingomonas, Brevibacterium, Corynebacterium, Mycobacterium, Arthrobacter, Nocardia, Actinomyces* and *Comamonas;* mammalian cells such as human cell lines, hamster cell lines, and rodent cell lines; and insect cell lines such as baculovirus-based expression systems.

### IV. MBP Peptide Vectors

### A. Introduction

The MBP peptides of the present invention are linked to a vector for administration to a subject in need thereof. Selection of an appropriate vector will be based on many factors, such as: the particular route of administration employed; the dosage of peptide to be delivered; the frequency of dosage; the efficacy of prior treatments; the severity of the disease or symptom being treated; and the current stage of the disease in the subject.

The MBP peptide cargo can be linked to the vector in a covalent or non-covalent fashion. For example, the MBP peptide can be associated with the vector through a covalent bond, ionic bond, electrostatic interactions, hydrophobic interaction, Van der Waals force, embedded in, tethered to, or physically entrapped by the vector.

In one embodiment, the vector linked to the MBP peptide is a nanoparticle. Non-limiting examples of nanoparticles include: liposomes, micelles, block copolymer micelles; polymersomes; niosomes; lipid-coated nanobubbles; dendrimers; metallic particles (for example, an iron oxide particle or gold particle); and silica particles. The peptide cargo can be encapsulated within, embedded in, carried on the surface of, or tethered to the nanoparticle vector.

The use of nanoparticles for the delivery of a therapeutic agent provides several advantages, as compared to administration of the therapeutic agent alone. For example, nanoparticles can be used to shield otherwise labile therapeutic agents, such as native peptides or polynucleotides, from intracellular and/or extracellular insult. Nanoparticles can also function to reduce or eliminate toxic effects caused by the therapeutic agent. Thus, higher doses of a potentially harmful or toxic therapeutic agent can be delivered via nanoparticle formulation.

Nanoparticles can be linked to water soluble or high molecular weight non-immunogenic polymers to increase the serum half-life of the liposome (for example, see, U.S. Patent Nos. 5,013,556, 5,676,971, and 6,132,763). Non-limiting examples of suitable water soluble polymers include: poly(alkylene glycols) such as polyethylene glycol (PEG), poly(propylene glycol) ("PPG"), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(saccharides), poly(α-hydroxy acid), poly(vinyl alcohol), polyphosphasphazene, polyoxazoline, poly(N-acryloylmorpholine), poly(alkylene oxide) polymers, poly(maleic acid), poly(DL-alanine), polysaccharides, such as polysialic acid or carboxymethylcellulose, dextran, starch or starch derivatives such as hydroxyethyl starch (HES), hyaluronic acid and chitin, poly(meth)acrylates, and combinations of any of the foregoing.

In certain embodiments, the MBP peptide vector is further linked to a targeting moiety. In other embodiments, the vector linked to the MBP peptide is a targeting moiety. In a specific embodiment, the targeting moiety (i) increase the delivery of an MBP peptide to a cell (*e.g*., an immune cell), as compared to an MBP peptide not linked to the targeting moiety; and/or (ii) increase the intake of an MBP peptide into a cell (*e.g*., an immune cell), as compared to an MBP peptide not linked to the targeting moiety. In a specific embodiment, the targeting moiety specifically binds to a class or type of cells, for example to immune cells comprising a particular cell-surface antigen.

### B. Liposomes

In a specific embodiment, the MBP peptide vector is a liposome. The use of liposomes for delivery of therapeutic agents is well known in the art (for review, see, Chrai, R. Murari, and I. Ahmad. Liposomes (a review). Part two: Drug delivery systems. BioPharm. 15(1):40,42-43,49 (2002)). Examples of liposome compositions include U.S. Pat. Nos.: 4,983,397, 6,476,068, 5,834,012, 5,756,069, 6,387,397, 5,534,241, 4,789,633, 4,925,661, 6,153,596, 6,057,299, 5,648,478, 6,723,338, and 6,627218; U.S. Pat. App. Publication Nos: 2003/0224037, 2004/0022842, 2001/0033860, 2003/0072794, 2003/0082228, 2003/0212031, 2003/0203865, 2004/0142025, and 2004/0071768; International Patent Publications: WO 00/74646, WO 96/13250, and WO 98/33481; Papahadjopolulos D. et al. (Proc Natl Acad Sci U.S.A. (1991) 88: 11460-11464), Allen and Martin (Semin Oncol (2004) 31: 5-15 (suppl 13)), and Weissig et al. (Pharm. Res. (1998) 15: 1552-1556).

In some embodiments, the liposome includes a phospholipid, for example, a phosphatidylcholine. The phospholipid may be naturally occurring, semi-synthetic, or synthetic. In some embodiments, the phospholipid is a non-naturally occurring phosphatidylcholine. In some embodiments, the phospholipid is cationic. In other embodiments the phospholipid is anionic. In still other embodiments, the phospholipid is neutral. Exemplary phospholipids include phosphatidylcholines (PCs), phosphatidic acid, phosphatidylserine, and phosphatidylglycerol.

In some embodiments, the phospholipid is a phosphatidylcholine. Non-limiting examples of phosphatidylcholine include: 2,3-dipalmitoyl-sn-glycero-l-phosphatydyl choline; distearoyl phosphatidyl choline (DSPC); dimyristoyl phosphatidylcholine (DMPC); dipalmitoyl phosphatidylcholine (DPPC); palmitoyl oleoyl phosphatidylcholine (POPC); egg phosphatidylcholine (EPC); and hydrogenated soya phosphatidylcholine (HSPC).

In some embodiments, the liposome comprises a cationic lipid. As used herein, cationic lipids refer to molecules comprised of at least one, and most typically two, fatty acid chains and a positively charged polar head group. Typical cationic lipids have either dodecyl (C₁₂) or hexadecyl (cetyl, C₁₆) fatty acid chains, although the term "cationic lipid" also is intended to encompass lipids with fatty acid chains of other lengths. Non-limiting examples of cationic lipids include: DOTAP (1,2-diacyl-3-trimethylammonium propane), DOPE (dioleoyl phosphatidylethanolamine), DOTMA ([2,3-bis(oleoyl)propyl]trimethyl anunonium chloride), DOGS (dioctadecyl amido glycyl spermine), DODAB (dioctadecyl diammonium bromide), DODAC (dioctadecyl diammonium chloride), DOSPA (2,3 dioleoyloxy-N-[sperminecarboxaminoethyl]-N-N-dimethyl-1-propanaminium), DC-Chol (3β[N-(n', N'-dimethylaminoethane)-carbamoyl]cholesterol, dioleoyl), DOIC (1-[2-(oleoyloxy)-ethyl]-2-oleoyl-3-(2-hydroxyethyl) imidazolinium chloride), DOPC (dioleoyl phosphatidylcholine), and DMRIE (dimyristooxypropyl dimethyl hydroxyethyl ammonium bromide).

In certain embodiments, the liposome will further comprise cholesterol, a cholesterol analog, and/or a cholesterol derivative that imparts additional fluidity to the lipid bilayer of the liposome. Non-limiting examples of cholesterol analogs and derivatives that can be used in the liposomes provided herein include 5-cholestene, 5-pregnen-3β-ol-20-one, 4-cholesten-3-one and 5-cholesten-3-one. In certain embodiments, cholesterol will be present in a liposome at a concentration from about 0.01 mol% to about 25 mol%. In certain embodiments, cholesterol is present at a concentration of from about 0.1 mol% and about 10 mol% in the liposome. In yet other embodiments, the concentration of cholesterol in a liposome is about 0.01 mol%, 0.02 mol%, 0.03 mol%, 0.04 mol%, 0.05 mol%, 0.06 mol%, 0.07 mol%, 0.08 mol%, 0.09 mol%, 0.1 mol%, 0.2 mol%, 0.3 mol%, 0.4 mol%, 0.5 mol%, 0.6 mol%, 0.7 mol%, 0.8 mol%, 0.9 mol%, 1 mol%, 2 mol%, 3 mol%, 4 mol%, 5 mol%, 6 mol%, 7 mol%, 8 mol%, 9 mol%, 10 mol%, 11 mol%, 12 mol%, 13 mol%, 14 mol%, 15 mol%, 16 mol%, 17 mol%, 18 mol%, 19 mol%, 20 mol%, 21 mol%, 22 mol%, 23 mol%, 24 mol%, 25 mol%, or higher.

In a particular embodiment, the liposome includes a lipid linked to a targeting moiety. Non-limiting examples of targeting moieties that may be linked to a lipid used for the formation of a liposome include: sugar moieties (*e.g*., mannose or a carbohydrate containing one or more mannose residues, analogs, or derivatives thereof); peptides (*e.g*., a cell receptor ligand), polypeptides (*e.g*., an antibody or functional fragment thereof); and nucleic acids (*e.g*., an aptamer or Spiegelmer®).

In one embodiment, the lipid linked to a targeting moiety is included at a final concentration of at least 0.01% of the total lipid content of the liposome. In another embodiment, the concentration of the lipid linked to the targeting moiety in the liposome is at least 0.1% of the total lipid content. In another embodiment, the concentration of the lipid linked to the targeting moiety in the liposome is at least 1% of the total lipid content. In another embodiment, the concentration of the lipid linked to the targeting moiety is at least 5%, or at least 10% of the total lipid content of the liposome. In yet other embodiments, the concentration of the lipid linked to the targeting moiety is about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% of the total lipid content of the liposome.

In a specific embodiment, the liposome comprises a lipid linked to one or more mannose residues (*i.e*., a mannosylated liposome). Non-limiting examples of mannosylated lipids include: ManDOG lipid (Espuelas et al., Bioorg Med Chem Lett. 2003 Aug 4; 13(15):2557-60); tetramannosyl-3-L-lysine-dioleoyl glycerol lipid (Espuelas *et al*., *supra);* and mannosylated phosphatidylinositols (Barratt et al. (1986) Biochim. Biophy. Acta, 862:153-164).

In certain embodiments, the liposomes described herein have an average diameter from about 50 to about 500 nm. For example, the liposome mean diameter may be from about 50 to about 400 nm, from about 50 to about 300 nm, from about 50 to about 250 nm, from about 50 to about 225 nm, from about 50 to about 200 nm, from about 50 to about 175 nm, from about 75 to about 500 nm, from about 75 to about 400 nm, from about 75 to about 300 nm, from about 75 to about 250 nm, from about 75 to about 225 nm, from about 75 to about 200 nm, from about 100 to about 500 nm, from about 100 to about 400 nm, from about 100 nm to about 300 nm, from about 100 to about 250 nm, from about 100 to about 225 nm, from about 100 to about 200 nm. In a particular embodiment, the average diameter of the liposome will be from about 100 nm to about 200 nm.

Liposomes can be made by a number of well-known techniques in the art, including, extrusion, reverse phase evaporation, sonication, agitation, and self-assembly in aqueous solution (for review, *see*, Torchilin VP, Weissig V (2003) Liposomes: a practical approach. Practical approach series, Vol 264, 2nd edition, Oxford University). Furthermore, it is well known that methods for making liposomes can also be used for making compositions of liposomally encapsulated cargos.

Non limiting examples of methods for preparing liposomal compositions are described in International Patent Publications: WO 1999/65465 and WO 2010/052326; U.S. Patent Nos.: 7,381,421, 7,604,803, 8,075,896, 7,790,696, 7,384,923, 7,008,791 and US Patent Application Publication Nos: 2009/0068254 and 2008/0317838. An exemplary scheme for forming a liposomal composition of peptide(s) consists of a five step method: step 1) formation of dry irregular lipid layers by evaporation of organic solvent (lipids in chloroform); step 2) rehydration dry irregular lipid layers leading to the multi-layer MLV liposomes formation; step 3) generation of SUV liposomes from MLV liposomes, by high-pressure homogenization; step 4) dehydration, by freeze drying, of a mix of SUV liposomes with a peptide(s) mixture together with excess sugar; and step 5) rehydration of dehydrated mix of SUV liposomes with the peptide(s) mixture together with excess sugar into the SUV liposomes with size approximately 100-200 nm.

### V. Targeting Moieties

### A. Introduction

To enhance their therapeutic effect, MBP peptide compositions described herein may be linked to a targeting moiety. The targeting moiety can be covalently or non-covalently linked to an MBP peptide, for example, through a covalent bond, ionic bond, electrostatic interaction, hydrophobic interaction, or physical entrapment. In certain embodiments, the linkage can be mediated through a linker or vector structure. Examples of targeting moieties include a sugar moiety (*e.g*., mannose or a carbohydrate containing one or more mannose residues, analogs, or derivatives thereof), a peptide (*e.g*., a cell receptor ligand), a polypeptide (*e.g*., an antibody or functional fragment thereof), and a nucleic acid (*e.g*., an aptamer or Spiegelmer®).

When associated, a targeting moiety improves the efficacy of an MBP peptide, as compared to the efficacy of the cargo alone. In one embodiment, a targeting moiety improves the delivery of the associated MBP peptide to an *in vivo* location or cell type; and/or improves the uptake of the MBP peptide into a cell or location *in vivo.* In a particular embodiment, the targeting moiety improves the delivery of the associated MBP peptide to an immune cell (*e.g.,* a B cell or APC); and/or improves the uptake of the MBP peptide into an immune cell (*e.g.,* a B cell or APC).

When covalently or non-covalently linked to one or more MBP peptide(s), a targeting moiety may: (i) increase the delivery of an MBP peptide to a cell (*e.g*., an immune cell) by at least 10%, as compared to an MBP peptide not linked to the targeting moiety; and/or (ii) increase the intake of an MBP peptide into a cell (*e.g*., an immune cell) by at least 10%, as compared to an MBP peptide not linked to the targeting moiety. In a specific embodiment, the immune cell is a B-cell or antigen presenting cell (APC). In one embodiment, the targeting moiety (i) increases the delivery; and/or (ii) increases the intake of an MBP peptide into a cell by at least 25% as compared to an MBP peptide not linked to the targeting moiety. In another embodiment, the targeting moiety (i) increases the delivery; and/or (ii) increases the intake of an MBP peptide into a cell by at least 50%, 75%, or 100%, as compared to an MBP peptide not linked to the targeting moiety. In yet another embodiment, targeting moiety (i) increases the delivery; and/or (ii) increases the intake of an MBP peptide into a cell, by at least 2-fold as compared to an MBP peptide not linked to the targeting moiety. In yet other embodiments, the targeting moiety (i) increases the delivery; and/or (ii) increases the intake of an MBP peptide into a cell, by at least 4-fold, 5-fold, 6-fold, 7-fold, 8-fold, 9-fold, 10-fold, 15-fold, 20-fold, 25-fold, 30-fold, 40-fold, 50-fold, 75-fold, 100-fold, 150-fold, 200-fold, 250-fold, 300-fold, 400-fold, 500-fold, 600-fold, 700-fold, 800-fold, 900-fold, or 1000-fold as compared to an MBP peptide not linked to the targeting moiety.

### B. Sugar Residues

In one embodiment, the targeting moiety comprises a carbohydrate moiety (*e.g*., a sugar residue). In certain embodiments, the sugar moiety may be a monosaccharide, a disaccharide, or a polysaccharide. The sugar moiety may be naturally occurring, an analog of a naturally occurring sugar, or a derivative of a naturally occurring sugar. Non-limiting examples of sugar moieties that may be used as targeting moieties include: mannose, glucose, fructose, galactose, xylose, ribose, galactosamine, glucosamine, sialic acid, N-acetylglucosamine, sucrose, lactulose, lactose, maltose, cellobiose, trehalose, kojibiose, nigerose, isomaltose, β,β-trehatose, α,β-trehatose, sophorose, laminaribiose, gentiobiose, turanose, maltulose, palatinose, gentiobiulose, mannobiose, melibiose, melibiulose, rutinose, rutinulose, xylobiose, analogs thereof, or derivatives thereof.

Non-limiting examples of mannose derivatives and analogs include 1-deoxymannojirimycin, methyl-a-D-mannopyranoside, 2-deoxy-D-glucose (2-DG), 2-deoxy-2-fluoro-mannose (2-FM), and 2-deoxy-2-chloro-mannose (2-CM), any of which may be conjugated to a lipid.

### VI. Therapeutic Compositions

### A. Introduction

In one aspect, the present invention provides a therapeutic composition for the treatment of multiple sclerosis (MS) comprising an myelin basic protein (MBP) peptide, as defined herein, linked to a vector. In one embodiment, the MBP peptide comprising at least 6 consecutive amino acids of MBP(43-64) (SEQ ID NO:11) or MBP(115-170) (SEQ ID NO:12). Generally, the MBP peptide will consist of from 6 to 100 amino acids in length. In one embodiment, the MBP peptide will consist of from 6 to 50 amino acids in length. In another embodiment, the MBP peptide will consist of from 6 to 25 amino acids in length. In yet other embodiments, the MBP peptide will consist of about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more amino acids. In one embodiment, the vector is a liposome. In a more specific embodiment, the vector comprises a targeting moiety and is a mannosylated liposome.

### B. Vector Compositions

In one embodiment, the composition comprises at least two MBP peptides, each of which is linked to a vector. In one embodiment, both MBP peptides are linked to a single vector (*e.g*., encapsulated within a single liposome). In another embodiment, each MBP peptide is linked to a separate vector (*e.g*., encapsulated in separate liposomes) and the respective MBP-vector complexes are admixed prior to administration.

In one embodiment, the composition comprises a first MBP peptide comprising at least 6 consecutive amino acids of SEQ ID NO:1 linked to a first vector and a second MBP peptide comprising at least 6 consecutive amino acids of SEQ ID NO:2 linked to a second vector. In a specific embodiment, the first MBP peptide comprises the amino acid sequence of SEQ ID NO:1 and the second MBP peptide comprises the amino acid sequence of SEQ ID NO:2. In a more specific embodiment, the first MBP peptide consist of the amino acid sequence of SEQ ID NO:1 and the second MBP peptide consists of the amino acid sequence of SEQ ID NO:2.

In one embodiment, the composition comprises a first MBP peptide comprising at least 6 consecutive amino acids of SEQ ID NO:1 linked to a first vector and a second MBP peptide comprising at least 6 consecutive amino acids of SEQ ID NO:3 linked to a second vector. In a specific embodiment, the first MBP peptide comprises the amino acid sequence of SEQ ID NO:1 and the second MBP peptide comprises the amino acid sequence of SEQ ID NO:3. In a more specific embodiment, the first MBP peptide consist of the amino acid sequence of SEQ ID NO:1 and the second MBP peptide consists of the amino acid sequence of SEQ ID NO:3.

In one aspect, the present invention provides a composition for the treatment of multiple sclerosis, the composition comprising an MBP peptide as defined herein, the peptide linked to a vector comprising a targeting moiety. In a specific embodiment, the vector comprising a targeting moiety increases: (i) delivery of the peptide to an immune cell; or (ii) intake of the peptide into an immune cell, as compared to a peptide linked to a vector in the absence of a targeting moiety. In a specific embodiment, the vector comprises a liposome. In another specific embodiment, the targeting moiety comprises a mannosylated lipid.

In certain embodiments of the compositions described herein, the vector is covalently or non-covalently linked to a targeting moiety. In one embodiment, the targeting moiety (i) increases the delivery of an MBP peptide to a cell (*e.g*., an immune cell), as compared to an MBP peptide not linked to the targeting moiety; and/or (ii) increases the intake of an MBP peptide into a cell (*e.g*., an immune cell), as compared to an MBP peptide not linked to the targeting moiety. In a specific embodiment, the cell is an immune cell. In a more specific embodiment, the immune cell is a B cell or an antigen presenting cell (APC).

### C. Liposomal Vector Compositions

In specific embodiments of the invention, the therapeutic compositions described herein comprise liposomal vectors. Accordingly, the present invention provides a composition for the treatment of MS comprising a liposomally encapsulated MBP peptide. In a specific embodiment, the liposome is linked to a targeting moiety. In a more specific embodiment, the targeting moiety is a mannosylated lipid present in the liposome bilayer.

In a specific embodiment, the liposome is linked to a mannose targeting moiety, *i.e*., is a mannosylated liposome. In certain embodiments, at least 0.01% of the lipids comprising a mannosylated liposome will be conjugated to at least one mannose residue. In another embodiment, at least 0.1% of the lipids comprising a mannosylated liposome will be conjugated to at least one mannose residue. In another embodiment, at least 1% of the lipids comprising a mannosylated liposome will be conjugated to at least one mannose residue. In yet other embodiments, at least 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% of the lipids comprising a mannosylated liposome will be conjugated to at least one mannose residue.

Liposomal compositions may be formulated according to methods well known in the art. Liposomal formulations may comprise one or more of: a buffering agent (*e.g*., acetate buffer, phosphate buffer, citrate buffer, borate buffer, or tartrate buffer); a sugar (*e.g*., trehalose, maltose, sucrose, lactose, mannose, dextrose, or fructose); a sugar alcohol (*e.g*., sorbitol, maltitol, lactitol, mannitol, or glycerol), an alcohol (*e.g*., ethanol or t-butanol); a salt (*e.g*., sodium chloride, potassium chloride, sodium citrate, sodium phosphate, or potassium phosphate); an antioxidant (*e.g*., glutathione).

### D. Targeting Moiety

In certain embodiments, the targeting moiety is a sugar moiety (*e.g*., mannose or a carbohydrate containing one or more mannose residues); a peptide (*e.g*., a cell receptor ligand), polypeptides (*e.g*., an antibody or functional fragment thereof); or nucleic acid (*e.g*., an aptamer or Spiegelmer®). In a specific embodiment, the targeting moiety is a mannose residue.

In one embodiment, the targeting moiety: (i) increases the delivery of an MBP peptide to a cell (*e.g*., an immune cell) by at least 10%, as compared to an MBP peptide not linked to the targeting moiety; and/or (ii) increases the intake of an MBP peptide into a cell (*e.g*., an immune cell) by at least 10%, as compared to an MBP peptide not linked to the targeting moiety. In a specific embodiment, the immune cell is a B-cell or antigen presenting cell (APC). In one embodiment, the targeting moiety (i) increases the delivery; and/or (ii) increases the intake of an MBP peptide into a cell by at least 25% as compared to an MBP peptide not linked to the targeting moiety. In another embodiment, the targeting moiety (i) increases the delivery; and/or (ii) increases the intake of an MBP peptide into a cell by at least 50%, 75%, or 100%, as compared to an MBP peptide not linked to the targeting moiety. In yet another embodiment, targeting moiety (i) increases the delivery; and/or (ii) increases the intake of an MBP peptide into a cell, by at least 2-fold as compared to an MBP peptide not linked to the targeting moiety. In yet other embodiments, the targeting moiety (i) increases the delivery; and/or (ii) increases the intake of an MBP peptide into a cell, by at least 4-fold, 5-fold, 6-fold, 7-fold, 8-fold, 9-fold, 10-fold, 15-fold, 20-fold, 25-fold, 30-fold, 40-fold, 50-fold, 75-fold, 100-fold, 150-fold, 200-fold, 250-fold, 300-fold, 400-fold, 500-fold, 600-fold, 700-fold, 800-fold, 900-fold, or 1000-fold as compared to an MBP peptide not linked to the targeting moiety.

In one aspect, the invention provides a composition for the treatment of MS comprising an MBP peptide, as defined herein, covalently linked to a targeting moiety (*e.g*., wherein the vector is a targeting moiety). In certain embodiments, the targeting moiety is covalently linked directly to the MBP peptide. The targeting moiety may be linked, for example, at the N- or C-terminus of the MBP protein, at a primary amine group of a lysine, glutamine, or asparagine side chain, at a hydroxyl group of a serine or threonine side chain, or at a free thiol of a cysteine side chain. In certain embodiments, the targeting moiety linked directly to the MBP peptide is a sugar moiety (*e.g.,* a mannose residue or a carbohydrate containing one or more mannose residues, an analog thereof, or a derivative thereof), a peptide (*e.g*., a cell receptor ligand), a polypeptide (*e.g*., an antibody or functional fragment thereof), or a nucleic acid (*e.g*., an aptamer or Spiegelmer®). In a specific embodiment, the targeting moiety is a mannose residue.

### E. Combination therapies

No cure for multiple sclerosis currently exists. However, several therapeutic modalities have been approved for the management of symptoms associated with MS. These therapies include: fingolimod, a sphingosine 1-phosphate receptor modulator that sequesters lymphocytes in lymph nodes, preventing them from contributing to an autoimmune reaction; interferon β-1a and β-1b, which likely functions to reduction in the rate of MS relapses, and to slow the progression of disability in MS patients through their anti-inflammatory properties; glatiramer acetate (copaxone), a non-interferon, non-steroidal immunomodulator, which is a random polymer of four predominant amino acids found in MBP, glutamine (Glu), lysine (Lys), alanine (Ala), and tyrosine (Tyr); mitoxantrone, a type II topoisomerase inhibitor used for the treatment of secondary progressive MS; and natalizumab, a humanized monoclonal antibody against the cellular adhesion molecule α4-integrin.

Additionally, the use of the following treatments may provide some therapeutic benefit for patients diagnosed with, or at risk of developing, MS: (i) Administration of glatiramer acetate (GA) which is approved for the treatment of relapsing remitting MS (RRMS). GA is a synthetic random copolymer of Glu, Lys, Ala and Tyr, which induces a population of Th2 regulatory T cells with the ability to cross the BBB and produce anti-inflammatory cytokines IL-4, IL-6, IL-10, and brain-derived nerve grown factor (Aharoni R. et al., Proc Natl Acad Sci U S A 2003;100:14157-62); (ii) Administration of so-called "altered peptide ligands" (APLs) interacting with T cell receptors (TCR). APLs carry modified (Luca ME et al., J Neuroimmunol 2005;160:178-87), mutated (Katsara M. et al., J Med Chem 2009;52:214-8), or restricted (Warren KG et al., Eur J Neurol 2006;13:887-95) TCR-binding moieties and are capable of partly activating T cells, switching phenotype Th1 to Th2, and in some cases inducing T cell anergy. MBP peptide MBP(82-98), an APL derived from encephalitogenic fragment of MBP, shows promising inhibition of MS progression in patients with HLA-DR2-DR4 haplotype. However, a pharmaceutical composition based on this peptide failed in a phase III clinical trial (Fontoura and Garren, Results Probl Cell Differ 2010;51:259-85). A double mutation of MBP(83-99) peptide induces IL-4 responses and antagonizes IFN-gamma responses (Katsara M. et al., J Neuroimmunol 2008;200:77-89); (iii) IFNβ administration; (iv) Monoclonal Abs such as rituximab (anti-CD20) targeted towards B cells (Hauser SL et al., N Engl J Med 2008;358:676-88), daclizumab (anti-CD25, alpha subunit of IL-2 receptor) depleting activated T cells (Rose JW et al., Ann Neurol 2004;56:864-7) and alemtuzumab (anti-CD52, glycoprotein of unknown function presented on all mature lymphocytes and monocytes) (Coles A. et al., Clin Neurol Neurosurg 2004;106:270-4); (v) Oral therapies such as FTY720 in phosphorylated form (inhibitor of SP1-accociated G-protein-coupled receptors), teriflunomide (inhibitor of T cell proliferation), BG-12 (inducer of Th2-cytokines), laquinimod (inhibitor of T cell and macrophage traffic into the CNS, Th2/Th3 shift trigger), cladribine (substrate for deoxycytidine kinase, interfering with DNA repair and lymphocyte death) (all reviewed in Fontoura and Garren, Results Probl Cell Differ 2010;51:259-85); (vi) Inactivated T cell injection or vaccination by TCR hypervariable regions to stimulate TCR-specific counterregulatory CD8+ cells; (vii) Tolerization of immune system: induction of "nasal" or "oral tolerance" by autoantigen, or DNA-vaccination by BHT-3009 plasmid which encodes entire MBP molecule and triggers significant tolerization of both T cells and autoantibodies towards several myelin antigens. (viii) Novel specific B cell-targeted depletion therapy recently proposed (Stepanov AV et al., PLoS One; 6:e20991).

In one aspect, the present invention provides combination therapy for patients diagnosed with or at risk for multiple sclerosis. In one embodiment, the therapy comprises co-administration of an MBP peptide composition described herein and a previously identified therapeutic agent, *e.g*., fingolimod, interferon β-1a, interferon β-1b, glatiramer acetate, mitoxantrone, a type II topoisomerase inhibitor used for the treatment of secondary progressive MS, and an anti-α4-integrin antibody.

In one embodiment, co-administration comprises simultaneous or sequential administration of an antigenic MBP peptide linked to a vector and the second therapeutic agent. In another embodiment, co-administration comprises administration of a first full therapeutic cycle with a first drug, either the MBP peptide composition or the alternative therapy, followed by administration of a full therapeutic regime with the other treatment. In this embodiment, administration of the two drugs does not overlap, rather, the therapies are cycled opposite one another.

### VII. Treatment of Multiple Sclerosis

### A. Introduction

In one aspect, the present invention provides B-cell epitope MBP peptide linked to a vector, as described herein, for use in methods for treating multiple sclerosis (MS) in a subject in need thereof. In a specific embodiment, the method comprises administering a liposomally encapsulated MBP peptide comprising a sequence that is substantially identical to one of SEQ ID NOS:1 to 3 to a subject in need thereof.

In one embodiment, the method comprises administering a therapeutic myelin basic protein (MBP) peptide comprising at least 6 consecutive amino acids of MBP(43-64) (SEQ ID NO:11) or MBP(115-170) (SEQ ID NO:12) linked to a vector (*e.g*., a liposome). Generally, the MBP peptide will consist of from 6 to 100 amino acids in length. In one embodiment, the MBP peptide will consist of from 6 to 50 amino acids in length. In another embodiment, the MBP peptide will consist of from 6 to 25 amino acids in length. In yet other embodiments, the MBP peptide will consist of about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more amino acids. In one embodiment, the vector is a liposome. In a more specific embodiment, the vector is a mannosylated liposome.

In a specific embodiment, the method comprises administering at least two MBP peptides, each respective MBP peptide comprising at least 6 consecutive amino acids of SEQ ID NO:1, SEQ ID NO:2, or SEQ ID NO:3, the MBP peptides linked to a vector. In a more specific embodiment, the MBP peptides each comprise an amino acid sequence selected from SEQ ID NOS:1-3. In a more specific embodiment, the MBP peptides each consist of an amino acid sequence selected from SEQ ID NOS:1-3. In one embodiment, the vector is a liposome. In a more specific embodiment, the liposome is a mannosylated liposome.

In certain embodiments, the subject has been diagnosed with multiple sclerosis. In one embodiment, the subject has been diagnosed with relapsing remitting multiple sclerosis (RRMS). In another embodiment, the subject has been diagnosed with secondary progressive multiple sclerosis (SPMS). In another embodiment, the subject has been diagnosed with primary progressive multiple sclerosis (PPMS). In another embodiment, the subject has been diagnosed with progressive relapsing multiple sclerosis (PRMS).

In one embodiment, the treatment comprises administration of an MBP peptide composition during or immediately following an acute symptomatic attack. In one embodiment, the acute symptomatic attack is an initial attack. In another embodiment, the acute symptomatic attack is a relapse attack. In certain embodiments, the subject may be co-administered intravenous corticosteroids (*e.g*., methylprednisolone) during the acute symptomatic attack. In certain embodiments, the subject has been diagnosed with primary progressive, secondary progressive, or relapsing remitting MS. In one embodiment, the treatment will lessen the severity of the acute attack or improve one or more physical or physiological symptom in the subject.

In another embodiment, the treatment comprises administration of an MBP peptide composition during a period of MS remission in the subject. In certain embodiments, the subject has been diagnosed with secondary progressive or relapsing remitting MS. In one embodiment, the treatment will prevent the onset of an acute attack, delay the onset of an acute attack, lessen the severity of a subsequent acute attack, or improve one or more physical or physiological symptom in the subject.

In another embodiment, the treatment comprises administration of an MBP peptide composition during a period of progressive decline in the subject. In certain embodiments, the subject has been diagnosed with secondary progressive, primary progressive, or progressive relapsing MS. In one embodiment, the treatment will prevent the onset of an acute attack, delay the onset of an acute attack, lessen the severity of a subsequent acute attack, reduce the progression of the disease, stop the progression of the disease, or improve one or more physical or physiological symptom in the subject.

In yet another embodiment, the treatment comprises administration of an MBP peptide composition to a subject diagnosed with an increased risk of developing MS. In certain embodiments, the subject will have one or more risk factors of MS, including: a family history of MS, the presence, up-regulation, or down-regulation of a disease biomarker (*e.g*., interleukin-6, nitric oxide and nitric oxide synthase, osteopontin, fetuin-A, and anti-MBP autoantibodies), and a genetic marker of MS. In certain embodiments, prophylactic administration of an MBP peptide composition to a subject in need thereof will prevent the disease, delay the onset of the disease, prevent an initial acute attack, delay the onset of an initial acute attack, lessen the severity of the disease, or lessen the severity of an initial acute attack.

In certain embodiments, the subject will have been previously treated for MS. In other embodiments, the subject will not have received previous treatment for MS.

### B. Administration

The MBP peptide compositions of the present invention may be administered according to any known administrative route, for example, topical, enteric, parenteral, intravenous, subcutaneous, subdermal, intramuscular, intraperitoneal, inhalation, epidural, cannulation (*e.g*., intravenous, nasal, oral, or intercranial cannula), administration directly to the central nervous system, or any other similar route of administration. The route of administration chosen for a particular therapeutic treatment will depend, for example, the pharmaceutical composition, the dosage of therapeutic agent to be delivered, the status of the disease state being treated; results provided by clinical trials such as the efficacy of a particular formulation and the safety profile for a particular formulation, and the expected patient compliance. In a specific embodiment, the MBP peptide composition is administered subcutaneously.

In certain embodiments, an MBP peptide composition is administered such that the composition is delivered or accumulates in the central nervous system. In one embodiment, the composition is administered directly to the central nervous system (CNS), for example, by spinal epidural, intranasal administration (for review see, Liu X., Expert Opin Drug Deliv. 2011 Dec;8(12):1681-90 and Wen MM., Discov Med. 2011 Jun; 11(61):497-503), or implantation of a drug delivery system (for review see, Tresco and Winslow, Crit Rev Biomed Eng. 2011;39(1):29-44).

Since direct administration of therapeutics to the CNS presents many challenges, including the risk of potentially lethal infections, the composition may also be administered external to the CNS. Therapeutics delivered in this fashion must pass through the blood-brain barrier (BBB) to enter the CNS. Several strategies have been proposed to enhance the passage of therapeutics through the BBB (*see*, Hossain S et al. Curr Drug Deliv. 2010 Dec; 7(5):389-97). For example, the use of vector systems displaying BBB receptor ligands, peptides, or antibodies specific for the BBB on their surface (for review see, Costantino L., Future Med Chem. 2010 Nov; 2(11):1681-701 and Craparo et al., CNS Neurosci Ther. 2011 Dec; 17(6):670-7) or a chimeric peptide comprising an MBP peptide fused to a BBB transport vector such as an endogenous peptide, modified protein, or peptidomimetic monoclonal antibody (MAb) that undergoes RMT through the BBB on endogenous endothelial receptor systems (see, Pardridge, W.M., Mol. Interv., 2003, 3(2), 90-105).

In one embodiment, the treatment comprises periodic administration, for example, once a month, twice a month, once a week, twice a week, three times a week, every other day, every day, or twice a day, for a given period of time. Depending on the therapeutic regimen and disease status of the patient, a treatment cycle may continue for at least a month, or at least 2, 3, 4, 5, 6, 7, 8, 9, 10, 11, or 12 months. Where appropriate, the treatment cycle may continue for at least a year, or at least 2, 3, 4, 5, 6, 7, 8, 9, 10, or more years. The effect of the treatment on the patient can be monitored and adjusted as needed, for example, to improve efficacy or reduce side effects, by a skilled physician.

The dosage administered to a patient will vary dependent on a number of factors, including: frequency of administration; severity of the condition (*e.g*., multiple sclerosis); subtype of the condition (*e.g*., relapsing remitting MS, secondary progressive MS, primary progressive MS, and progressive relapsing MS); stage of the condition (*e.g*., initial attack, relapse, and remission); size and tolerance of the subject; route of administration employed; risk of side effects; risk of adverse drug interactions; and response to prior treatments, each of which can be readily determined by a skilled physician.

As discussed above, many factors will contribute to what constitutes an appropriate dosage, including the frequency of administration. In one embodiment, an MBP peptide composition provided herein may be administered at a dosage of from about 0.01 mg/kg to about 1000 mg/kg. In other embodiments, the dosage may be from about 0.05 mg/kg to about 500 mg/kg. In another embodiment, the dosage may be from about 0.1 mg/kg to about 250 mg/. In another embodiment, the dosage may be from about 0.25 mg/kg to about 100 mg/kg. In another embodiment, the dosage may be from about 0.5 mg/kg to about 50 mg/kg. In yet another embodiment, the dosage may be from about 1 mg/kg to about 25 mg/kg. In yet other embodiments, the dosage may be from about 0.1 mg/kg to about 10 mg/kg, from about 5 mg/kg to about 25 mg/kg, from about 20 to about 50 mg/kg, from about 50 to about 100 mg/kg, from about 100 to about 250 mg/kg, or from about 200 mg/kg to about 500 mg/kg. In one embodiment, the dosage is administered daily. In another embodiment, the dosage is administered every other day, every third day, every fourth day, every fifth day, every sixth day, or every seventh day. In one embodiment, the dosage is administered once a week. In another embodiment, the dosage is administered once every two weeks. In other embodiments, the dosage is administered every third, fourth, fifth, sixth, seventh, eighth, ninth, tenth, eleventh, or twelfth week.

### VIII. Examples

### Example 1 - Preparation of Liposomes Containing MBP Peptides by Sonication

45 g of a phospholipid mixture containing 1 part by weight of mannosylated DOG lipid (ManDOG) and 99 parts by weight of 2,3-dipalmitoyl-sn-glycero-l-phosphatydyl choline was dissolved in 450 mL of chloroform and placed in a 1 L vacuum evaporator flask. The chloroform was evaporated under vacuum to form a lipid film on the flask walls. After evaporation, the flask was filled with nitrogen gas, and 800 mL of water for injections (WFI) was slowly introduced therein. The flask was placed in the ultrasonic bath for 30 minutes to disrupt pre-formed liposomes. Liposomes re-formed after sonication, resulting in an aqueous emulsion of liposomes.

0.75 g of an MBP peptide mixture containing GGDRGAPKRGSGKDSHH (SEQ ID NO:1; MBP1), GFGYGGRASDYKSAHK (SEQ ID NO:2; MBP2), and QGTLSKIFKLGGRDSRSGSPMARR (SEQ ID NO:3; MBP3) and excess lactose (lactose to lipid ratio of 3:1) in equal amounts was then dissolved in 40 mL of water for injections. The liposome emulsion was added to the MBP peptide solution, and the mixture was stirred for 30 minutes, resulting in an emulsion of liposomes with sizes between 100 nm and 200 nm and un-encapsulated MBP peptides. The resulting emulsion of monolamellar liposomes was then lyophilized.

The next step was rehydration under controlled conditions followed by washing of the resulting SUV liposomes by centrifugation to remove non-incorporated materials. The washed pellets were re-suspended in PBS to the required dose volume. Peptide incorporation was estimated on the basis of reversed-phase HPLC using linear gradient of acetonitrile applied on a C18 column. The z-average diameter and zeta potential of liposomes were measured on a Brookhaven ZetaPlus zetasizer at 25°C by diluting 20 µl of the dispersion to the required volume with PBS or appropriate media. Control liposomes without peptides (vehicle) and liposomes lacking mannosylated lipid were obtained identically except addition of MBP peptides and ManDOG respectively.

### Example 2 - Preparation of Liposomes Containing MBP Peptides via Disintegration

45 g of a phospholipid mixture containing 1 part by weight of mannosylated DOG lipid (ManDOG) and 99 parts by weight of 2,3-dipalmitoyl-sn-glycero-l-phosphatydyl choline was dissolved in 450 mL of chloroform and placed in a 1 L vacuum evaporator flask. The chloroform was evaporated under vacuum to form a lipid film on the flask walls. After evaporation, the flask was filled with nitrogen gas, and 800 mL of water for injections was slowly introduced therein. The resulting mixture is transferred to a flowing disintegrator receiver and the stroke volume pressure of the disintegrator is set at 150 MPa. 100 mL. of the mixture is added to the flowing disintegrator per load, and the resulting liposomal emulsion is collected from the disintegrator receiver.

0.75 g of an MBP peptide mixture containing GGDRGAPKRGSGKDSHH (SEQ ID NO:1; MBP1), GFGYGGRASDYKSAHK (SEQ ID NO:2; MBP2), and QGTLSKIFKLGGRDSRSGSPMARR (SEQ ID NO:3; MBP3) and excess lactose (lactose to lipid ratio of 3:1) in equal amounts was then dissolved in 40 mL of water for injections. The liposome emulsion was added to the MBP peptide solution, and the mixture was stirred for 30 minutes, resulting in an emulsion of liposomes with sizes between 100 nm and 200 nm and un-encapsulated MBP peptides. The resulting emulsion of monolamellar liposomes was then lyophilized.

The next step was rehydration under controlled conditions followed by washing of the resulting SUV liposomes by centrifugation to remove non-incorporated materials. The washed pellets were re-suspended in PBS to the required dose volume. Peptide incorporation was estimated on the basis of reversed-phase HPLC using linear gradient of acetonitrile applied on C18 column. The z-average diameter and zeta potential of liposomes were measured on a Brookhaven ZetaPlus zetasizer at 25°C by diluting 20 µl of the dispersion to the required volume with PBS or appropriate media. Control liposomes without peptides (vehicle) and liposomes lacking mannosylated lipid were obtained identically except addition of MBP peptides and ManDOG respectively.

### Example 3 - Aqueous Formulation of Liposomes Containing MBP Peptides

100 mL of buffered phosphate saline solution (FBR) was added to 1000 mg of lyophilized MBP-peptide liposomes, prepared as described in Example 1, under sterile conditions and stirred. Beta-carotene was added then added to the composition at a final concentration of 0.01%, as an antioxidant. The liposome composition was then dispensed into hydrolytic class I glass containers under sterile conditions and a nitrogen atmosphere. The containers were subsequently sealed with rubber plugs and fitted with aluminum caps.

### Example 4 - Lyophilized Formulation of Liposomes Containing MBP Peptides

To 1000 mg of lyophilized MBP peptide liposomes, prepared as described in Example 1, 2 mg of solid Alpha tocopherol was added under sterile conditions. 100 mg of the resulting dry mixture was dispensed into hydrolytic class I glass containers under sterile conditions and a nitrogen atmosphere. The containers were subsequently sealed with rubber plugs and fitted with aluminum caps. Prior to use, the dried MBP liposomal mixture was reconstituted with 1 to 2 mL of WFI per container and shaken for 1-2 minutes to form a homogenous liposome emulsion.

### Example 5 - Treatment of EAE in DA Rats with Liposomally Encapsulated MBP Peptides

To induce experimental allergic encephalomyelitis (EAE) in DA rats having a mass of 220-250 g (12-14 months of age), 10 µg of myelin basic protein encephalogenic peptide fragment ARTTHYGSLPQKSQRSQ (SEQ ID NO:4; Anaspec, US) emulsified in Freund's complete adjuvant (Difco, US) at a concentration of 10% (m/v) was subdermally injected into the forepaw. The rats were weighted and monitored for EAE neurological symptoms daily. The EAE symptom profile of each rate was scored according to the following scale: (0) - absence of EAE symptoms; (1) - decrease in tail tonicity; (2) - decrease in righting reflex; (3) - paresis; (4) - full paralysis; and (5) - agony or death. Intermediary symptoms were scored by increasing or decreasing the value by 0.5 units. Six days after EAE induction, animals were randomly distributed into several groups (12 animals each).

Between days six and eleven after induction, rats in each respective group were subdermally injected with the liposomal preparation described in Example 2 (emulsified in phosphate saline solution, pH 7.4), a positive control preparation of glatiramer acetate (GA; copaxone, Teva Pharmaceutical Industries Ltd, Israel), a prototype peptide: DENPVVIIFFKNIVTPRT (SEQ ID NO:5), or a buffered phosphate saline solution (pH 7.4) placebo. The peptide, Glatiramer acetate, and liposomal preparations were formulated immediately prior to administration using buffered phosphate saline solution (pH 7.4). 0.1 mL of each composition was administered daily at a concentration of 150 µg per animal. Test results are presented in Table 2:

**Table 2. The effect of prototype, the disclosed liposomes, the placebo and the glatiramer acetate on EAE disease course in rats of DA line.**

| | Evaluation of EAE symptom intensity, average | | | |
|---|---|---|---|---|
| Day after EAE induction, No. | Disclosed liposomes | Prototype | Placebo | GA |
| -1 | 0,00 | 0,00 | 0,00 | 0,00 |
| 0 | 0,00 | 0,00 | 0,00 | 0,00 |
| 1 | 0,00 | 0,00 | 0,00 | 0,00 |
| 2 | 0,00 | 0,00 | 0,00 | 0,00 |
| 3 | 0,00 | 0,00 | 0,00 | 0,00 |
| 4 | 0,00 | 0,00 | 0,00 | 0,00 |
| 5 | 0,00 | 0,00 | 0,00 | 0,00 |
| 6 | 0,00 | 0,00 | 0,00 | 0,00 |
| 7 | 0,00 | 0,00 | 0,00 | 0,00 |
| 8 | 0,75 | 0,58 | 0,50 | 0,50 |
| 9 | 1,25 | 1,33 | 1,50 | 1,67 |
| 10 | 1,92 | 2,42 | 2,33 | 2,17 |
| 11 | 1,58 | 2,33 | 2,42 | 2,25 |
| 12 | 1,50 | 2,50 | 2,34 | 2,25 |
| 13 | 1,17 | 2,17 | 1,92 | 2,83 |
| 14 | 1,08 | 2,00 | 2,17 | 2,75 |
| 15 | 0,50 | 1,83 | 1,92 | 2,33 |
| 16 | 0,08 | 1,67 | 1,58 | 2,08 |
| 17 | 0,17 | 1,33 | 1,93 | 2,17 |
| 18 | 0,08 | 1,42 | 1,83 | 2,17 |
| 19 | 0,08 | 1,08 | 1,75 | 1,75 |
| 20 | 0,00 | 0,83 | 1,67 | 1,67 |

As shown in Table 2, administration of the MBP peptide liposomal composition provided significantly greater therapeutic benefit by reducing the intensity and rate of EAE progression in DA rats, as compared to administration of either the prototype peptide or Glatiramer acetate compositions (compare average EAE scores at day 20).

### Example 6 - Treatment of a Female Human Subject Diagnosed with Multiple Sclerosis with Liposomally Encapsulated MBP Peptides

A female patient (age 30) diagnosed with multiple sclerosis (cerebrospinal form, progressive course) was treated previously with corticosteroid, beta-interferon and glatiramer acetate, each of which proved ineffective, as her neurological deficit and cognitive impairment continued to progress. Serum levels of MBP antibodies in the subject were determined to be 10⁷ units per mL and the patient's T-lymphocyte stimulation index (SI) was measured at 6.5.

With proper consent, the liposomal MBP tripeptide composition prepared as described in Example 3 was administered subdermally at a dosage of 200 mg every other week for 6 months. Over the course of treatment, regression of the patient's neurological deficit by 1.5 units (EDSS scale) was observed. Serum levels of anti-MBP IgG antibodies were reduced to non-detectable levels. And SI of T-lymphocytes was measured after 6 months at 2.

These results demonstrate that MS can be effectively treated in humans by administration of the liposomal MBP peptide compositions described herein.

### Example 7 - Treatment of a Male Human Subject Diagnosed with Multiple Sclerosis with Liposomally Encapsulated MBP Peptides

A male patient (age 36) diagnosed with multiple sclerosis (cerebrospinal form, remissive course), in the relapse stage, was previously subjected to repeated corticosteroid treatment. The patient had displayed symptoms of MS for 3 years. At the time of treatment, the patient presented with: lateral nystagmus when looking to the right, active tendon reflex, S < D in the legs, active upper abdominal reflex, absent middle and lower abdominal reflex, bilateral Babinski's symptom, foot clonus (more expressed in the right foot), no paresis in the extremities, muscle tonicity unchanged, ataxic gait, loss of balance in the Romberg stance, ataxia when performing hccl-shin test, and urine retention. The value of the patient's neurological deficit on the EDSS scale was 6.

The patient also presented with ophthalmic blanching of temporal halves of optic nerve heads. After studying the patient's PBMC, the activity of T-lymphocytes with respect to MBP was determined at a stimulation index (SI) of 7.45. Levels of serum IgG antibodies were 75 units per mL.

With the patient's consent, the prototype peptide composition was administered once every two weeks at a dosage of 500 mg. 7 days after each injection, the patient's peripheral (venous) blood was sampled in order to determine changes in T-lymphocyte activity with respect to MBP, as well as autoantibody levels. Over the course of 7 weeks, the patient's clinical presentation of MS progressed slightly. At week four, the patients IgG autoantibody level increased to 112 units per mL, and T-cell SI doubled.

After the initial course of treatment with the prototype peptide, the patient consented to treatment with the liposomal MBP tripeptide composition described above. The patient was administered 100 mg of the composition once every two weeks for twelve weeks (6 total injections).

Three weeks into the liposomal MBP tripeptide treatment regime, signs of remission began to appear. By week eight, the patient's IgG autoantibody level had dropped to 25 units per mL and T-cell SI was three times lower than the level measured prior to treatment. Clinically, the patient's uresis and gait normalized, he was able to maintain balance in the Romberg stance, he easily passes the heel-shin test, and his neurological deficit value on the EDSS scale dropped to 5.

These results demonstrate that MS can be effectively treated in humans by administration of the liposomal MBP peptide compositions described herein.

### Example 8 - Identification of a Relevant Rodent Model for Multiple Sclerosis

EAE may be induced in many species by immunization by myelin antigens, as serves as a model for multiple sclerosis (MS). These MS models, although used in many studies, are not fully relevant to the MS disease. For example, a number of studies showing efficacy of a proposed MS therapy in an EAE animal model have failed to translate into a beneficial effect, and even cause exacerbation, upon treatment of a human MS patient (Hohlfeld and Wekerle, Proc Natl Acad Sci U S A 2004;101:14599-606). Thus, a careful examination of EAE rodent models was performed to identify a system more closely matching the spectra of MBP autoantibodies (autoAb) present in MS patients.

An MBP epitope library representing fragments of this neuroantigen fused with thioredoxin carrier protein was previously prepared (Belogurov AA et al., J Immunol 2008;180:1258-67). It was reported that the pattern of autoAb binding to the MBP epitope library may be regarded as a molecular signature, or snapshot of pathogenic B cell answer in MS. To identify the most relevant rodent model for MS, EAE was induced in three rodent strains: SJL and C57BL/6 mice, and DA rats (Figure 1B). MBP epitope library was tested by hybridization with anti-MBP and anti-c-myc mAb (Figure 1A) to determine the anti-MBP autoantibody binding pattern of the EAE-induced rodent models. This binding pattern was then compared to the anti-MBP autoantibody binding pattern determined for 12 human MS patients.

All animal studies were carried out in animal facilities of Assaf Harofeh Medical Center (Zerifin, Israel) using standard approved practices for animal care. Induction of EAE was performed in 8-9 weeks Dark Agouti (DA) female rats. Briefly, rats were injected intradermally at the base of the tail with a total volume of 200 µL of inoculum, containing 50 µg of MBP(63-81) (ANASPEC), in saline mixed (1:1) with CFA, (incomplete Freund's adjuvant (IFA), Sigma), and 1 mg MT (strain H37 RA; Difco Laboratories, Detroit, MI). Animals developing symptoms of MS were included to the study. Rats were treated with different liposomes formulations (Table 3), copaxone, or placebo (vehicle) under similar conditions for 6 days. All formulations were administrated by one subcutaneous injection per day. Animals were followed up till day 28th post EAE induction. Clinical signs score was performed daily during all study periods. Score gradation was the following: 0 - Normal 1 - tail weakness, 2 - hind leg weakness or paralysis, 3 - hind leg paralysis, dragging hind limbs, 4 - complete paralysis, unable to move, 5 - death.

SPF female SJL mice, 6 to 8 weeks old, were immunized according to the established protocol (Coligan JE, Current protocols in immunology. [New York]: Wiley, 1996, p. Suppl. 19, Unit 5.1 & Suppl. 21, Unit 2.8) with bovine MBP injected at 50 µg per mouse in complete Freund's adjuvant containing 2 mg/mL *M*. *tuberculosis.* SPF female C57BL/6 mice, 6 to 8 weeks old, were immunized according to the established protocol (Oliver AR et al., J Immunol 2003;171:462-8) with recombinant extracellular domain of MOG injected at 100 µg per mouse in complete Freund's adjuvant containing 0.5 mg/mL *M*. *tuberculosis.* Between 14 and 28 days after a second immunization, mice with pronounced clinical symptoms were euthanized and their sera were collected for analysis.

10 mL blood samples from 12 relapsing-remitting MS patients were obtained from Moscow Multiple Sclerosis Center at the City Hospital #11. The MS patients were between 23 and 61 years of age (median 32.2 years). Their Expanded Disability Status Scale (EDSS) scores ranged from 0 to 4 (median 2.0). The EDSS is scored on a scale of 0 to 10, with higher scores indicating greater disability. None of the patients had received treatment with corticosteroids for at least one month before the blood samples were taken (Kurtzke JF, Neurology 1983;33:1444-52). All patients signed the Information Consent in accordance with the regulations of the Ministry of Health of the Russian Federation, approved by the Ethic Committee of City Hospital #11.

To determine anti-MBP autoantibody binding patters in sera from the EAE-induced rodent models and human MS patients, ELISA experiments were performed as follows. Microtiter plates (MaxiSorp-Nunc) were coated with 50 µL of a 10 µg/mL MBP solution or recombinant MBP peptides in 100 mM carbonate/bicarbonate buffer pH 9.0, in odd column wells. Plates were sealed with ELISA plate sealer (Costar) and incubated at 4°C overnight and then washed (300 µl/well) three times with phosphate-buffered saline (PBS) containing 0.15% Tween-20. All the wells were then blocked with 250 µl 2% bovine serum albumin (BSA) (Sigma) in carbonate/bicarbonate buffer pH 9.0 and incubated for 1 hr at 37°C. The plates were washed with PBS containing 0.15% Tween-20. Serum antibodies were diluted in PBS containing 0.15% Tween-20 and 0.5% BSA at the final dilution of 1:1000-1:50000, and 50 µl of the diluted sample was added to each well of the plate. Rat anti-MBP monoclonal antibody (ab7349, Abcam) was used as control. Plates were incubated for 1 h at 37°C, washed three times with PBS 0.15% Tween-20. To each well, 50 µl of goat anti-whole anti-rat IgG conjugated to horseradish peroxidase (A9037, Sigma) diluted 1:4000, was added in buffer and incubated for 1 h at 37°C. After five washes with PBS 0.15% Tween-20, 50 µl of tetra methyl benzidine was added to each well and stored in the dark from 5 to 15 min. The reaction was stopped with 50 µl/well of 10% phosphoric acid. The OD₄₅₀ values were measured using a microplate reader Varioscan (Thermo, USA).

In terms of autoAb response: one immunodominant region, MBP(124-147), was identified in C57BL/6 mice; two in SJL mice, MBP(24-44) and MBP(72-139); and two in DA rats; MBP(40-60) and MBP(107-170). The last two are correlated with human MS patterns, including two fragments MBP(43-64) and MBP(115-170), suggesting the immunological response seen in DA rats induced with EAE is significantly relevant as a model for human MS. Three peptides were selected for further analysis: MBP(46-62) ("MBP1"); MBP(124-139) ("MBP2"); and MBP(147-170) ("MBP3"), which were the most immunodominant in both MS patients and DA rats. Significantly, high-avidity myelin-specific CD4+ T cells described by Bielekova *et al.* (Bielekova B et al., J Immunol 2004; 172:3893-904) possess reactivity towards MBP peptides 111-129 and 146-170, which overlap with the MBP fragments identified in the present study, demonstrating cross-reactivity between these T- and B-cell epitopes.

### Example 9 - EAE DA Rats Immunized with MBP63-81 Release Anti-MBP Autoantibodies Recognizing Encephalitogenic and C-Terminal MBP Peptides

Previously, only GPBP(62-84), and to a lesser extent GPBP(68-88), have been shown to be capable of inducing EAE in DA rats (Miyakoshi A. et al., J Immunol 2003;170:6371-8). However, given that encephalitogenic peptide MBP(81-104) plays a significant role in MS evaluation (Aharoni R. et al,. J Neuroimmunol 1998;91:135-46), an immunization protocol resulting in reproducibly high level of autoantibodies to the C-terminal MBP fragment and MBP encephalitogenic region was desired. To ensure that epitope spreading, a hallmark of MS, would be included in the EAE pathogenesis of the rodent model, spinal cord homogenate was excluded from the homogenate used in the present study.

Briefly, DA rats were immunized with the peptide MBP(63-81), in order to achieve the desired EAE pathologies. Analysis of serum antibodies from the DA rats after immunization revealed an enhanced autoantibody response to three MBP fragments: MDHARHGFLPRH (SEQ ID NO:6); QDENPVVHFFKNIV (SEQ ID NO:7) and IFKLGGRDSRSGSPMARR (SEQ ID NO:8). The specificity of the polyclonal IgG anti-MBP autoantibodies was determined according to the binding with MBP epitope library and further theoretical calculation based on the assumption of peptides overlapping (Figure 2A). No significant activity of serum autoAb in EAE DA rats against MBP(63-81) was identified. Since the MBP(63-81) peptide was used as the antigen, this suggested the involvement of epitope spreading during EAE development in the DA rats. This observation is also in accordance with earlier findings that MBP(62-75), a major encephalitogenic peptide in DA rats, is not immunodominant as defined by Sercarz *et al.* (Sercarz EE et al., Annu Rev Immunol 1993;11:729-66).

To quantify autoAb recognition of the identified MBP epitopes, and to confirm their sequence *in vitro,* the affinity of polyclonal serum anti-MBP antibodies isolated from immunized DA rats for biotinylated MBP peptides was determined by surface plasmon resonance (Figure 2B). The effective dissociation constant of the full-length MBP protein (1.5x10⁻⁸) was determined to be nanomolar, as were the dissociation constants for MBP encephalitogenic (9.6x10⁻⁹) and C-terminal (8.4x10⁻⁹) fragments, verifying their identity as major B cell epitopes. Binding to the MDHARHGFLPRH (SEQ ID NO:6) peptide was not detectable, and thus, it was excluded from the further evaluation in this study as a biomarkers for EAE progression.

All surface plasmon resonance measurements were performed on BiaCore T-200 apparatus (GE Healthcare, US). Biotinylated MBP peptides (50 µg/mL) (listed on Figure 2) and MBP were immobilized on SA and CM-5 chips respectively. All procedures were performed according manufacture's recommendations. Flow rate of HBS-EP buffer was kept as 10 µl/min during all measurements. Antibodies (50 µg/mL) were tested on both chips with standard association/dissociation time 300/300 s. Dissociation constants were calculated using BiaCore T-200 Evaluation Software 1.0.

### Example 10 - Administration of MBP-Derived B Cell Epitopes Encapsulated in Mannosylated SUV Liposomes Significantly Ameliorates EAE in a MS Rat Model

Treatment of EAE in Lewis rats was performed previously with encapsulated myelin autoantigens different from those provided herein (see, St Louis J. et al., J Neuroimmunol 1997;73:90-100; and Avrilionis and Boggs, J Neuroimmunol 1991;35:201-10). At the same time, the group of Nagelkerken showed that administration of mannosylated APL M-PLP139-151 induces peptide-specific tolerance to EAE in SJL mice (Luca ME et al., J Neuroimmunol 2005;160:178-87).

Newly identified B cell MBP peptide antigens were encapsulated into small, unilamellar vesicles (SUV) liposomes bearing mannose residues on their surface. The major benefit of this approach is that immunodominant non-modified peptides are present in native form inside the liposome, while delivery to antigen-presenting cells (APCs) is enhanced by the surface-exposed mannose. APCs have high levels of mannose receptors on their surface, enhancing endocytosis of the mannosylated liposome particles into the cytosol (Keler T. et al., Expert Opin Biol Ther 2004;4:1953-62). On the other hand, administration of cationic liposomes lacking mannose may significantly increase antibody response, as described by Durova *et al.* for an "anti-HIV vaccine" (Durova OM et al., Mol Immunol 2009;47:87-95), and should be avoided for self-tolerance induction.

Liposomes were assembled from a mixture of egg phosphatidylcholine (PC) and one molar percent mannosylated DOG (ManDOG) (Durova OM, *supra*). Assembly of SUV liposomes was performed as illustrated in Figure 3: (i) formation of irregular lipid layers during evaporation of organic solvent, followed by re-hydration leading to the multi-layer multilamellar vesicle (MLV) liposomes formation; (ii) high-pressure homogenization resulting in formation of empty SUV; (iii) freeze drying of SUV liposomes with peptides - at this stage peptides are located between collapsed SUV liposomes; and (iv) encapsulation of peptides during second re-hydration into the SUV liposomes with an average diameter of approximately 60-100 nm, containing 1.0 % mannose residues on their surface. Four formulations were used for this study: each B cell epitope MBP peptide (MBP1, MBP2, and MBP3) individually and a 1:1:1 mixture (by mass) of all three peptides.

Small unilamellar vesicles (SUV) were prepared from egg phosphatidylcholine (PC) and mannosylated DOG (Espuelas S. et al., Synthesis of an amphiphilic tetraantennary mannosyl conjugate and incorporation into liposome carriers, 2003 Bioorg Med Chem Lett., Aug 4; 13(15):2557-60) (1:100 molar ratio) by high pressure homogenization (Durova OM, *supra*). Briefly, lipid mixture (100 mg/mL) in CHCl₃ was dried under vacuum, further re-suspended in Milli-Q water to a final lipid concentration 50 mg/mL, followed by high-pressure homogenization (20,000 psi). Resulting SUV were mixed with peptides (lipid to peptide ratio 330:1) together with excess sugar (lactose to lipid ratio 3:1) with subsequent freeze-drying. Following rehydration under controlled conditions, the resulting SUV liposomes were washed by centrifugation to remove non-incorporated materials. The washed pellets were re-suspended in PBS to the required dose volume. Peptide incorporation was estimated on the basis of reversed-phase HPLC using linear gradient of acetonitrile applied on C18 column. The z-average diameter and zeta potential of liposomes were measured on a Brookhaven ZetaPlus zetasizer at 25°C by diluting 20 µl of the dispersion to the required volume with 1 mM PBS or appropriate media.

To test the therapeutic potential of the four formulations, DA rates presenting with induced EAE were subcutaneously injected with one of the four mannosylated liposomal formulations, copaxone (positive control), free (un-encapsulated) MBP1 peptide (negative control), and empty mannosylated liposomes (vehicle control; Table 3).

**Table 3. Liposomal characteristics and experimental groups of DA rats involved in the study.**

| Composition | size (nm) | PDI | zeta (mV) | peptide entrapment (%) | dose per rat/per day (6 injections total) (g) | | | | |
|---|---|---|---|---|---|---|---|---|---|
| | | | | | MBP1 | MBP2 | MBP3 | copaxone | liposomes |
| vehicle | 80 | 0.20 | -10.0 | - | - | - | - | - | + |
| MBP1 SUV | 95 | 0.19 | -10.5 | 91 | 150 | - | - | - | + |
| MBP2 SUV | 85 | 0.18 | -8.3 | 93 | - | 150 | - | - | + |
| MBP3 SUV | 81 | 0.21 | -9.2 | 90 | - | - | 150 | - | + |
| MBP1/2/3 SUV | 73 | 0.22 | -7.5 | 93 | 150 | 150 | 150 | - | + |
| copaxone | - | - | - | - | - | - | - | 450 | - |
| MBP1 | - | - | - | - | 150 | - | - | - | - |

Treatment of each rat was initiated at the first sign of EAE clinical manifestation. As shown in Table 4, treatment with liposomal MBP1 and MBP1/2/3 peptide formulations significantly reduced maximal and cumulative disease score in EAE induced DA rats. Furthermore, mortality was reduced in all of the groups treated with liposomal MBP peptides (MBP2 SUV- 1/11; all MBP SUV - 1/54), as compared to the group treated with empty liposomal vehicle (3/17). One death also occurred in the group treated with free MBP1 (1/15).

**Table 4. Effect of MBP peptides entrapped into the mannosylated SUV liposomes on the EAE development in DA rats.**

| **Treatment Group** | **N** | **Median Maximal Disease Score (IQR³)** | **Median Cumulative Disease Score (IQR³)** | **Mortality** |
|---|---|---|---|---|
| vehicle | 17 | 3 (0.5) | 22 (12.5) | 3/17 |
| MBP1 SUV | 12 | 2 (1)¹ | 17 (5.5)¹ | 0/12 |
| MBP2 SUV | 12 | 3 (0.75)² | 17.5 (26.75)² | 1/12 |
| MBP3 SUV | 12 | 3 (0)² | 15 (11)² | 0/12 |
| MBP1/2/3 SUV | 18 | 3 (0.75)¹ | 14 (5.25)¹ | 0/18 |
| copaxone | 12 | 2 (1.25)¹ | 18.5 (12.5)² | 0/12 |
| MBP1 | 15 | 3 (0.5)² | 19.5 (22)² | 1/15 |

| | | | | |
|---|---|---|---|---|
| ¹p<0.05 statistically significant differences observed from control group; one way Anova for nonparametric statistics: Wilcoxon ²p>0.05 statistically significant differences not observed from control group; one way Anova for nonparametric statistics: Wilcoxon ³Interquartilie range, value in parenthesis (). | | | | |

The mean disease score and rate of gliosis/demyelination was determined for each treatment group, as shown in Figure 4. As can be seen, treatment with liposomal formulations of MBP1 provided the greatest reduction in maximal disease score during the initial attack (panel 4A). Treatment with liposomal formulations of MBP2 and MBP3 limited progression of disease in the remission stage (panels 4C and 4D). Administration of a liposomal formulation of a mixture of all three MBP peptides significantly ameliorated protracted EAE, decreasing the overall disease profile (panel 4E).

Treatment with free MBP1 peptide did not provide any beneficial effects (panel 4G), while copaxone treatment resulted in an EAE amelioration rate similar to treatment with the MBP1/2/3 SUV formulation (panel 4F). However, copaxone-treated did not fully recover from EAE after the initial attack, as did rats treated with the liposomal MBP1/2/3 SUV formulation. These data are in accordance with representative hematoxylin and eosin staining and calculated gliosis/demyelination score (Figure 4, right panels). Moreover, MBP1 and MBP1/2/3 SUV significantly decreased both median maximal disease and median cumulative disease scores, suggesting their high therapeutic potential (Table 4).

### Example 11 - Liposomal MBP Peptides Inhibit EAE Development by Down-regulation of Th1 Cytokines and Induction of BDNF Production in CNS

To investigate the immunological status of EAE induced DA rats after treatment with liposomally encapsulated MBP peptides, serum isolated from rats treated as described in Example 10 was analyzed for anti-MBP antibodies and CNS cytokine staining (Figure 5). A significant decrease in anti-MBP autoAb concentration was observed in the serum of rats treated with liposomally encapsulated MBP peptides, as compared to the serum of rats treated with the vehicle negative control (Figure 5A). Levels of autoantibodies specific for both of the identified major MBP B cell epitopes, as well autoantibodies reactive against full-length MBP. Notably, MBP(81-103) epitopes were not present in any of the liposomal formulation, yet the concentration of autoantibodies recognizing this epitope was reduced to the same degree as for autoantibodies recognizing the full-length MBP protein. Thus, it is concluded that the observed effect could not be explained by primitive neutralization of pathogenic antibodies in the bloodstream.

MBP peptide compositions described herein may in part act through a mechanism involving autoreactive T cells, due to overlapping of the B- and T-cell epitopes (Belogurov A. et al., Bioessays 2009;31:1161-71). To investigate this possibility, staining for Th1 cytokines was performed on samples from EAE-induced DA rates treated as described in Example 10 (Figure 5B). It was found that IL-2 and IFNγ levels were significantly down-regulated in rats treated with the liposomal MBP peptide formulations (Table 5), suggesting the designed formulations function as anti-inflammatory drugs. Decreased demyelination was also observed in EAE-induced DA rats treated with the liposomal MBP peptide compositions. This observation correlated with enhanced BDNF production (Figure 5B), suggesting that the liposomally-entrapped MBP peptides function through a mechanism that is similar to that of copaxone, which is known up-regulate BDNF expression (Aharoni R et al., Proc Natl Acad Sci U S A 2003;100:14157-62).

**Table 5. Serum anti-MBP autoantibody titer and CNS cytokines profile in EAE-developing rats in response to administration of liposome-entrapped MBP peptides.**

| **Treatment group** | **Serum anti-MBP Antibody titer** | **anti-IL2 Staining** | **anti-IFN Staining** | **anti-BDNF Staining** |
|---|---|---|---|---|
| vehicle | 2.2±0.2 | ++ | +++ | + |
| MBP1 SUV | 1.1±0.2 | 0 | + | +++ |
| MBP1/2/3 SUV | 1.9±0.1 | 0 | + | +++ |
| copaxone | 1.0±0.1 | + | + | ++ |
| non-immunized | 0.02±0.0012 | - | - | - |

Histology analysis and cytokines staining was performed as follows. Spinal cords of the animals were collected, embedded in paraffin, dissected to slices and stained with an H&E and luxol fast blue (LFB). Histological parameters are the following: the grade of gliosis (scoring grade from 0 to 3; 0 corresponds to the absence of gliosis, 1 to mild gliosis (up to 5-10 cells), 2 to moderate gliosis (between 10-50 cells per focus) and 3 to severe gliosis (more than 50 cells per focus). The grade of demyelination score is graded from 0 to 3; 0 corresponds to the absence of demyelination, 1 to mild demyelination, 2 to moderate demyelination and 3 to severe demyelination. Staining for IL-2, IFN and BDNF cytokines was performed according manufacture's protocol.

The present studies identify two immunodominant regions of MBP in EAE-induced DA rats, which are also identified in human MS patients. When encapsulated in mannosylated liposomes, administration of peptides corresponding to these immunodominant regions significantly decrease EAE in DA rats, reducing first attack and enhancing recovery from exacerbation. It was found that these compositions down-regulate Th1 cytokines, induce BDNF expression, and inhibit anti-MBP Ab production (Table 5). Without being bound by theory, one possible mechanism of action for this therapeutic effect is that mannose residues present on the surface of the liposomes loaded with MBP peptides increases intake of the liposomally encapsulated MBP peptides into APC cells, which in turn leads to an increased induction of tolerance towards myelin basic protein and subsequent disease amelioration. The observed beneficial effect that liposomally encapsulated MBP fragments have on EAE disease progression in DA rats, coupled with the immunological similarities between EAE-induced DA rats and human patients with MS, suggests a novel therapeutic modality for MS treatment.

### Example 12 - Therapeutic Efficacy of Liposomally Encapsulated MBP Peptides in an MS Rat Model (DA-EAE-28-01)

To evaluate the use of MBP B-cell epitope peptides for the treatment of MS, a study was conducted in EAE-induced DA rats. The objectives of the study included: 1) to confirm the therapeutic efficacy of the MBP1 peptide; 2) to determine if SUV encapsulation provides additional therapeutic benefit; 3) to determine if MSL based SUV formulation provides additional therapeutic benefit; 4) to determine if the addition of flanking regions of MBP1 provide additional benefit in mixture mode in SUV; 5) to determine if the addition of flanking regions of MBP1 provide additional benefit in mixture mode in MSL based SUV; 6) to compare MBP1/MBP1FL/MBP1FR activity in SUV MSL formulations by use of the acute EAE model in female Dark Agouti (DA) rats.

Each of the seven formulations being evaluated was provided as a lyophilized powder and stored at 4°C. Rehydration of each daily group dose was done with water for injection according to Table 6. MBP peptide formulations were re-suspended in water for injection (Cure Medical) and copaxone (Teva LTD) was diluted with Saline to a final concentration of 150 µg/mL. Each subject animal was administered the test formulation for 6 consecutive days by subcutaneous injection.

**Table 6. Re-suspension protocols for the MBP peptide formulations under investigation.**

| **Formulation** | **Actions** | **Total Mass (mg)** | **Daily Dosage (mg)** | **Administration Volume (mL)** |
|---|---|---|---|---|
| 1 | Re-suspended in 15 mL | 2.3 | 0.3 | 0.33 |
| 2,3,6,7 | Re-suspended daily in 2.3 mL | 2664 | 380 | 0.33 |
| 4,5 | Re-suspended daily in 6.7 mL | 8016 | 1200 | 1 |

9 week old Dark Agouti (DA) female rats (Harlan Laboratories, Inc.), weighing between 125 and 145 grams were used as the test subjects. The health status of the animals used in this study was examined on arrival. Only animals in good health were acclimatized to laboratory conditions and were used in the study. Animals were provided Protein Rodent Diet (Teklad) *ad libitum* and free access to drinking water. Animals were housed in a controlled environment at between 20°C and 24°C with a relative humidity of 30-70% and a 12 hr light/12 hr dark cycle. Animals were randomly assigned to their respective test group. This study was performed following the review by the Committee for Ethical Conduct in the Care and Use of Laboratory Animals of the Assaf Harofeh medical center, Beer Yaakov, Ethical Committee number: 68/2009.

For induction of EAE, rats were intradermally injected at the base of the tail with a total volume of 200 µL of inoculum containing 50 µg of MBP(63-81) (ANASPEC), in saline mixed (1:1) with CFA, (IFA, Sigma) and 1 mg *Mt* (strain H37 RA; Difco Laboratories, Detroit, MI).

The rats were evaluated up daily starting 24 hours after immunization. On day 8 post immunization, more than 50% of the rats developed signs of paralysis. The animals displaying symptoms of MS were separated into 9 groups for the beginning of treatment. Prior to treatment, blood was collected from two rats from each group. At day 9 and 10 post immunization, 55 of 60 rats developed signs of paralysis.

7-10 days post EAE induction, 54 animals were divided to 9 groups (6 rats in each), and blood was collected from 2 rats of each group before the initiation of the treatment. Each group of rats was treated once daily with the formulation according to Table 7 for 6 consecutive days. The formulations were administered by subcutaneous injection into the lower area of the abdominal side. Blood was collected from all rats 24 h after the last injection. The animals were maintained and evaluated until day 28th post EAE-induction. Clinical scores were assigned daily during the study period. The animals were sacrificed 28 days post EAE induction, blood plasma and serum were collected from the rats' hearts. The animals were perfused with 4% PFA, brain and spinal cord were collected and fixed in 4% formaldehyde.

**Table 7. Study test groups and treatment protocols.**

| **Group** | **Number of Animals** | **Formulation** | **Peptide dose** | **Frequency and route** |
|---|---|---|---|---|
| 1 | 6 | MBP1 | 50 µg | Daily s.c. Injection (6 Total) |
| 2 | 6 | MBP1 SUV | 50 µg | Daily s.c. Injection (6 Total) |
| 3 | 6 | MBP1 SUV MSL | 50 µg | Daily s.c. Injection (6 Total) |
| 4 | 6 | MBP1/MBP1FL/MBP1FR SUV MSL | 150 µg | Daily s.c. Injection (6 Total) |
| 5 | 6 | MBP1/MBP1FL/MBP1FR SUV | 150 µg | Daily s.c. Injection (6 Total) |
| 6 | 6 | MBP1FL SUV MSL | 50 µg | Daily s.c. Injection (6 Total) |
| 7 | 6 | MBP1FR SUV MSL | 50 µg | Daily s.c. Injection (6 Total) |
| 8 | 6 | copaxone | 50 µg | Daily s.c. Injection (6 Total) |
| 9 | 6 | control | Water for injection | Daily s.c. Injection (6 Total) |

Animals were observed individually and clinical signs were recorded once daily during all study periods. Observations included changes in the fur, eyes, respiratory rate, vocalization, paralysis, activity and behavior pattern. Scoring of paralysis signs related to MS for each animal was performed daily according to the criteria in Table 8. The body weight of each animals was determined daily during all study periods. All animals with a paralysis score of more than 1 received 2 mL of water and 2 mL of rewetted Protein Rodent Diet (Teklad) daily by gavage feeding needle.

**Table 8. Study test groups and treatment protocols.**

| **Score** | **Parameters** |
|---|---|
| 0 | Normal |
| 1 | Tail weakness |
| 2 | Hind leg weakness or paralysis |
| 3 | Hind leg paralysis, dragging hind limbs |
| 4 | Complete paralysis, unable to move |
| 5 | Death |

Blood collection during life phase: Blood was collected from the orbital sinus of live rats. Blood was collected into 2 types of tubes: EDTA tubes and 2 mL Eppendorf tubes. Separated plasma and serum of each animal were further evaluated for cytokine IL-2, IL-4, IL-10, IL-17, TNF-alpha, IFN-gamma, and TGF-beta concentrations by ELISA. Blood collection during termination phase: Immediately after sacrificing, blood was collected from rats' hearts into 2 types of tubes: EDTA tubes and 2 mL Eppendorf tubes. Serum and plasma were separated and stored at -20°C.

After sacrifice, perfusion was performed with 0.5 L of 4% PFA. Immediately following blood collection, the vascular system was washed with 20 mL saline solution and 0.5 L of PFA was perfused using 180-200 mmHg via right heart chamber. Brain and spinal cord of each animal were collected and fixed in 4% formaldehyde. Tissues were trimmed, embedded in paraffin, sectioned at approximately 5 microns thickness and stained with Hematoxylin & Eosin (H&E) and PAS staining.

### Results

As summarized in Table 9, mortality occurred in groups treated with free MBP1 (Group 1; 1/6), (Group 2; 1/6), and Group 3; 2/6), copaxone (Group VIII; 2/6), and WFI (Group IX; 2/6). Conversely, no rats died in the groups treated with (Groups IV-VII, respectively).

**Table 9. Mortality rate in 54 EAE induced DA rats.**

| **Group** | **I** | **II** | **III** | **IV** | **V** | **VI** | **VII** | **VIII** | **IX** |
|---|---|---|---|---|---|---|---|---|---|
| **Mortality Rate** | 1/6 | 1/6 | 2/6 | 0/6 | 0/6 | 0/6 | 0/6 | 2/6 | 2/6 |

A statistically significant reduction in paralysis score, as compared to the other groups, was observed in rats treated with the formulation of B cell epitope peptides MBP1/MBP1FL/MBP1FR in mannosylated liposomes (Group IV) at days 3 and 4 post treatment (Figure 6A; (x)).

Body weight gain of all animals was found to be within range of normally expected values during acclimatization period. Body weight loss was observed during disease peak in animals of all groups. Body weight gain occurred during post disease peak period in all groups. No statistically significant differences were found between the treated groups and control groups for any of the body weight measurements (Figure 7).

No statistically significant differences were detected in levels of IL-2, IL-4, IL-10, IL-17, TNF-alpha, IFN-gamma, and TGF-beta before and after the treatment and between the groups treated with the tested formulations. Comparison of differences in cytokine levels between groups treated with MBP peptide formulations and the controls was not statistically significant.

To evaluate myelination in the EAE-induced rats treated with formulations I-IX, histology was performed blindly, i.e., without knowing which animals were treated with which substance, by a single pathologist. The results were compared to the histology of an untreated animal.

Briefly, all slides were stained with HE, Periodic Acid Schiff's (PAS), and Luxol Fast Blue (LFB) stains. Histological parameters were chosen to characterize the nature of the lesions. Gliosis was scored from 0 to 3, according to the following scale: 0 = no gliosis, 1 = mild gliosis (up to 5-10 cells), 2 = moderate gliosis (between 10-50 cells per focus), and 3 = severe gliosis (more than 50 cells per focus). Demyelination was scored from 0 to 3, according to the following scale: 0 = no demyelination, 1 = mild demyelination, 2 = moderate demyelination and 3 = severe demyelination. Additional lesions were also noted, if present.

Histopathological analysis of the spinal cord from 2 randomly selected animals from each group revealed the appearance of gliosis in all analyzed rats. However, a significant improvement in myelination was seen in both animals from group IV (MBP1/MBP1FL/MBP1FR encapsulated in mannosylated liposomes) and in one of the rats from group V (MBP1/MBP1FL/MBP1FR encapsulated in unmodified liposomes), as compared to animals from the other groups. Exemplary H&E staining patterns are shown in Figures 8A-C.

This study demonstrates that the administration of B cell epitope peptides MBP1, MBP1FL, and MBP1FR co-encapsulated in a mannosylated liposome results in a statistically significant reduction of paralysis in MS rodent models. This study examines the efficacy of various peptide sequences: MBP1, MBP1FL and MBP1FR in a liposomal (1% molar mannosylated lipid composition at a 1:330 peptide to lipid ratio) formulation. The liposomal formulation of all three peptides has significantly efficacious response (Group 4), as compared the individual peptides alone (Groups 2, 3, 6, and 7), and the negative control (Group 9). By comparison, the three peptides together in a liposomal composition without the 1% molar mannose lipid (Group 5 vs. Group 4) shows no significant efficacy, indicating an enhanced response by the inclusion of the mannosylated lipid. The overall disease score was comparable for the MBP1 peptide alone (Group 1) or liposomally formulated (Group 2); however histopathological analysis for gliosis and myelination of spinal cords harvested from 2 randomly selected rats from each group demonstrates a low demyelination score, indicating an improved pathological result for the liposomal formulation of the MBP1 peptide alone (Group 2). The best (i.e., lowest) demyelination score was obtained by administration of the liposomal formulation of all three MBP peptides (Group 4), which also resulted in a significant improvement in overall disease score.

### Example 13 - Therapeutic Efficacy of Liposomally Encapsulated MBP Peptides in an MS Rat Model (DA-EAE-28-02)

To further evaluate the use of MBP B-cell epitope peptides for the treatment of MS, a study was conducted in EAE-induced DA rats. Mannosylated liposomal formulations of various combinations of MBP peptides MBP1, MBP1FL, MBP1FR, MBP2, and MBP3 were tested for their therapeutic potential in the EAE-induced DA rat model of MS, described above.

Each of the eight MBP peptide formulations were provided as a lyophilized powder and stored at 4°C. Rehydration of each formulation with water for injection was done daily according to Table 10. MBP peptide formulations were re-suspended in water for injection (Cure Medical) and copaxone (Teva LTD) was diluted with saline to a final concentration of 450 µg/mL. Each subject animal was administered the test formulation for 6 consecutive days by subcutaneous injection.

**Table 10. Re-suspension protocols for the MBP peptide formulations under investigation.**

| **Formulation** | **Actions** | **Total Mass (mg)** | **Daily Dose per group (mg/day)** | **Administration Volume (mL)** |
|---|---|---|---|---|
| MBP F1 | Re-suspended daily in 7.08 mL | 11133 | 1417 | 1.01 |
| MBP F2 | Re-suspended daily in 2.36 mL | 4384 | 472 | 0.34 |
| MBP F3F4 | Re-suspended daily in 9.60 mL | 19328 | 1919 | 1.03/0.34 |
| MBP F5 | Re-suspended daily in 2.36 mL | 4986 | 472 | 0.34 |
| MBP F6 | Re-suspended daily in 2.35 mL | 4910 | 471 | 0.34 |
| MBP F7 | Re-suspended daily in 7.05 mL | 14712 | 1411 | 1.01 |
| MBP F8 | Re-suspended daily in 11.86 mL | 19987 | 2371 | 1.69 |

8-9 week old Dark Agouti (DA) female rats (Harlan Laboratories, Inc.), weighing between 110 and 145 grams were used as the test subjects. The health status of the animals used in this study was examined on arrival. Only animals in good health were acclimatized to laboratory conditions and were used in the study. Animals were provided food *ad libitum* and free access to drinking water. Animals were housed in a controlled environment at between 20°C and 24°C with a relative humidity of 30-70% and a 12 hr light/12 hr dark cycle. Animals were randomly assigned to their respective test group. This study was performed following the review by the Committee for Ethical Conduct in the Care and Use of Laboratory Animals of the Assaf Harofeh medical center, Beer Yaakov, ethical committee number: 830_b2451_6.

For induction of EAE, rats were intradermally injected at the base of the tail with a total volume of 200 µL of inoculum containing 50 µg of MBP(63-81) (ANASPEC), in saline mixed (1:1) with CFA, (IFA, Sigma) and 1 mg Mt (strain H37 RA; Difco Laboratories, Detroit, MI).

The rats were evaluated up daily starting 24 hours after immunization. On day 9 post immunization, more than 50% of the rats developed signs of paralysis. The animals displaying symptoms of MS were separated into 10 groups for the beginning of treatment. Prior to treatment, blood was collected from two rats from each group. At day 9 and 11 post immunization, 54 of 60 rats developed signs of paralysis.

9-11 days post EAE induction, 54 animals were divided to 10 groups (5-6 rats in each), and blood was collected from 2 rats of each group before the initiation of the treatment. Each group of rats was treated once daily with the formulation according to Table 11 for 6 consecutive days. The animals were maintained and evaluated until day 28th post EAE-induction. Clinical scores were assigned daily during the study period. The animals were sacrificed 28 days post EAE induction using isoflurane. Immediately after sacrificing, blood was collected from rats' hearts. Serum and plasma were separated and stored at -20°C. The animals were perfused with 4% PFA, brain and spinal cord were collected and fixed in 4% formaldehyde.

**Table 11. Study test groups and treatment protocols.**

| **Group** | **Number of Animals** | **Formulation** | **Daily Dose per group (mg/day)** | **Daily Volume (mL)** | **Administration Volume (mL)** | **Frequency and route** |
|---|---|---|---|---|---|---|
| **1** | 5 | MBP F1 | 1417 | 7.085 | 1.01 | Daily s.c. Injection (6 Total) |
| **2** | 5 | MBP F2 | 472 | 2.361 | 0.34 | Daily s.c. Injection (6 Total) |
| **3** | 5 | MBP F3F4 | 1919 | 9.595 | 0.34 | Daily s.c. Injection (6 Total) |
| **4** | 5 | MBP F3F4 | | | 1.03 | Daily s.c. Injection (6 Total) |
| **5** | 6 | MBP F5 | 472 | 2.358 | 0.34 | Daily s.c. Injection (6 Total) |
| **6** | 6 | MBP F6 | 471 | 2.354 | 1.01 | Daily s.c. Injection (6 Total) |
| **7** | 6 | MBP F7 | 1411 | 7.054 | 1.69 | Daily s.c. Injection (6 Total) |
| **8** | 6 | MBP F8 | 2371 | 11.857 | 1.69 | Daily s.c. Injection (6 Total) |
| **9** | 5 | Copaxone | 2.5 | 5 | 0.33 | Daily s.c. Injection (6 Total) |
| **10** | 5 | Water for injection | - | 0.33 | 0.33 | Daily s.c. Injection (6 Total) |

Animals were observed individually and clinical signs were recorded once daily during all study periods. Observations included changes in the fur, eyes, respiratory rate, vocalization, paralysis, activity and behavior pattern. Scoring of paralysis signs related to MS for each animal was performed daily according to the criteria in Table 8. The body weight of each animals was determined daily during all study periods. All animals with a paralysis score of more than 1 received 2 mL of water and 2 mL of rewetted Protein Rodent Diet (Teklad) daily by gavage feeding needle.

### Results

As summarized in Table 12, 1 rat died from each group treated with free liposomally encapsulated MBP2 (Group V; 1/6), copaxone (Group IX; 1/5), and WFI (Group X; 1/5).

**Table 12. Mortality rate in 54 EAE induced DA rats.**

| **Group** | **I** | **II** | **III** | **IV** | **V** | **VI** | **VII** | **VIII** | **IX** | **X** |
|---|---|---|---|---|---|---|---|---|---|---|
| **Mortality Rate** | 0/5 | 0/5 | 0/5 | 0/5 | 1/6 | 0/6 | 0/6 | 0/6 | 1/5 | 1/5 |

A statistically significant reduction in paralysis score, as compared to the water control (Group X), was observed in rats treated with a high dose of liposomally encapsulated MBP1 peptide (Group IV) at 2 and 3 days post treatment (Figure 10 (Δ)).

The weight gain of all animals was found to be within the range of expected values during the acclimatization period. Weight loss was observed during disease peak in animals of all groups. Weight gain occurred during post disease peak period in all animals of groups. No statistically significant differences were found between the groups administered liposomally encapsulated MBP peptides and the control groups (Figure 11).

With the exception of Group 2, this study tested a single liposomal formulation (1% molar mannose Lipid composition and a 1:330 peptide to lipid ratio) and examined the therapeutic efficacy of various B cell epitope MBP peptides, and combinations thereof. Notably, a statistically significant reduction in paralysis of rats treated with 200 µg (nominal) of liposomally encapsulated MBP(46-62) (Group 4) was observed, as compared to the negative control. Furthermore, a non-statistically significant difference (tendency) in paralysis of rats treated with 200 mg of liposomally encapsulated MBP(46-62) (Group 4) was observed, as compared to the copaxone treated rats (Group 9), on days 4 and 5 post treatment.

The disease severity profile observed in this study had distinct primary and relapse phases. When comparing the results in rats treated with the same dose (50 µg/day) of liposomally encapsulated single MBP peptides, it was discovered that administration of MBP(46-62) (Group III) provided the greatest therapeutic benefit in the primary disease phase, while administration of liposomally encapsulated MBP (124-139) (Group V) or MBP(147-170) (Group VI) provided therapeutic benefit during the relapse disease phase. This bias was not observed as absolute and may be negated by the peptide dose, as administration of high dose liposomally encapsulated MBP(46-62) (Group 4) is the most efficacious across both phases. The therapeutic benefit of liposomal formulations containing multiple MBP peptides is more difficult to interpret, possible due to the low dose of each respective peptides. With respect to the use of the same peptide formulated at different peptide to lipid ratios (compare Groups I and II), no difference in the overall disease severity score was observed.

### Example 14 - Therapeutic Efficacy of Liposomally Encapsulated MBP Peptides in an MS Rat Model (DA-EAE-28-05)

To further evaluate the use of MBP B-cell epitope peptides for the treatment of MS, a study was conducted in EAE-induced DA rats. The objectives of the study included: 1) further confirmation that liposomal formulations of MBP1 alone and MBP1/2/3 provide a therapeutic benefit in an MS rat model; and 2.) examination of the therapeutic effect of different dosages and peptide to lipid ratios for the liposomal MBP formulations.

Each of the four MBP peptide formulations being evaluated were provided as lyophilized powders and stored at 4°C. Each formulation was rehydrated in water for injection according to Table 13. Copaxone (Teva LTD) was diluted with Saline to a final concentration of 720 µg/mL. Each subject animal was administered the test formulation for 6 consecutive days by subcutaneous injection.

**Table 13. Re-suspension protocols for the MBP peptide formulations under investigation.**

| **Formulation** | **Actions** | **Total Mass (mg)** | **Administration Volume (mL)** |
|---|---|---|---|
| MBP F I | Re-suspended daily in 1.5 mL | 300 | 0.21 |
| MBP F II | Re-suspended daily in 4.5 mL | 900 | 0,63 |
| MBP F III | Re-suspended daily in 0.7 mL | 140 | 0.1 |
| MBP F IV | Re-suspended daily in 2.0 mL | 400 | 0.26 |

8-9 week old Dark Agouti (DA) female rats (Harlan Laboratories, Inc.), weighing between 110 and 145 grams were used as the test subjects. The health status of the animals used in this study was examined on arrival. Only animals in good health were acclimatized to laboratory conditions and were used in the study. Animals were provided food *ad libitum* and free access to drinking water. Animals were housed in a controlled environment at between 20°C and 24°C with a relative humidity of 30-70% and a 12 hr light/12 hr dark cycle. Animals were randomly assigned to their respective test group. This study was performed following the review by the Committee for Ethical Conduct in the Care and Use of Laboratory Animals of the Science in action LTD, Rehovot. Ethical Committee number: IL-10-11-109.

For induction of EAE, rats were intradermally injected at the base of the tail with a total volume of 200 µL of inoculum containing 50 µg of MBP(63-81) (ANASPEC), in saline mixed (1:1) with CFA, (IFA, Sigma) and 1 mg Mt (strain H37 RA; Difco Laboratories, Detroit, MI).

The rats were evaluated up daily starting 24 hours after immunization. 42 of 50 rats developed signs of paralysis at days 6-10 post immunization. The animals displaying symptoms of MS were separated into 7 groups (6 rats in each) for the beginning of treatment. Each group of rats was treated once daily with the formulation according to Table 14 for 6 consecutive days. The formulations were administered by subcutaneous injection into the lower area of the abdominal side. Blood was collected from all rats 24 h after the last injection. The animals were maintained and evaluated until day 28th post EAE-induction. Clinical scores were assigned daily during the study period. The animals were sacrificed 28 days post EAE induction, blood plasma and serum were collected from the rats' hearts. The animals were perfused with 4% PFA, brain and spinal cord were collected and fixed in 4% formaldehyde.

**Table 14. Study test groups and treatment protocols.**

| **Group** | **Number of Animals** | **Formulation** | **Peptide dose (µg)** | **Frequency and route** |
|---|---|---|---|---|
| 1 | 6 | control | Water for injection | Daily s.c. Injection (6 Total) |
| 2 | 6 | MBP F-I | 150 | Daily s.c. Injection (6 Total) |
| 3 | 6 | MBP F-II | 450 | Daily s.c. Injection (6 Total) |
| 4 | 6 | MBP F-III | 150 | Daily s.c. Injection (6 Total) |
| 5 | 6 | MBP F-IV | 450 | Daily s.c. Injection (6 Total) |
| 6 | 6 | Copaxone | 450 | Daily s.c. Injection (6 Total) |

Animals were observed individually and clinical signs were recorded once daily during all study periods. Observations included changes in the fur, eyes, respiratory rate, vocalization, paralysis, activity and behavior pattern. Scoring of paralysis signs related to MS for each animal was performed daily according to the criteria in Table 8. The body weight of each animals was determined daily during all study periods.

### Results

No rats died prior to day 28 post EAE-induction, in this study.

Statistically significant reductions in paralysis score, as compared to the water control (Group 1), was observed in rats treated with: MBP1 peptide formulated at 1:330 (peptide:lipid; Group 2), and MBP1/2/3 peptides formulated at 1:330 (peptide:lipid; Group 3) and 1:110 (peptide:lipid; Group 5, at days 1-4 post-treatment (Figure 12). A non-statistically significant difference (tendency) in the paralysis of rats treated with copaxone (Groups 6 and 7) was observed, as compared to the water control (Group 1).

The weight gain of all animals was found to be within the range of expected values during the acclimatization period. Weight loss was observed during disease peak in animals of all groups. Weight gain occurred during post disease peak period in all animals of groups. No statistically significant differences were found between the groups administered liposomally encapsulated MBP peptides and the control groups (Figure 13).

This study shows that treatment with liposomally formulated B cell epitope peptide MBP1 and co-liposomally formulated B cell epitope peptides MBP1/2/3 provide statistically significant therapeutic benefit in a rat model of MS. At higher peptide to lipid ratios, co-formulations of MBP1/2/3 appear to provide greater therapeutic than MBP1 peptide alone. Conversely, at lower peptide to lipid ratios, formulations of MBP1 alone appear to provide greater therapeutic than MBP1/2/3 co-formulations. In both cases, however, liposomally formulated MBP1 peptides provided greater therapeutic benefit than copaxone, a therapeutic approved for the treatment of relapsing-remitting multiple sclerosis.

It is understood that the examples and embodiments described herein are for illustrative purposes only and that various modifications or changes in light thereof will be suggested to persons skilled in the art.

## Claims

1. A composition for use in medicine, the composition consisting of two myelin basic protein (MBP) peptides linked to a vector, the first MBP peptide consisting of the amino acid sequence of SEQ ID NO: 11 and the second MBP peptide consisting of the amino acid sequence of SEQ ID NO:12, and the vector comprising a liposome having a surface exposed targeting moiety comprising a manDOG mannose residue.

2. The composition for use according to claim **1**, wherein one or more of the MBP peptides is covalently linked to the vector.

3. The composition for use according to claim **1** or **2**, wherein one or more of the MBP peptides is non-covalently linked to the vector.

4. The composition for use according to claim **1**, wherein the targeting moiety increases:
(a) delivery of the MBP peptide to an immune cell; or
(b) intake of the MBP peptide into an immune cell;
as compared to an MBP peptide linked to a vector in the absence of a targeting moiety.

5. The composition for use according to any one of claims **1** to **4**, wherein the MBP peptides are encapsulated by the liposome.

6. The composition for use according to any one of claims **1** to **5**, wherein the liposome has an average diameter of from 100 nm to 200 nm.

7. A pharmaceutical composition comprising the composition according to any one of claims **1** to **6** and a pharmaceutically acceptable excipient or carrier, for use in the treatment of multiple sclerosis in a patient in need thereof.

8. The pharmaceutical composition for use according to claim **7**, wherein the composition is in a weekly dosage form.

9. The pharmaceutical composition for use according to claim **7**, wherein the composition is in a daily dosage form.

10. The pharmaceutical composition for use according to any one of claims **7** to **9**, wherein the composition is in a form suitable for topical administration, enteric administration, or parenteral administration.

11. The pharmaceutical composition for use according to any one of claims **7** to **10**, wherein the multiple sclerosis is relapsing remitting multiple sclerosis (RRMS), secondary progressive multiple sclerosis (SPMS), primary progressive multiple sclerosis (PPMS), or progressive relapsing multiple sclerosis (PRMS).

12. A method of making the composition according to any one of claims **1** to **6**, comprising:
(i) mixing manDOG mannose residue with a lipid in the presence of an organic solvent;
(ii) evaporating the organic solvent, thereby forming irregular lipid layers;
(iii) re-hydrating the irregular lipid layers of step (ii) to obtain multi-layer multilamellar vesicles (MLV);
(iv) homogenizing the MLV of step (iii) to obtain small unilamellar vesicles (SUV);
(v) mixing the SUV of step (iv) with the two MBP peptides and excess sugar, and freeze drying the mixture;
(vi) re-hydrating the mixture of step (v) to obtain SUV having an average diameter of approximately 60 to 100 nm.

## Patentansprüche

1. Zusammensetzung für den medizinischen Gebrauch, wobei die Zusammensetzung aus zwei Myelin-Basische Protein (MBP)-Peptiden besteht, die zu einem Vektor verknüpft sind, wobei das erste MBP-Peptid aus der Aminosäuresequenz SEQ ID Nr.: 11 besteht und das zweite MBP-Peptid aus der Aminosäuresequenz SEQ ID Nr.: 12 besteht, und der Vektor ein Liposom umfasst, welches einer oberflächenexponierten Targeting-anteil aufweist, welcher einen ManDOG Mannose-Rückstand umfasst.

2. Zusammensetzung für den Gebrauch nach Anspruch **1**, wobei ein oder mehrere der MBP-Peptide kovalent mit dem Vektor verknüpft sind.

3. Zusammensetzung für den Gebrauch nach Anspruch **1** oder **2**, wobei ein oder mehrere der MBP-Peptide nicht kovalent mit dem Vektor verknüpft sind.

4. Zusammensetzung für den Gebrauch nach Anspruch **1**, wobei der Targeting-anteil Folgendes erhöht:
(a) Abgabe des MBP-Peptids an eine Immunzelle; oder
(b) Einnahme des MBP-Peptids in eine Immunzelle;
wie verglichen mit einem MBP-Peptid, das mit einem Vektor in Abwesenheit eines Targeting-anteils verknüpft ist.

5. Zusammensetzung für den Gebrauch nach einem der Ansprüche **1** bis **4**, wobei die MBP-Peptide durch das Liposom eingekapselt sind.

6. Zusammensetzung für den Gebrauch nach einem der Ansprüche **1** bis **5**, wobei das Liposom einen mittleren Durchmesser von 100 nm bis 200 nm aufweist.

7. Pharmazeutische Zusammensetzung, die Zusammensetzung nach einem der Ansprüche **1** bis **6** umfassend und einen pharmazeutisch unbedenklichen Hilfsstoff oder Träger, für den Gebrauch bei der Behandlung von Multiple Sklerose bei einem Patienten der sie benötigt.

8. Pharmazeutische Zusammensetzung für den Gebrauch nach Anspruch **7**, wobei die Zusammensetzung in einer wöchentlichen Darreichungsform ist.

9. Pharmazeutische Zusammensetzung für den Gebrauch nach Anspruch **7**, wobei die Zusammensetzung in einer täglichen Darreichungsform ist.

10. Pharmazeutische Zusammensetzung für den Gebrauch nach einem der Ansprüche **7** bis **9**, wobei die Zusammensetzung in einer Form ist, die sich zur topischen Verabreichung, enterischen Verabreichung oder parenteralen Verabreichung eignet.

11. Pharmazeutische Zusammensetzung für den Gebrauch nach einem der Ansprüche **7** bis **10**, wobei die Multiple Sklerose eine schubförmig remittierende Multiple Sklerose (RRMS), sekundär progrediente Multiple Sklerose (SPMS), primär progrediente Multiple Sklerose (PPMS), oder progressiv rezidivierende Multiple Sklerose (PRMS) ist.

12. Verfahren zur Herstellung der Zusammensetzung nach einem der Ansprüche **1** bis **6**, umfassend:
(i) mischen eines ManDOG Mannose-Rückstands mit einem Lipid in Gegenwart eines organischen Lösungsmittels;
(ii) verdampfen des organischen Lösungsmittels, wodurch unregelmäßige Lipid-Schichten gebildet werden;
(iii) rehydrieren der unregelmäßigen Lipid-Schichten aus Schritt (ii), um mehrschichtige mehrlamellare Vesikel (MLV) zu erhalten;
(iv) homogenisieren der MLV aus Schritt (iii), um kleine unilamellare Vesikel (SUV) zu erhalten;
(v) mischen der SUV aus Schritt (iv) mit den beiden MBP-Peptiden und Zuckerüberschuss, und Gefriertrocknen der Mischung;
(vi) rehydrieren der Mischung aus Schritt (v), um SUV zu erhalten, das einen mittleren Durchmesser von etwa 60 bis 100 nm aufweist.

## Revendications

1. Une composition pour son utilisation en médecine, la composition consistant en deux peptides de la protéine basique de la myéline (PBM) couplées à un vecteur, le premier peptide PBM consistant en la séquence d'acides aminés de SEQ ID NO: 11 et le deuxième peptide PBM consistant en la séquence d'acides aminés de SEQ ID NO: 12, et le vecteur comprenant un liposome ayant un fragment de ciblage exposé à la surface comprenant un résidu mannose manDOG.

2. La composition pour son utilisation selon la revendication **1**, dans laquelle l'un ou plus des peptides PBM est couplé de manière covalente au vecteur.

3. La composition pour son utilisation selon la revendication **1** ou **2**, dans laquelle l'un ou plus des peptides PBM est couplé de manière non-covalente au vecteur.

4. La composition pour son utilisation selon la revendication **1**, dans laquelle le fragment de ciblage augmente :
(a) la distribution du peptide PBM à une cellule immunitaire ; ou
(b) l'apport du peptide PBM dans une cellule immunitaire ;
par comparaison à un peptide PBM couplé à un vecteur en l'absence de fragment de ciblage.

5. La composition pour son utilisation selon l'une quelconque des revendications **1** à **4**, dans laquelle les peptides PBM sont encapsulés par le liposome.

6. La composition pour son utilisation selon l'une quelconque des revendications **1** à **5**, dans laquelle le liposome a un diamètre moyen de 100 nm à 200 nm.

7. Une composition pharmaceutique comprenant la composition selon l'une quelconque des revendications **1** à **6** et un excipient ou un véhicule pharmaceutiquement acceptable, pour son utilisation dans le traitement de la sclérose en plaque chez un patient en ayant besoin.

8. La composition pharmaceutique pour son utilisation selon la revendication **7**, dans laquelle la composition est sous une forme de doses hebdomadaires.

9. La composition pharmaceutique pour son utilisation selon la revendication **7**, dans laquelle la composition est sous une forme de doses journalières.

10. La composition pharmaceutique pour son utilisation selon l'une quelconque des revendications **7** à **9**, dans laquelle la composition est sous une forme adaptée à l'administration topique, l'administration entérique, ou l'administration parentérale.

11. La composition pharmaceutique pour son utilisation selon l'une quelconque des revendications **7** à **10**, dans laquelle la sclérose en plaque est une sclérose en plaque rémittente-récurrente (RRMS), une sclérose en plaque secondairement progressive (SPMS), une sclérose en plaque primaire progressive (PPMS), ou une sclérose en plaque progressive récurrente (PRMS).

12. Une méthode de production de la composition selon l'une quelconque des revendications **1** à **6**, comprenant :
(i) mélanger un résidu mannose manDOG avec un lipide en présence d'un solvant organique ;
(ii) évaporer le solvant organique, formant ainsi des couches lipidiques irrégulières ;
(iii) réhydrater les couches lipidiques irrégulières de l'étape (ii) pour obtenir des vésicules multicouches multi-lamellaires (VML) ;
(iv) homogénéiser les VML de l'étape (iii) pour obtenir des petites vésicules uni-lamellaires (PVU) ;
(v) mélanger les PVU de l'étape (iv) avec les deux peptides PBM et un excès de sucre, et lyophiliser le mélange ;
(vi) réhydrater le mélange de l'étape (v) pour obtenir des PVU ayant un diamètre moyen d'environ 60 à 100 nm.
